(19) Europäisches Patentamt European Patent Office Office européen des brevets

(11) **EP 3 455 242 B1**

(12) **EUROPEAN PATENT SPECIFICATION**

(45) Date of publication and mention of the grant of the patent:
**23.06.2021 Bulletin 2021/25**

(21) Application number: **17727807.4**

(22) Date of filing: **10.05.2017**

(51) Int Cl.:
***C07K 14/31*** *(2006.01)*

(86) International application number:
**PCT/EP2017/061160**

(87) International publication number:
**WO 2017/194594 (16.11.2017 Gazette 2017/46)**

(54) **SEPARATION METHOD**

TRENNVERFAHREN

MÉTHODE DE SÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.05.2016 GB 201608229**
**11.05.2016 GB 201608232**
**30.09.2016 US 201615282367**
**10.11.2016 US 201615348699**

(43) Date of publication of application:
**20.03.2019 Bulletin 2019/12**

(73) Proprietor: **Cytiva BioProcess R&D AB**
**751 84 Uppsala (SE)**

(72) Inventors:
• **FORSS, Annika**
**751 84 Uppsala (SE)**
• **RODRIGO, Gustav, José**
**751 84 Uppsala (SE)**
• **BJORKMAN, Tomas**
**751 84 Uppsala (SE)**
• **ANDER, Mats**
**751 84 Uppsala (SE)**
• **HANSSON, Jesper, Ulf**
**751 84 Uppsala (SE)**

(74) Representative: **Larsson, Jan Anders**
**GE Healthcare Bio-Sciences AB**
**Björkgatan 30**
**751 84 Uppsala (SE)**

(56) References cited:
**WO-A1-2012/083425    WO-A1-2015/005859**
**WO-A1-2016/079033**

**Description**

Technical field of the invention

The present invention relates to the field of affinity chromatography, and more specifically to mutated immunoglobulin-binding domains of Protein A, which are useful in affinity chromatography of immunoglobulins. The invention also relates to multimers of the mutated domains and to separation matrices containing the mutated domains or multimers.

Background of the invention

Immunoglobulins represent the most prevalent biopharmaceutical products in either manufacture or development worldwide. The high commercial demand for and hence value of this particular therapeutic market has led to the emphasis being placed on pharmaceutical companies to maximize the productivity of their respective mAb manufacturing processes whilst controlling the associated costs.

Affinity chromatography is used in most cases, as one of the key steps in the purification of these immunoglobulin molecules, such as monoclonal or polyclonal antibodies. A particularly interesting class of affinity reagents is proteins capable of specific binding to invariable parts of an immunoglobulin molecule, such interaction being independent on the antigen-binding specificity of the antibody. Such reagents can be widely used for affinity chromatography recovery of immunoglobulins from different samples such as but not limited to serum or plasma preparations or cell culture derived feed stocks. An example of such a protein is staphylococcal protein A, containing domains capable of binding to the Fc and Fab portions of IgG immunoglobulins from different species. These domains are commonly denoted as the E-, D-, A-, B- and C-domains.

Staphylococcal protein A (SpA) based reagents have due to their high affinity and selectivity found a widespread use in the field of biotechnology, e.g. in affinity chromatography for capture and purification of antibodies as well as for detection or quantification. At present, SpA-based affinity medium probably is the most widely used affinity medium for isolation of monoclonal antibodies and their fragments from different samples including industrial cell culture supernatants. Accordingly, various matrices comprising protein A-ligands are commercially available, for example, in the form of native protein A (e.g. Protein A SEPHAROSE™, GE Healthcare, Uppsala, Sweden) and also comprised of recombinant protein A (e.g. rProtein A-SEPHAROSE™, GE Healthcare). More specifically, the genetic manipulation performed in the commercial recombinant protein A product is aimed at facilitating the attachment thereof to a support and at increasing the productivity of the ligand.

These applications, like other affinity chromatography applications, require comprehensive attention to definite removal of contaminants. Such contaminants can for example be non-eluted molecules adsorbed to the stationary phase or matrix in a chromatographic procedure, such as non-desired biomolecules or microorganisms, including for example proteins, carbohydrates, lipids, bacteria and viruses. The removal of such contaminants from the matrix is usually performed after a first elution of the desired product in order to regenerate the matrix before subsequent use. Such removal usually involves a procedure known as cleaning-in-place (CIP), wherein agents capable of eluting contaminants from the stationary phase are used. One such class of agents often used is alkaline solutions that are passed over said stationary phase. At present the most extensively used cleaning and sanitizing agent is NaOH, and the concentration thereof can range from 0.1 up to e.g. 1 M, depending on the degree and nature of contamination. This strategy is associated with exposing the matrix to solutions with pH-values above 13. For many affinity chromatography matrices containing proteinaceous affinity ligands such alkaline environment is a very harsh condition and consequently results in decreased capacities owing to instability of the ligand to the high pH involved.

An extensive research has therefore been focused on the development of engineered protein ligands that exhibit an improved capacity to withstand alkaline pH-values. For example, Gülich et al. (Susanne Gülich, Martin Linhult, Per-Ake Nygren, Mathias Uhlén, Sophia Hober, Journal of Biotechnology 80 (2000), 169-178) suggested protein engineering to improve the stability properties of a Streptococcal albumin-binding domain (ABD) in alkaline environments. Gülich et al. created a mutant of ABD, wherein all the four asparagine residues have been replaced by leucine (one residue), aspartate (two residues) and lysine (one residue). Further, Gülich et al. report that their mutant exhibits a target protein binding behavior similar to that of the native protein, and that affinity columns containing the engineered ligand show higher binding capacities after repeated exposure to alkaline conditions than columns prepared using the parental non-engineered ligand. Thus, it is concluded therein that all four asparagine residues can be replaced without any significant effect on structure and function.

Recent work shows that changes can also be made to protein A (SpA) to effect similar properties. US patent application publication US 2005/0143566 discloses that when at least one asparagine residue is mutated to an amino acid other than glutamine or aspartic acid, the mutation confers an increased chemical stability at pH-values of up to about 13-14 compared to the parental SpA, such as the B-domain of SpA, or Protein Z, a synthetic construct derived from the B-domain of SpA (US 5,143,844). The authors show that when these mutated proteins are used as affinity ligands, the separation media as expected can better withstand cleaning procedures using alkaline agents. Further mutations of protein A domains with the purpose of increasing the alkali stability have also been published in US 8,329,860, JP 2006304633A, US 8,674,073, US 2010/0221844, US 2012/0208234, US 9,051,375, US 2014/0031522, US 2013/0274451 and WO 2014/146350. However, the currently available mutants are still sensitive to alkaline pH and the NaOH concentration during cleaning is usually limited to 0.1 M, which means that complete cleaning is difficult to achieve. Higher NaOH concentrations, which would improve the cleaning, lead to unacceptable capacity losses. WO2015005859 and WO2012083425 disclose Protein A domain mutants with improved alkali stability.

There is thus still a need in this field to obtain a separation matrix containing protein ligands having a further improved stability towards alkaline cleaning procedures. There is also a need for such separation matrices with an improved binding capacity to allow for economically efficient purification of therapeutic antibodies.

Summary of the invention

An objective of the invention is to provide an efficient and economical method of isolating an immunoglobulin or other Fc-containing protein. This is achieved with a method as defined in Claim 1. The method comprises the steps of:

a) providing a separation matrix comprising at least 15 mg/ml multimers of immunoglobulin-binding alkali-stabilized Protein A domains covalently coupled to a porous support, wherein said porous support comprises cross-linked agarose particles having a volume-weighted median diameter (d50,v) of 56-70 micrometers, a dry solids weight of 55-80 mg/ml and a pore size corresponding to an inverse gel filtration chromatography Kd value of 0.69-0.85 for dextran of Mw 110 kDa, and wherein said separation matrix has a max pressure of at least 0.58, such as at least 0.60, MPa when packed at 300 +/-10 mm bed height in a FineLine™ 35 column,
b) contacting a liquid sample comprising an immunoglobulin with said separation matrix, wherein at least 40 mg immunoglobulin per ml separation matrix is contacted with said separation matrix,
c) washing said separation matrix with a washing liquid,
d) eluting the immunoglobulin from the separation matrix with an elution liquid, and
e) cleaning the separation matrix with a cleaning liquid,

wherein said alkali-stabilized Protein A domains comprise mutants of a parental Fc-binding domain of Staphylococcus Protein A (SpA), as defined by SEQ ID NO 51 or SEQ ID NO 52, wherein the amino acid residues at positions 13 and 44 of SEQ ID NO 51 or 52 are asparagines and wherein at least the asparagine residue at position 3 of SEQ ID NO 51 or 52 has been mutated to an amino acid selected from the group consisting of glutamic acid, lysine, tyrosine, threonine, phenylalanine, leucine, isoleucine, tryptophan, methionine, valine, alanine, histidine and arginine, such as to a glutamic acid.

Further suitable embodiments of the invention are described in the dependent claims.

Definitions

The terms "antibody" and "immunoglobulin" are used interchangeably herein, and are understood to include also fragments of antibodies, fusion proteins comprising antibodies or antibody fragments and conjugates comprising antibodies or antibody fragments.

The terms an "Fc-binding polypeptide" and "Fc-binding protein" mean a polypeptide or protein respectively, capable of binding to the crystallisable part (Fc) of an antibody and includes e.g. Protein A and Protein G, or any fragment or fusion protein thereof that has maintained said binding property.

The term "linker" herein means an element linking two polypeptide units, monomers or domains to each other in a multimer.

The term "spacer" herein means an element connecting a polypeptide or a polypeptide multimer to a support.

The term "% identity" with respect to comparisons of amino acid sequences is determined by standard alignment algorithms such as, for example, Basic Local Alignment Tool (BLAST™) described in Altshul et al. (1990) J. Mol. Biol., 215: 403-410. A web-based software for this is freely available from the US National Library of Medicine at http://blast.ncbi.nlm.nih.gov/Blast.cgi?PROGRAM=blastp&PAGE_TYPE=BlastSearch&LINK_ LOC=blasthome . Here, the algorithm "blastp (protein-protein BLAST)" is used for alignment of a query sequence with a subject sequence and determining i.a. the % identity.

As used herein, the terms "comprises," "comprising," "containing," "having" and the like can have the meaning ascribed to them in U.S. Patent law and can mean "includes," "including," and the like; "consisting essentially of" or "consists essentially" likewise has the meaning ascribed in U.S. Patent law and the term is open-ended, allowing for the presence of more than that which is recited so long as basic or novel characteristics of that which is recited is not changed by the presence of more than that which is recited, but excludes prior art embodiments.

Brief description of figures

Fig. 1 shows an alignment of the Fc-binding domains as defined by SEQ ID NO:1-7 and 51-52.

Fig. 2 shows results from Example 2 for the alkali stability of parental and mutated tetrameric Zvar (SEQ ID NO 7) polypeptide variants coupled to an SPR biosensor chip.

Fig. 3 shows results from Example 4 for the alkali stability (0.5 M NaOH) of parental and mutated tetrameric Zvar (SEQ ID NO 7) polypeptide variants coupled to agarose beads.

Fig. 4 shows results from Example 4 for the alkali stability (1.0 M NaOH) of parental and mutated tetrameric Zvar (SEQ ID NO 7) polypeptide variants coupled to agarose beads.

Fig. 5 shows results from Example 7 for the alkali stability (1.0 M NaOH) of agarose beads with different amounts of mutated multimer variants (SEQ ID NO. 20) coupled. The results are plotted as the relative remaining dynamic capacity (Qb10%, 6 min residence time) vs. incubation time in 1 M NaOH.

Fig. 6 shows results from Example 7 for the alkali stability (1.0 M NaOH) of agarose beads with different amounts of mutated multimer variants (SEQ ID NO. 20) coupled. The results are plotted as the relative remaining dynamic capacity (Qb10%, 6 min residence time) after 31 h incubation in 1 M NaOH vs. the ligand content of the prototypes.

Fig. 7 shows results from a pH gradient elution of polyclonal human IgG a) from the reference matrix MabSelect SuRe LX and b) a matrix according to the invention.

Fig. 8 shows analyses of the IgG1, IgG2 and IgG4 components in fractions from the chromatograms of Fig. 7. a) reference matrix and b) matrix according to the invention. For each fraction, the first bar (blue) represents IgG1, the second (red) IgG 4 and the third (green) IgG 2.

Fig. 9 shows results from accelerated alkali stability measurements with 1 M NaOH incubation for the reference matrix MabSelect SuRe LX (MSS LX) and a matrix according to the invention. The stability is expressed as the percentage of the 10% breakthrough capacity remaining after incubation.

Detailed description of embodiments

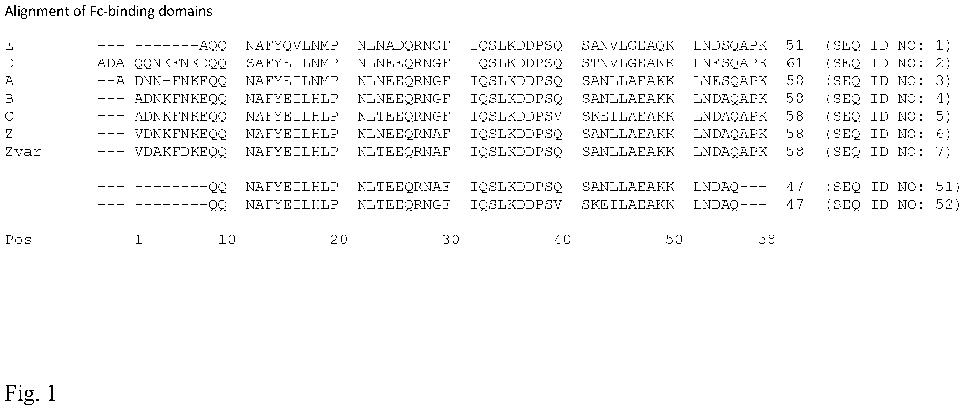

The method of the present invention uses an Fc-binding polypeptide, which comprises, or consists essentially of, a mutant of an Fc-binding domain of Staphylococcus Protein A (SpA). Fc-binding domains of SpA are illustrated by SEQ ID NO: 1 (E-domain), SEQ ID NO: 2 (D-domain), SEQ ID NO:3 (A-domain), SEQ ID NO:22 (variant A-domain), SEQ ID NO: 4 (B-domain), SEQ ID NO: 5 (C-domain), SEQ ID NO:6 (Protein Z), SEQ ID NO:7 (Zvar), SEQ ID NO 51 (Zvar without the linker region amino acids 1-8 and 56-58) or SEQ ID NO 52 (C-domain without the linker region amino acids 1-8 and 56-58) as illustrated in Fig. 1 . In the mutants, at least the asparagine residue at the position* corresponding to position 11 in SEQ ID NO:4-7 has been mutated to an amino acid selected from the group consisting of glutamic acid, lysine, tyrosine, threonine, phenylalanine, leucine, isoleucine, tryptophan, methionine, valine, alanine, histidine and arginine. Protein Z (SEQ ID NO:6) is a mutated B-domain as disclosed in US5143844, while SEQ ID NO 7 denotes a further mutated variant of Protein Z, here called Zvar, with the mutations N3A,N6D,N23T. SEQ ID NO:22 is a natural variant of the A-domain in Protein A from Staphylococcus aureus strain N315, having an A46S mutation, using the position terminology of Fig. 1. The mutation of N11 in these domains confers an improved alkali stability in comparison with the parental domain/polypeptide, without impairing the immunoglobulin-binding properties. Hence, the polypeptide can also be described as an Fc- or immunoglobulin-binding polypeptide, or alternatively as an Fc- or immunoglobulin-binding polypeptide unit.

*Throughout this description, the amino acid residue position numbering convention of Fig 1 is used, and the position numbers are designated as corresponding to those in SEQ ID NO 4-7. This applies also to multimers, where the position numbers designate the positions in the polypeptide units or monomers according to the convention of Fig. 1.

SEQ ID NO 51 (truncated Zvar)
QQ NAFYEILHLP NLTEEQRNAF IQSLKDDPSQ SANLLAEAKK LNDAQ

SEQ ID NO 52 (truncated C domain)
QQ NAFYEILHLP NLTEEQRNGF IQSLKDDPSV SKEILAEAKK LNDAQ

The N11 ($X_2$) mutation (e.g. a N1 IE or N11K mutation) may be the only mutation or the polypeptide may also comprise further mutations, such as substitutions in at least one of the positions corresponding to positions 3, 6, 9, 10, 15, 18, 23, 28, 29, 32, 33, 36, 37, 40, 42, 43, 44, 47, 50, 51, 55 and 57 in SEQ ID NO:4-7. In one or more of these positions, the original amino acid residue may e.g. be substituted with an amino acid which is not asparagine, proline or cysteine. The original amino acid residue may e.g. be substituted with an alanine, a valine, a threonine, a serine, a lysine, a glutamic acid or an aspartic acid. Further, one or more amino acid residues may be deleted, e.g. from positions 1-6 and/or from positions 56-58.

In some embodiments, the amino acid residue at the position corresponding to position 9 in SEQ ID NO:4-7 ($X_1$) is an amino acid other than glutamine, asparagine, proline or cysteine, such as an alanine or it can be deleted. The combination of the mutations at positions 9 and 11 provides particularly good alkali stability, as shown by the examples. In specific embodiments, in SEQ ID NO: 7 the amino acid residue at position 9 is an alanine and the amino acid residue at position 11 is a lysine or glutamic acid, such as a lysine. Mutations at position 9 are also discussed in copending application PCT/SE2014/050872.

In some embodiments, the amino acid residue at the position corresponding to position 50 in SEQ ID NO:4-7 ($X_{13}$) is an arginine or a glutamic acid.

In certain embodiments, the amino acid residue at the position corresponding to position 3 in SEQ ID NO:4-7 is an alanine and/or the amino acid residue at the position corresponding to position 6 in SEQ ID NO:4-7 is an aspartic acid. One of the amino acid residues at positions 3 and 6 may be an asparagine and in an alternative embodiment both amino acid residues at positions 3 and 6 may be asparagines.

In some embodiments the amino acid residue at the position corresponding to position 43 in SEQ ID NO:4-7 ($X_{11}$) is an alanine or a glutamic acid, such as an alanine or it can be deleted. In specific embodiments, the amino acid residues at positions 9 and 11 in SEQ ID NO: 7 are alanine and lysine/glutamic acid respectively, while the amino acid residue at position 43 is alanine or glutamic acid.

In certain embodiments the amino acid residue at the position corresponding to position 28 in SEQ ID NO:4-7 ($X_5$) is an alanine or an asparagine, such as an alanine.

In some embodiments the amino acid residue at the position corresponding to position 40 in SEQ ID NO:4-7 ($X_9$) is selected from the group consisting of asparagine, alanine, glutamic acid and valine, or from the group consisting of glutamic acid and valine or it can be deleted. In specific embodiments, the amino acid residues at positions 9 and 11 in SEQ ID NO: 7 are alanine and glutamic acid respectively, while the amino acid residue at position 40 is valine. Optionally, the amino acid residue at position 43 may then be alanine or glutamic acid.

In certain embodiments, the amino acid residue at the position corresponding to position 42 in SEQ ID NO:4-7 ($X_{10}$) is an alanine, lysine or arginine or it can be deleted.

In some embodiments the amino acid residue at the position corresponding to position 18 in SEQ ID NO:4-7 ($X_3$) is a lysine or a histidine, such as a lysine.

In certain embodiments the amino acid residue at the position corresponding to position 33 in SEQ ID NO:4-7 ($X_7$) is a lysine or a serine, such as a lysine.

In some embodiments the amino acid residue at the position corresponding to position 37 in SEQ ID NO:4-7 ($X_8$) is a glutamic acid or an aspartic acid, such as a glutamic acid.

In certain embodiments the amino acid residue at the position corresponding to position 51 in SEQ ID NO:4-7 ($X_{14}$) is a tyrosine or a leucine, such as a tyrosine.

In some embodiments, the amino acid residue at the position corresponding to position 44 in SEQ ID NO:4-7 ($X_{12}$) is a leucine or an isoleucine. In specific embodiments, the amino acid residues at positions 9 and 11 in SEQ ID NO: 7 are alanine and lysine/glutamic acid respectively, while the amino acid residue at position 44 is isoleucine. Optionally, the amino acid residue at position 43 may then be alanine or glutamic acid.

In some embodiments, the amino acid residues at the positions corresponding to positions 1, 2, 3 and 4 or to positions 3, 4, 5 and 6 in SEQ ID NO: 4-7 have been deleted. In specific variants of these embodiments, the parental polypeptide is the C domain of Protein A (SEQ ID NO: 5). The effects of these deletions on the native C domain are described in US9018305 and US8329860.

In some embodiments, the polypeptide comprises or consists essentially of a sequence defined by or having at least 90%, 95% or 98% identity to an amino acid sequence selected from the group consisting of: SEQ ID NO 8, SEQ ID NO 9, SEQ ID NO 10, SEQ ID NO 11, SEQ ID NO 12, SEQ ID NO 13, SEQ ID NO 14, SEQ ID NO 15, SEQ ID NO 16, SEQ ID NO 23, SEQ ID NO 24, SEQ ID NO 25, SEQ ID NO 26, SEQ ID NO 27, SEQ ID NO 28, SEQ ID NO 29, SEQ ID NO 36, SEQ ID NO 37, SEQ ID NO 38, SEQ ID NO 39, SEQ ID NO 40, SEQ ID NO 41, SEQ ID NO 42, SEQ ID NO 43, SEQ ID NO 44, SEQ ID NO 45, SEQ ID NO 46, SEQ ID NO 47, SEQ ID NO 48, SEQ ID NO 49 and SEQ ID NO 50. It may e.g. comprise or consist essentially of a sequence defined by or having at least 90%, 95% or 98% identity to an amino acid sequence selected from the group consisting of: SEQ ID NO 8, SEQ ID NO 9, SEQ ID NO 10, SEQ ID NO 11, SEQ ID NO 16, SEQ ID NO 23, SEQ ID NO 24, SEQ ID NO 25, SEQ ID NO 26, SEQ ID NO 27, SEQ ID NO 28 and SEQ ID NO 29. It can also comprise or consist essentially of a sequence defined by or having at least 90%, 95% or 98% identity to an amino acid sequence selected from the group consisting of: SEQ ID NO 8, SEQ ID NO 9, SEQ ID NO 10, SEQ ID NO 11, SEQ ID NO 16, SEQ ID NO 23, SEQ ID NO 24, SEQ ID NO 25, SEQ ID NO 27, SEQ ID NO 28, SEQ ID NO 38, SEQ ID NO 40; SEQ ID NO 41; SEQ ID NO 42; SEQ NO 43, SEQ ID NO 44, SEQ ID NO 45, SEQ ID NO 46, SEQ ID NO 47 and SEQ ID NO 48.

In certain embodiments, the polypeptide comprises or consists essentially of a sequence defined by or having at least 90%, 95% or 98% identity to an amino acid sequence selected from the group consisting of SEQ ID NO 54-70. comprises or consists essentially of a sequence defined by or having at least 90%, 95% or 98% identity to an amino acid sequence selected from the group consisting of SEQ ID NO 71-75, or it may comprise or consist essentially of a sequence defined by or having at least 90%, 95% or 98% identity to an amino acid sequence selected from the group consisting of SEQ ID NO 76-79. It may further comprise or consist essentially of a sequence defined by or having at least 90%, 95% or 98% identity to an amino acid sequence selected from the group consisting of SEQ ID NO 89-95.

The polypeptide may e.g. be defined by a sequence selected from the groups above or from subsets of these groups, but it may also comprise additional amino acid residues at the N- and/or C-terminal end, e.g. a leader sequence at the N-terminal end and/or a tail sequence at the C-terminal end.

SEQ ID NO 8 Zvar(Q9A,N11E,N43A)

VDAKFDKEAQ EAFYEILHLP NLTEEQRNAF IQSLKDDPSQ SAALLAEAKK
LNDAQAPK

SEQ ID NO 9 Zvar(Q9A,N11E,N28A,N43A)

VDAKFDKEAQ EAFYEILHLP NLTEEQRAAF IQSLKDDPSQ SAALLAEAKK
LNDAQAPK

SEQ ID NO 10 Zvar(Q9A,N11E,Q40V,A42K,N43E,L44I)

VDAKFDKEAQ EAFYEILHLP NLTEEQRNAF IQSLKDDPSV SKEILAEAKK
LNDAQAPK

SEQ ID NO 11 Zvar(Q9A,N11E,Q40V,A42K,N43A,L44I)

VDAKFDKEAQ EAFYEILHLP NLTEEQRNAF IQSLKDDPSV SKAILAEAKK
LNDAQAPK

SEQ ID NO 12 Zvar(N11E,Q32A)

VDAKFDKEQQ EAFYEILHLP NLTEEQRNAF IASLKDDPSQ SANLLAEAKK
LNDAQAPK

SEQ ID NO 13 Zvar(N1IE)

VDAKFDKEQQ EAFYEILHLP NLTEEQRNAF IQSLKDDPSQ SANLLAEAKK
LNDAQAPK

SEQ ID NO 14 Zvar(N11E,Q32E,Q40E)

VDAKFDKEQQ EAFYEILHLP NLTEEQRNAF IESLKDDPSE SANLLAEAKK
LNDAQAPK

SEQ ID NO 15 Zvar(N11E,Q32E,K50R)

VDAKFDKEQQ EAFYEILHLP NLTEEQRNAF IESLKDDPSQ SANLLAEAK**R**
LNDAQAPK

SEQ ID NO 16 Zvar(N11K)

VDAKFDKEQQ **K**AFYEILHLP NLTEEQRNAF IQSLKDDPSQ SANLLAEAKK
LNDAQAPK

SEQ ID NO 23 Zvar(N11K,H18K,S33K,D37E,A42R,N43A,L441,K50R,L51Y)

VDAKFDKEQQ **K**AFYEIL**K**LP NLTEEQRNAF IQ**K**LKD**E**PSQ S**RAI**LAEAK**R**
**Y**NDAQAPK

SEQ ID NO 24 Zvar(Q9A,N11E,N28A,Q40V,A42K,N43A,L44I)

VDAKFDKE**A**Q **E**AFYEILHLP NLTEEQR**A**AF IQSLKDDPS**V** SK**AI**LAEAKK
LNDAQAPK

SEQ ID NO 25 Zvar(Q9A,N11K,H18K,S33K,D37E,A42R,N43A,L44I,K50R,L51Y)

Zvar(Q9A,N11K,H18K,S33K,D37E,A42R,N43A,L44I,K50R,L51Y)
VDAKFDKE**A**Q **K**AFYEIL**K**LP NLTEEQR**A**AF IQ**K**LKD**E**PSQ S**RAI**LAEAK**R**
**Y**NDAQAPK

SEQ ID NO 26 Zvar(N11K, H18K, D37E, A42R, N43A, L44I)

VDAKFDKEQQ **K**AFYEIL**K**LP NLTEEQRNAF IQSLKD**E**PSQ S**RAI**LAEAKK
LNDAQAPK

SEQ ID NO 27 Zvar(Q9A, N11K, H18K, D37E, A42R, N43A, L44I)

VDAKFDKE**A**Q **K**AFYEIL**K**LP NLTEEQRNAF IQSLKD**E**PSQ S**RAI**LAEAKK
LNDAQAPK

SEQ ID NO 28 Zvar(Q9A, N11K, H18K, D37E A42R N43A L44I, K50R)

VDAKFDKE**A**Q **K**AFYEIL**K**LP NLTEEQRNAF IQSLKD**E**PSQ
S**RAI**LAEAK**R** LNDAQAPK

SEQ ID NO 29 Zvar(Q9A,N11K,H18K,D37E,A42R)

VDAKFDKE**AQ K**AFYEIL**K**LP NLTEEQRNAF IQSLKD**E**PSQ S**R**NLLAEAKK LNDAQAPK

SEQ ID NO 36 B(Q9A,N11E,Q40V,A42K,N43A,L44I)

ADNKFNKE**AQ E**AFYEILHLP NLEEQRNGF IQSLKDDPSV S**KAI**LAEAKK LNDAQAPK

SEQ ID NO 37 C(Q9A,N11E,E43A)

ADNKFNKE**AQ E**AFYEILHLP NLTEEQRNGF IQSLKDDPSV SKA**I**LAEAKK LNDAQAPK

SEQ ID NO 38 Zvar(N11Y)

VDAKFDKEQQ **Y**AFYEILHLP NLTEEQRNAF IQSLKDDPSQ SANLLAEAKK LNDAQAPK

SEQ ID NO 39 Zvar(N1IT)

VDAKFDKEQQ **T**AFYEILHLP NLTEEQRNAF IQSLKDDPSQ SANLLAEAKK LNDAQAPK

SEQ ID NO 40 Zvar(N1IF)

VDAKFDKEQQ **F**AFYEILHLP NLTEEQRNAF IQSLKDDPSQ SANLLAEAKK LNDAQAPK

SEQ ID NO 41 Zvar(N11L)

VDAKFDKEQQ **L**AFYEILHLP NLTEEQRNAF IQSLKDDPSQ SANLLAEAKK LNDAQAPK

SEQ ID NO 42 Zvar(N11W)

VDAKFDKEQQ **W**AFYEILHLP NLTEEQRNAF IQSLKDDPSQ SANLLAEAKK LNDAQAPK

SEQ ID NO 43 Zvar(N11I)

VDAKFDKEQQ **I**AFYEILHLP NLTEEQRNAF IQSLKDDPSQ SANLLAEAKK LNDAQAPK

SEQ ID NO 44 Zvar(N11M)

VDAKFDKEQQ **M**AFYEILHLP NLTEEQRNAF IQSLKDDPSQ SANLLAEAKK LNDAQAPK

SEQ ID NO 45 Zvar(N11V)

> VDAKFDKEQQ **V**AFYEILHLP NLTEEQRNAF IQSLKDDPSQ SANLLAEAKK
> LNDAQAPK

SEQ ID NO 46 Zvar(N11A)

> VDAKFDKEQQ **A**AFYEILHLP NLTEEQRNAF IQSLKDDPSQ SANLLAEAKK
> LNDAQAPK

SEQ ID NO 47 Zvar(N11H)

> VDAKFDKEQQ **H**AFYEILHLP NLTEEQRNAF IQSLKDDPSQ SANLLAEAKK
> LNDAQAPK

SEQ ID NO 48 Zvar(N11R)

> VDAKFDKEQQ **R**AFYEILHLP NLTEEQRNAF IQSLKDDPSQ SANLLAEAKK
> LNDAQAPK

SEQ ID NO 49 Zvar(Q9A,N11E,D37E,Q40V,A42K,N43A,L44I)

> VDAKFDKE**A**Q **E**AFYEILHLP NLTEEQRNAF IQSLKD**E**PS**V SKAI**LAEAKK
> LNDAQAPK

SEQ ID NO 50 Zvar(Q9A,N11E,D37E,Q40V,A42R,N43A,L44I)

> VDAKFDKE**A**Q **E**AFYEILHLP NLTEEQRNAF IQSLKD**E**PS**V SRAI**LAEAKK
> LNDAQAPK

SEQ ID NO 54 Zvar(Q9A,N11E, A29G,Q40V,A42K,N43A,L44I)

> VDAKFDKE**A**Q **E**AFYEILHLP NLTEEQRN**G**F IQSLKDDPS**V SKAI**LAEAKK
> LNDAQAPK

SEQ ID NO 55 Zvar(Q9A,N11E, A29S,Q40V,A42K,N43A,L44I)

> VDAKFDKE**A**Q **E**AFYEILHLP NLTEEQRN**S**F IQSLKDDPS**V SKAI**LAEAKK
> LNDAQAPK

SEQ ID NO 56 Zvar(Q9A,N11E, A29Y,Q40V,A42K,N43A,L44I)

> VDAKFDKE**A**Q **E**AFYEILHLP NLTEEQRN**Y**F IQSLKDDPS**V SKAI**LAEAKK
> LNDAQAPK

SEQ ID NO 57 Zvar(Q9A,N11E, A29Q,Q40V,A42K,N43A,L44I)

VDAKFDKEAQ EAFYEILHLP NLTEEQRNQF IQSLKDDPSV SKAILAEAKK
LNDAQAPK

SEQ ID NO 58 Zvar(Q9A,N11E, A29T,Q40V,A42K,N43A,L44I)

VDAKFDKEAQ EAFYEILHLP NLTEEQRNTF IQSLKDDPSV SKAILAEAKK
LNDAQAPK

SEQ ID NO 59 Zvar(Q9A,N11E, A29N,Q40V,A42K,N43A,L44I)

VDAKFDKEAQ EAFYEILHLP NLTEEQRNNF IQSLKDDPSV SKAILAEAKK
LNDAQAPK

SEQ ID NO 60 Zvar(Q9A,N11E, A29F,Q40V,A42K,N43A,L44I)

VDAKFDKEAQ EAFYEILHLP NLTEEQRNFF IQSLKDDPSV SKAILAEAKK
LNDAQAPK

SEQ ID NO 61 Zvar(Q9A,N11E, A29L,Q40V,A42K,N43A,L44I)

VDAKFDKEAQ EAFYEILHLP NLTEEQRNLF IQSLKDDPSV SKAILAEAKK
LNDAQAPK

SEQ ID NO 62 Zvar(Q9A,N11E, A29W,Q40V,A42K,N43A,L44I)

VDAKFDKEAQ EAFYEILHLP NLTEEQRNWF IQSLKDDPSV SKAILAEAKK
LNDAQAPK

SEQ ID NO 63 Zvar9AN11E, A29I,Q40V,A42K,N43A,L44I)

VDAKFDKEAQ EAFYEILHLP NLTEEQRNIF IQSLKDDPSV SKAILAEAKK
LNDAQAPK

SEQ ID NO 64 Zvar(Q9A,N11E, A29M,Q40V,A42K,N43A,L44I)

VDAKFDKEAQ EAFYEILHLP NLTEEQRNMF IQSLKDDPSV SKAILAEAKK
LNDAQAPK

SEQ ID NO 65 Zvar(Q9A,N11E, A29V,Q40V,A42K,N43A,L44I)

VDAKFDKEAQ EAFYEILHLP NLTEEQRNVF IQSLKDDPSV SKAILAEAKK
LNDAQAPK

SEQ ID NO 66 Zvar(Q9A,N11E, A29D,Q40V,A42K,N43A,L44I)

VDAKFDKEAQ EAFYEILHLP NLTEEQRNDF IQSLKDDPSV SKAILAEAKK
LNDAQAPK

SEQ ID NO 67 Zvar(Q9A,N11E, A29E,Q40V,A42K,N43A,L44I)

VDAKFDKEAQ EAFYEILHLP NLTEEQRNEF IQSLKDDPSV SKAILAEAKK
LNDAQAPK

SEQ ID NO 68 Zvar(Q9A,N11E, A29H,Q40V,A42K,N43A,L44I)

VDAKFDKEAQ EAFYEILHLP NLTEEQRNHF IQSLKDDPSV SKAILAEAKK
LNDAQAPK

SEQ ID NO 69 Zvar(Q9A,N11E, A29R,Q40V,A42K,N43A,L44I)

VDAKFDKEAQ EAFYEILHLP NLTEEQRNRF IQSLKDDPSV SKAILAEAKK
LNDAQAPK

SEQ ID NO 70 Zvar(Q9A,N11E, A29K,Q40V,A42K,N43A,L44I)

VDAKFDKEAQ EAFYEILHLP NLTEEQRNKF IQSLKDDPSV SKAILAEAKK
LNDAQAPK

SEQ ID NO 71 Zvar(Q9A,N11E, Q40V,A42K,N43A,L44I,D53F)

VDAKFDKEAQ EAFYEILHLP NLTEEQRNAF IQSLKDDPSV SKAILAEAKK
LNFAQAPK

SEQ ID NO 72 Zvar(Q9A,N11E, Q40V,A42K,N43A,L44I,D53Y)

VDAKFDKEAQ EAFYEILHLP NLTEEQRNAF IQSLKDDPSV SKAILAEAKK
LNYAQAPK

SEQ ID NO 73 Zvar(Q9A,N11E, Q40V,A42K,N43A,L44I,D53W)

VDAKFDKEAQ EAFYEILHLP NLTEEQRNAF IQSLKDDPSV SKAILAEAKK
LNWAQAPK

SEQ ID NO 74 Zvar(Q9A,N11E, Q40V,A42K,N43A,L44I,D53K)

VDAKFDKEAQ EAFYEILHLP NLTEEQRNAF IQSLKDDPSV SKAILAEAKK
LNKAQAPK

SEQ ID NO 75 Zvar(Q9A,N11E, Q40V,A42K,N43A,L44I,D53R)

VDAKFDKEAQ EAFYEILHLP NLTEEQRNAF IQSLKDDPSV SKAILAEAKK
LNRAQAPK

SEQ ID NO 76 Zvar(Q9del,N11E, Q40V,A42K,N43A,L44I)

VDAKFDKE_Q EAFYEILHLP NLTEEQRNAF IQSLKDDPSV SKAILAEAKK
LNDAQAPK

SEQ ID NO 77 Zvar(Q9A,N11E, Q40del,A42K,N43A,L44I)

VDAKFDKEAQ EAFYEILHLP NLTEEQRNAF IQSLKDDPS_ SKAILAEAKK
LNDAQAPK

SEQ ID NO 78 Zvar(Q9A,N11E, Q40V,A42del,N43A,L44I)

VDAKFDKEAQ EAFYEILHLP NLTEEQRNAF IQSLKDDPSV S_AILAEAKK
LNDAQAPK

SEQ ID NO 79 Zvar(Q9A,N11E, Q40V,A42K,N43del,L44I)

VDAKFDKEAQ EAFYEILHLP NLTEEQRNAF IQSLKDDPSV SK_ILAEAKK
LNDAQAPK

SEQ ID NO 89 Zvar(D2del,A3del,K4del,Q9A,N11E,Q40V,A42K,N43A,L44I)

V___FDKEAQ EAFYEILHLP NLTEEQRNAF IQSLKDDPSV SKAILAEAKK
LNDAQAPK

SEQ ID NO 90 Zvar(V1del,D2del,Q9A,N11E,Q40V,A42K,N43A,L44I,K58del)

__AKFDKEAQ EAFYEILHLP NLTEEQRNAF IQSLKDDPSV SKAILAEAKK
LNDAQAP_

SEQ ID NO 91
Zvar(K4del,F5del,D6del,K7del,E8del,Q9A,N11E,Q40V,A42K,N43A,L44I)

VDA_____AQ EAFYEILHLP NLTEEQRNAF IQSLKDDPSV SKAILAEAKK
LNDAQAPK

SEQ ID NO 92 Zvar(Q9A,N11E,Q40V,A42K,N43A,L44I,A56del,P57del,K58del)

VDAKFDKEAQ EAFYEILHLP NLTEEQRNAF IQSLKDDPSV SKAILAEAKK
LNDAQ___

SEQ ID NO 93 Zvar(V1del,,D2del,A3del,Q9A,N11E,Q40V,A42K,N43A,L44I)

___KFDKEAQ EAFYEILHLP NLTEEQRNAF IQSLKDDPSV SKAILAEAKK
LNDAQAPK

SEQ ID NO 94
Zvar(V1del,D2del,A3del,K4del,F5del,D6del,K7del,E8del,Q9A,N11E,Q40V,A42K,N43A,L44I)
_AQ EAFYEILHLP NLTEEQRNAF IQSLKDDPSV SKAILAEAKK LNDAQAPK

SEQ ID NO 95 Zvar(Q9A,N11E,Q40V,A42K,N43A,L44I,K58_insYEDG)

VDAKFDKEAQ EAFYEILHLP NLTEEQRNAF IQSLKDDPSV SKAILAEAKK LNDAQAPKYE DG

The present invention uses a multimer comprising, or consisting essentially of, a plurality of polypeptide units as defined above. The use of multimers may increase the immunoglobulin binding capacity and multimers may also have a higher alkali stability than monomers. The multimer can e.g. be a dimer, a trimer, a tetramer, a pentamer, a hexamer, a heptamer, an octamer or a nonamer. It can be a homomultimer, where all the units in the multimer are identical or it can be a heteromultimer, where at least one unit differs from the others. Advantageously, all the units in the multimer are alkali stable, such as by comprising the mutations disclosed above. The polypeptides can be linked to each other directly by peptide bonds between the C-terminal and N-terminal ends of the polypeptides. Alternatively, two or more units in the multimer can be linked by linkers comprising oligomeric or polymeric species, such as linkers comprising peptides with up to 25 or 30 amino acids, such as 3-25 or 3-20 amino acids. The linkers may e.g. comprise or consist essentially of a peptide sequence defined by, or having at least 90% identity or at least 95% identity, with an amino acid sequence selected from the group consisting of APKVDAKFDKE, APKVDNKFNKE, APKADNKFNKE, APKVFDKE, APAKFDKE, AKFDKE, APKVDA, VDAKFDKE, APKKFDKE, APK, APKYEDGVDAKFDKE and YEDG or alternatively selected from the group consisting of APKADNKFNKE, APKVFDKE, APAKFDKE, AKFDKE, APKVDA, VDAKFDKE, APKKFDKE, APKYEDGVDAKFDKE and YEDG. They can also consist essentially of a peptide sequence defined by or having at least 90% identity or at least 95% identity with an amino acid sequence selected from the group consisting of APKADNKFNKE, APKVFDKE, APAKFDKE, AKFDKE, APKVDA, VDAKFDKE, APKKFDKE, APK and APKYEDGVDAKFDKE. In some embodiments the linkers do not consist of the peptides APKVDAKFDKE or APKVDNKFNKE, or alternatively do not consist of the peptides APKVDAKFDKE, APKVDNKFNKE , APKFNKE, APKFDKE, APKVDKE or APKADKE.

The nature of such a linker should preferably not destabilize the spatial conformation of the protein units. This can e.g. be achieved by avoiding the presence of proline in the linkers. Furthermore, said linker should preferably also be sufficiently stable in alkaline environments not to impair the properties of the mutated protein units. For this purpose, it is advantageous if the linkers do not contain asparagine. It can additionally be advantageous if the linkers do not contain glutamine. The multimer may further at the N-terminal end comprise a plurality of amino acid residues e.g. originating from the cloning process or constituting a residue from a cleaved off signaling sequence. The number of additional amino acid residues may e.g. be 20 or less, such as 15 or less, such as 10 or less or 5 or less. As a specific example, the multimer may comprise an AQ, AQGT, VDAKFDKE, AQVDAKFDKE or AQGTVDAKFDKE sequence at the N-terminal end.

In certain embodiments, the multimer may comprise, or consist essentially, of a sequence selected from the group consisting of: SEQ ID NO 80-87. These and additional sequences are listed below and named as Parent(Mutations)n, where n is the number of monomer units in a multimer.

SEQ ID NO 17 Zvar(Q9A,N11E,N43A)4

AQGT VDAKFDKEAQ EAFYEILHLP NLTEEQRNAF IQSLKDDPSQ SAALLAEAKK LNDAQAPK VDAKFDKEAQ EAFYEILHLP NLTEEQRNAF IQSLKDDPSQ SAALLAEAKK LNDAQAPK VDAKFDKEAQ EAFYEILHLP NLTEEQRNAF IQSLKDDPSQ SAALLAEAKK LNDAQAPK VDAKFDKEAQ EAFYEILHLP NLTEEQRNAF IQSLKDDPSQ SAALLAEAKK LNDAQAPKC

SEQ ID NO 18 Zvar(Q9A,N11E,N28A,N43A)4

AQGT VDAKFDKEAQ EAFYEILHLP NLTEEQRAAF IQSLKDDPSQ SAALLAEAKK LNDAQAPK VDAKFDKEAQ EAFYEILHLP NLTEEQRAAF IQSLKDDPSQ SAALLAEAKK LNDAQAPK VDAKFDKEAQ EAFYEILHLP NLTEEQRAAF IQSLKDDPSQ SAALLAEAKK LNDAQAPK VDAKFDKEAQ EAFYEILHLP NLTEEQRAAF IQSLKDDPSQ SAALLAEAKK LNDAQAPKC

SEQ ID NO 19 Zvar(Q9A,N11E,Q40V,A42K,N43E,L44I)4

AQGT VDAKFDKEAQ EAFYEILHLP NLTEEQRNAF IQSLKDDPSV SKEILAEAKK
LNDAQAPK VDAKFDKEAQ EAFYEILHLP NLTEEQRNAF IQSLKDDPSV SKEILAEAKK
LNDAQAPK VDAKFDKEAQ EAFYEILHLP NLTEEQRNAF IQSLKDDPSV SKEILAEAKK
LNDAQAPK VDAKFDKEAQ EAFYEILHLP NLTEEQRNAF IQSLKDDPSV SKEILAEAKK
LNDAQAPKC

SEQ ID NO 20 Zvar(Q9A,N11E,Q40V,A42K,N43A,L44I)4

AQGT VDAKFDKEAQ EAFYEILHLP NLTEEQRNAF IQSLKDDPSV SKAILAEAKK
LNDAQAPK VDAKFDKEAQ EAFYEILHLP NLTEEQRNAF IQSLKDDPSV SKAILAEAKK
LNDAQAPK VDAKFDKEAQ EAFYEILHLP NLTEEQRNAF IQSLKDDPSV SKAILAEAKK
LNDAQAPK VDAKFDKEAQ EAFYEILHLP NLTEEQRNAF IQSLKDDPSV SKAILAEAKK
LNDAQAPKC

SEQ ID NO 30 Zvar(N11K,H18K,S33K,D37E,A42R,N43A,L44I,K50R,L51Y)4

AQGT VDAKFDKEQQ KAFYEILKLP NLTEEQRNAF IQKLKDEPSQ SRAILAEAKR
YNDAQAPK VDAKFDKEQQ KAFYEILKLP NLTEEQRNAF IQKLKDEPSQ
SRAILAEAKR YNDAQAPK VDAKFDKEQQ KAFYEILKLP NLTEEQRNAF
IQKLKDEPSQ SRAILAEAKR YNDAQAPK VDAKFDKEQQ KAFYEILKLP
NLTEEQRNAF IQKLKDEPSQ SRAILAEAKR YNDAQAPKC

SEQ ID NO 31 Zvar(Q9A,N11K,H18K,D37E,A42R)4

AQGT VDAKFDKEAQ KAFYEILKLP NLTEEQRNAF IQSLKDEPSQ SRNLLAEAKK
LNDAQAPK VDAKFDKEAQ KAFYEILKLP NLTEEQRNAF IQSLKDEPSQ
SRNLLAEAKK LNDAQAPK VDAKFDKEAQ KAFYEILKLP NLTEEQRNAF
IQSLKDEPSQ SRNLLAEAKK LNDAQAPK VDAKFDKEAQ KAFYEILKLP
NLTEEQRNAF IQSLKDEPSQ SRNLLAEAKK LNDAQAPKC

SEQ ID NO 32 Zvar(Q9A,N11E,N28A,Q40V,A42K,N43A,L44I)4

AQGT VDAKFDKEAQ EAFYEILHLP NLTEEQRAAF IQSLKDDPSV SKAILAEAKK
LNDAQAPK VDAKFDKEAQ EAFYEILHLP NLTEEQRAAF IQSLKDDPSV
SKAILAEAKK LNDAQAPK VDAKFDKEAQ EAFYEILHLP NLTEEQRAAF
IQSLKDDPSV SKAILAEAKK LNDAQAPK VDAKFDKEAQ EAFYEILHLP
NLTEEQRAAF IQSLKDDPSV SKAILAEAKK LNDAQAPKC

SEQ ID NO 33 Zvar(Q9A,N11E,Q40V,A42K,N43A,L44I)6

AQGT VDAKFDKEAQ EAFYEILHLP NLTEEQRNAF IQSLKDDPSV SKAILAEAKK
LNDAQAPK VDAKFDKEAQ EAFYEILHLP NLTEEQRNAF IQSLKDDPSV SKAILAEAKK
LNDAQAPK VDAKFDKEAQ EAFYEILHLP NLTEEQRNAF IQSLKDDPSV SKAILAEAKK
LNDAQAPK VDAKFDKEAQ EAFYEILHLP NLTEEQRNAF IQSLKDDPSV SKAILAEAKK
LNDAQAPK VDAKFDKEAQ EAFYEILHLP NLTEEQRNAF IQSLKDDPSV SKAILAEAKK
LNDAQAPK VDAKFDKEAQ EAFYEILHLP NLTEEQRNAF IQSLKDDPSV SKAILAEAKK
LNDAQAPKC

SEQ ID NO 34 Zvar(Q9A,N11E,D37E,Q40V,A42K,N43A,L44I)4


AQGT VDAKFDKEAQ EAFYEILHLP NLTEEQRNAF IQSLKDEPSV SKAILAEAKK
LNDAQAPK VDAKFDKEAQ EAFYEILHLP NLTEEQRNAF IQSLKDEPSV
SKAILAEAKK LNDAQAPK VDAKFDKEAQ EAFYEILHLP NLTEEQRNAF

IQSLKDEPSV SKAILAEAKK LNDAQAPK VDAKFDKEAQ EAFYEILHLP
NLTEEQRNAF IQSLKDEPSV SKAILAEAKK LNDAQAPKC

SEQ ID NO 35 Zvar(Q9A,N11E,D37E,Q40V,A42R,N43A,L44I)4


AQGT VDAKFDKEAQ EAFYEILHLP NLTEEQRNAF IQSLKDEPSV SRAILAEAKK
LNDAQAPK VDAKFDKEAQ EAFYEILHLP NLTEEQRNAF IQSLKDEPSV
SRAILAEAKK LNDAQAPK VDAKFDKEAQ EAFYEILHLP NLTEEQRNAF
IQSLKDEPSV SRAILAEAKK LNDAQAPK VDAKFDKEAQ EAFYEILHLP
NLTEEQRNAF IQSLKDEPSV SRAILAEAKK LNDAQAPKC

SEQ ID NO 80 Zvar(Q9A,N11E,Q40V,A42K,N43A,L44I)2 with D2, A3 and K4 in linker deleted


VDAKFDKEAQ EAFYEILHLP NLTEEQRNAF IQSLKDDPSV SKAILAEAKK
LNDAQAPK VFDKEAQ EAFYEILHLP NLTEEQRNAF IQSLKDDPSV SKAILAEAKK
LNDAQAPKC

SEQ ID NO 81 Zvar(Q9A,N11E,Q40V,A42K,N43A,L44I)2 with K58, V1 and D2 in linker deleted


VDAKFDKEAQ EAFYEILHLP NLTEEQRNAF IQSLKDDPSV SKAILAEAKK
LNDAQAP AKFDKEAQ EAFYEILHLP NLTEEQRNAF IQSLKDDPSV SKAILAEAKK
LNDAQAPKC

SEQ ID NO 82 Zvar(Q9A,N11E,Q40V,A42K,N43A,L44I)2 with P57, K58, V1, D2 and A3 in linker deleted


VDAKFDKEAQ EAFYEILHLP NLTEEQRNAF IQSLKDDPSV SKAILAEAKK
LNDAQAP AKFDKEAQ EAFYEILHLP NLTEEQRNAF IQSLKDDPSV SKAILAEAKK
LNDAQAPKC

SEQ ID NO 83 Zvar(Q9A,N11E,Q40V,A42K,N43A,L44I)2 with K4, F5, D6, K7 and E8 in linker deleted


VDAKFDKEAQ EAFYEILHLP NLTEEQRNAF IQSLKDDPSV SKAILAEAKK
LNDAQAPK VDAAQ EAFYEILHLP NLTEEQRNAF IQSLKDDPSV SKAILAEAKK
LNDAQAPKC

SEQ ID NO 84 Zvar(Q9A,N11E,Q40V,A42K,N43A,L44I)2 with A56, P57 and K58 in linker deleted

VDAKFDKEAQ EAFYEILHLP NLTEEQRNAF IQSLKDDPSV SKAILAEAKK LNDA**Q VDAKFDKE**AQ EAFYEILHLP NLTEEQRNAF IQSLKDDPSV SKAILAEAKK LNDAQAPKC

SEQ ID NO 85 Zvar(Q9A,N11E,Q40V,A42K,N43A,L44I)2 with V1, D2 and A3 in linker deleted

VDAKFDKEAQ EAFYEILHLP NLTEEQRNAF IQSLKDDPSV SKAILAEAKK LNDA**QAPK KFDKE**AQ EAFYEILHLP NLTEEQRNAF IQSLKDDPSV SKAILAEAKK LNDAQAPKC

SEQ ID NO 86 Zvar(Q9A,N11E,Q40V,A42K,N43A,L44I)2 with V1, D2, A3, K4, F5, D6, K7 and E8 in linker deleted

VDAKFDKEAQ EAFYEILHLP NLTEEQRNAF IQSLKDDPSV SKAILAEAKK LNDA**QAPK** AQ EAFYEILHLP NLTEEQRNAF IQSLKDDPSV SKAILAEAKK LNDAQAPKC

SEQ ID NO 87 Zvar(Q9A,N11E,Q40V,A42K,N43A,L44I)2 with YEDG inserted in linker between K58 and V1

VDAKFDKEAQ EAFYEILHLP NLTEEQRNAF IQSLKDDPSV SKAILAEAKK LNDA**QAPK YEDG VDAKFDKE**AQ EAFYEILHLP NLTEEQRNAF IQSLKDDPSV SKAILAEAKK LNDAQAPKC

SEQ ID NO 88 Zvar2

VDAKFDKEAQ EAFYEILHLP NLTEEQRNAF IQSLKDDPSV SKAILAEAKK LNDAQAPK VDAKFDKEAQ EAFYEILHLP NLTEEQRNAF IQSLKDDPSV SKAILAEAKK LNDAQAPKC

In some embodiments, the polypeptide and/or multimer, as disclosed above, further comprises at the C-terminal or N-terminal end one or more coupling elements, selected from the group consisting of one or more cysteine residues, a plurality of lysine residues and a plurality of histidine residues. The coupling element(s) may also be located within 1-5 amino acid residues, such as within 1-3 or 1-2 amino acid residues from the C-terminal or N-terminal end. The coupling element may e.g. be a single cysteine at the C-terminal end. The coupling element(s) may be directly linked to the C- or N-terminal end, or it/they may be linked via a stretch comprising up to 15 amino acids, such as 1-5, 1-10 or 5-10 amino acids. This stretch should preferably also be sufficiently stable in alkaline environments not to impair the properties of the mutated protein. For this purpose, it is advantageous if the stretch does not contain asparagine. It can additionally be advantageous if the stretch does not contain glutamine. An advantage of having a C-terminal cysteine is that endpoint coupling of the protein can be achieved through reaction of the cysteine thiol with an electrophilic group on a support. This provides excellent mobility of the coupled protein which is important for the binding capacity.

The alkali stability of the polypeptide or multimer can be assessed by coupling it to an SPR chip, e.g. to Biacore CM5 sensor chips as described in the examples, using e.g. NHS- or maleimide coupling chemistries, and measuring the immunoglobulin-binding capacity of the chip, typically using polyclonal human IgG, before and after incubation in alkaline solutions at a specified temperature, e.g. 22 +/- 2 °C. The incubation can e.g. be performed in 0.5 M NaOH for a number of 10 min cycles, such as 100, 200 or 300 cycles. The IgG capacity of the matrix after 100 10 min incubation cycles in 0.5 M NaOH at 22 +/- 2 °C can be at least 55, such as at least 60, at least 80 or at least 90% of the IgG capacity before the incubation. Alternatively, the remaining IgG capacity after 100 cycles for a particular mutant measured as above can be compared with the remaining IgG capacity for the parental polypeptide/multimer. In this case, the remaining IgG capacity for the mutant may be at least 105%, such as at least 110%, at least 125%, at least 150% or at least 200% of the parental polypeptide/multimer.

The present invention uses a separation matrix, wherein a plurality of polypeptides or multimers according to any embodiment disclosed above have been coupled to a solid support. The separation matrix comprises at least 15, such as 15-21 or 15-18 mg/ml Fc-binding ligands covalently coupled to a porous support, wherein:

> a) the ligands comprise multimers of alkali-stabilized Protein A domains,
> b) the porous support comprises cross-linked polymer particles having a volume-weighted median diameter (d50,v) of 56-70, such as 56-66, micrometers and a dry solids weight of 55-80, such as 60-78 or 65-78, mg/ml. The cross-linked polymer particles have a pore size corresponding to an inverse gel filtration chromatography Kd value of 0.69-0.85, such as 0.70-0.85 or 0.69-0.80, for dextran of Mw 110 kDa. Suitably, the cross-linked polymer particles can have a high rigidity, to be able to withstand high flow rates. The rigidity can be measured with a pressure-flow test as further described in Example 11, where a column packed with the matrix is subjected to increasing flow rates of distilled water. The pressure is increased stepwise and the flow rate and back pressure measured, until the flow rate starts to decrease with increasing pressures. The maximum flow rate achieved and the maximum pressure (the back pressure corresponding to the maximum flow rate) are measured and used as measures of the rigidity. When measured in a FineLine™ 35 column (GE Healthcare Life Sciences) at a bed height of 300 +/- 10 mm, the max pressure is at least 0.58 MPa, such as at least 0.60 MPa. This allows for the use of smaller particle diameters, which is beneficial for the dynamic capacity. The multimers may e.g. comprise tetramers, pentamers, hexamers or heptamers of alkali-stabilized Protein A domains, such as hexamers of of alkali-stabilized Protein A domains. The combination of the high ligand contents with the particle size range, the dry solids weight range and the optional Kd range provides for a high binding capacity, e.g. such that the 10% breakthrough dynamic binding capacity for IgG is at least 45 mg/ml, such as at least 50 or at least 55 mg/ml at 2.4 min residence time. Alternatively, or additionally, the 10% breakthrough dynamic binding capacity for IgG may be at least 60 mg/ml, such as at least 65, at least 70 or at least 75 mg/ml at 6 min residence time.

The alkali-stabilized Protein A multimers are highly selective for IgG and the separation matrix can suitably have a dissociation constant for human IgG2 of below 0.2 mg/ml, such as below 0.1 mg/ml, in 20 mM phosphate buffer, 180 mM NaCl, pH 7.5. This can be determined according to the adsorption isotherm method described in N Pakiman et al: J Appl Sci 12, 1136-1141 (2012).

The alkali-stabilized Protein A domains comprise mutants of a parental Fc-binding domain of Staphylococcus Protein A (SpA), as defined by SEQ ID NO 51 or SEQ ID NO 52, wherein at least the asparagine or serine residue at the position corresponding to position 11 in SEQ ID NO:4-7 has been mutated to an amino acid selected from the group consisting of glutamic acid, lysine, tyrosine, threonine, phenylalanine, leucine, isoleucine, tryptophan, methionine, valine, alanine, histidine and arginine, such as an amino acid selected from the group consisting of glutamic acid and lysine. The amino acid residue at the position corresponding to position 40 in SEQ ID NO:4-7 may further be, or be mutated to, a valine. The alkali-stabilized Protein A domains may also comprise any mutations as described in the polypeptide and/or multimer embodiments above.

The invention uses a separation matrix comprising at least 15, such as 15-21 or 15-18 mg/ml Fc-binding ligands covalently coupled to a porous support, wherein the ligands comprise multimers of alkali-stabilized Protein A domains. These multimers can suitably be as disclosed in any of the embodiments described above or as specified below.

Such a matrix is useful for separation of immunoglobulins or other Fc-containing proteins and, due to the improved alkali stability of the polypeptides/multimers, the matrix will withstand highly alkaline conditions during cleaning, which is essential for long-term repeated use in a bioprocess separation setting. The alkali stability of the matrix can be assessed by measuring the immunoglobulin-binding capacity, typically using polyclonal human IgG, before and after incubation in alkaline solutions at a specified temperature, e.g. 22 +/- 2 °C. The incubation can e.g. be performed in 0.5 M or 1.0 M NaOH for a number of 15 min cycles, such as 100, 200 or 300 cycles, corresponding to a total incubation time of 25, 50 or 75 h. The IgG capacity of the matrix after 96-100 15 min incubation cycles or a total incubation time of 24 or 25 h in 0.5 M NaOH at 22 +/- 2 °C can be at least 80, such as at least 85, at least 90 or at least 95% of the IgG capacity before the incubation. The capacity of the matrix after a total incubation time of 24 h in 1.0 M NaOH at 22 +/- 2 °C can be at least 70, such as at least 80 or at least 90% of the IgG capacity before the incubation. The the 10% breakthrough dynamic binding capacity (Qb10%) for IgG at 2.4 min or 6 min residence time may e.g. be reduced by less than 20 % after incubation 31 h in 1.0 M aqueous NaOH at 22 +/- 2 C.

As the skilled person will understand, the expressed polypeptide or multimer should be purified to an appropriate extent before being immobilized to a support. Such purification methods are well known in the field, and the immobilization of protein-based ligands to supports is easily carried out using standard methods. Suitable methods and supports will be discussed below in more detail.

A conventional affinity separation matrix is often of organic nature and based on polymers that expose a hydrophilic surface to the aqueous media used, i.e. expose hydroxy (-OH), carboxy (-COOH), carboxamido (-$CONH_2$, possibly in N- substituted forms), amino (-$NH_2$, possibly in substituted form), oligo- or polyethylenoxy groups on their external and, if present, also on internal surfaces. The solid support is porous. The porosity can be expressed as a Kav or Kd value (the fraction of the pore volume available to a probe molecule of a particular size) measured by inverse size exclusion chromatography, e.g. according to the methods described in Gel Filtration Principles and Methods, Pharmacia LKB Biotechnology 1991, pp 6-13. Kav is determined as the ratio $(V_e-V_0)/(V_t-V_0)$, where Ve is the elution volume of a probe molecule (e.g. Dextran 110 kD), Vo is the void volume of the column (e.g. the elution volume of a high Mw void marker, such as raw dextran) and Vt is the total volume of the column. Kd can be determined as $(V_e-V_0)/V_i$, where $V_i$ is the elution volume of a salt (e.g. NaCl) able to access all the volume except the matrix volume (the volume occupied by the matrix polymer molecules). By definition, both Kd and Kav values always lie within the range 0 - 1. The Kav value can advantageously be 0.6 - 0.95, e.g. 0.7 - 0.90 or 0.6 - 0.8, as measured with dextran of Mw 110 kDa as a probe molecule. The Kd value as measured with dextran of Mw 110 kDa is 0.69-0.85 or 0.70-0.85. An advantage of this is that the support has a large fraction of pores able to accommodate both the polypeptides/multimers of the invention and immunoglobulins binding to the polypeptides/multimers and to provide mass transport of the immunoglobulins to and from the binding sites.

The polypeptides or multimers may be attached to the support via conventional coupling techniques utilising e.g. thiol, amino and/or carboxy groups present in the ligand. Bisepoxides, epichlorohydrin, CNBr, N-hydroxysuccinimide (NHS) etc are well-known coupling reagents. Between the support and the polypeptide/multimer, a molecule known as a spacer can be introduced, which improves the availability of the polypeptide/multimer and facilitates the chemical coupling of the polypeptide/multimer to the support. Depending on the nature of the polypeptide/multimer and the coupling conditions, the coupling may be a multipoint coupling (e.g. via a plurality of lysines) or a single point coupling (e.g. via a single cysteine). Alternatively, the polypeptide/multimer may be attached to the support by non-covalent bonding, such as physical adsorption or biospecific adsorption.

The amount of coupled polypeptide/multimer can be controlled by the concentration of polypeptide/multimer used in the coupling process, by the activation and coupling conditions used and/or by the pore structure of the support used. As a general rule the absolute binding capacity of the matrix increases with the amount of coupled polypeptide/multimer, at least up to a point where the pores become significantly constricted by the coupled polypeptide/multimer. Without being bound by theory, it appears though that for the Kd values recited for the support, the constriction of the pores by coupled ligand is of lower significance. The relative binding capacity per mg coupled polypeptide/multimer will decrease at high coupling levels, resulting in a cost-benefit optimum within the ranges specified above.

In certain embodiments the polypeptides or multimers are coupled to the support via thioether bonds. Methods for performing such coupling are well-known in this field and easily performed by the skilled person in this field using standard techniques and equipment. Thioether bonds are flexible and stable and generally suited for use in affinity chromatography. In particular when the thioether bond is via a terminal or near-terminal cysteine residue on the polypeptide or multimer, the mobility of the coupled polypeptide/multimer is enhanced which provides improved binding capacity and binding kinetics. In some embodiments the polypeptide/multimer is coupled via a C-terminal cysteine provided on the protein as described above. This allows for efficient coupling of the cysteine thiol to electrophilic groups, e.g. epoxide groups, halohydrin groups etc. on a support, resulting in a thioether bridge coupling.

The support comprises agarose. The supports used in the present invention can easily be prepared according to standard methods, such as inverse suspension gelation (S Hjertén: Biochim Biophys Acta 79(2), 393-398 (1964). Alternatively, the base matrices are commercially available products, such as crosslinked agarose beads sold under the name of SEPHAROSE™ FF (GE Healthcare). In an embodiment, which is especially advantageous for large-scale separations, the support has been adapted to increase its rigidity using the methods described in US6602990 or US7396467-and hence renders the matrix more suitable for high flow rates.

The agarose support, is crosslinked, such as with hydroxyalkyl ether crosslinks. Crosslinker reagents producing such crosslinks can be e.g. epihalohydrins like epichlorohydrin, diepoxides like butanediol diglycidyl ether, allylating reagents like allyl halides or allyl glycidyl ether. Crosslinking is beneficial for the rigidity of the support and improves the chemical stability. Hydroxyalkyl ether crosslinks are alkali stable and do not cause significant nonspecific adsorption.

Matrices in beaded or particle form can be used as a packed bed or in a suspended form. Suspended forms include those known as expanded beds and pure suspensions, in which the particles or beads are free to move. In case of monoliths, packed bed and expanded beds, the separation procedure commonly follows conventional chromatography with a concentration gradient. In case of pure suspension, batch-wise mode will be used.

The present invention discloses a method of isolating an immunoglobulin, wherein a separation matrix as disclosed above is used. The method may comprise the steps of:

> a) contacting a liquid sample comprising an immunoglobulin with a separation matrix as disclosed above,
> b) washing the separation matrix with a washing liquid,
> c) eluting the immunoglobulin from the separation matrix with an elution liquid, and
> d) cleaning the separation matrix with a cleaning liquid, which may comprise 0.1 - 1.0 M NaOH or KOH, such as 0.4 - 1.0 M NaOH or KOH.

Steps a) - d) may be repeated at least 10 times, such as at least 50 times or 50 - 200 times.

The invention also discloses a method of isolating an immunoglobulin, comprising the steps of:

a) providing a separation matrix comprising multimers of immunoglobulin-binding alkali-stabilized Protein A domains covalently coupled to a porous support,
b) contacting a liquid sample comprising an immunoglobulin with said separation matrix,
c) washing said separation matrix with a washing liquid,
d) eluting the immunoglobulin from the separation matrix with an elution liquid, and
e) cleaning the separation matrix with a cleaning liquid,

wherein said alkali-stabilized Protein A domains comprise mutants of a parental Fc-binding domain of Staphylococcus Protein A (SpA), as defined by SEQ ID NO 51 or SEQ ID NO 52, wherein the amino acid residues at positions 13 and 44 of SEQ ID NO 51 or 52 are asparagines and wherein at least the asparagine residue at position 3 of SEQ ID NO 51 or 52 has been mutated to an amino acid selected from the group consisting of glutamic acid, lysine, tyrosine, threonine, phenylalanine, leucine, isoleucine, tryptophan, methionine, valine, alanine, histidine and arginine.

The glutamine residue at position 1 of SEQ ID NO 51 or 52 may further be mutated to an alanine and/or the asparagine or glutamic acid residue at position 35 of SEQ ID NO 51 or 52 may be mutated to an alanine.

The separation matrix comprises at least 15 mg/ml of the multimers of immunoglobulin-binding alkali-stabilized Protein A domains covalently coupled to the porous support.

In step b) at least 40 mg immunoglobulin per ml separation matrix, such as at least 50 mg per ml, 40-90, 40-80, 40-70 or 40-60 mg per ml matrix is contacted with the separation matrix. A high degree of loading in step b) is highly desirable for the process economy and can be achieved due to the high dynamic binding capacity of the matrix.

In step e) the cleaning liquid may comprise at least 0.5 M NaOH, such as at least 0.7 M, at least 0.9 M or at least 1 M NaOH. It may also comprise 0.5-1.5 M NaOH, such as 0.5-1.1, 0.7-1.2 or 0.9-1.1 M NaOH. As an alternative to NaOH it is also possible to use KOH, or NaOH/KOH mixtures, at the same concentrations.

Steps b) - e) may be repeated at least 10 times, such as at least 50 times, at least 100 times, 50 - 200 times or 50-150 times. The duration of step e) may e.g. be at least 5 min, such as at least 10 min or 5-60 min, such as 5-30 min or 10-20 min.

The liquid sample may be a clarified cell broth, e.g. a clarified mammalian cell broth such as a CHO cell broth. In step d) the immunoglobulin may be recovered as an eluate comprising less than 2000 ppm, such as less than 1500 ppm or less than 1200 ppm host cell proteins, e.g. CHO cell proteins. In relative terms, the ratio of the host cell protein concentration in the liquid sample to the host cell concentration in the eluate may be at least 100, such as at least 200 or at least 300. A high clearance of host cell proteins, in either relative or absolute terms, as early as possible in the process is desirable to facilitate the further purification of the antibody.

If so desired, the method may further comprise a virus inactivation step. The virus inactivation can be performed after elution in the conventional way but it may also be performed when the immunoglobulin is present in the column, e.g. as disclosed in WO2015048330 or WO2015166072.

In step d) the elution liquid may e.g. have a pH of 2.5-5.0 or 3.0-5.0, such as 2.5-4.5, 3.0-4.5 or 3.2-4.5. The elution liquid may in some cases contain additives such as salts, amino acids, specific buffering agents etc. The elution may be performed by a distinct step change or by the application of a gradient, e.g. a pH gradient. Suitable elution conditions are described in e.g. WO2014159064, US8084032, US8853371, US20080167450, US20110144311, US20130096284, US20120238730, US20140018525, WO2013033517, US20140228548, WO2014159064, US20150093800.

The immunoglobulin may in particular comprise IgG1, IgG2 and/or IgG4. As shown in the examples, the current matrices bind these IgG classes.

The washing liquid may e.g. have a pH of 5-8. The washing liquid may comprise an additive for improving the washing efficiency, e.g. to improve the host cell protein clearance. Such additives are known in the art and may comprise one or more of a detergent, a water-miscible organic solvent, a chaotrope, arginine or an arginine derivative, calcium ions and tetraalkylammonium ions. The following documents describe suitable additives: US6127526, US6870034, US7820799, US7834162, US8263750, US7714111, US9284347, US20120283416, US20130197197, WO2014186350, WO2014192877, US20140094593, US20160108084 and US20160024147.

The multimers may be coupled to the support via thioether links as described above.

In step b) the pH may e.g. be 6-8. The residence time in this step may e.g. be 2-20 min, such as 2-10 min. Specific loading conditions are described e.g. in US4704366, US4801687, US4933435, EP2782925A1, US20140154270.

The porous support comprises cross-linked agarose particles having a volume-weighted median diameter (d50,v) of 56-70 micrometers and a dry solids weight of 55-80 mg/ml. The cross-linked agarose particles have a pore size corresponding to an inverse gel filtration chromatography Kd value of 0.69-0.85 for dextran of Mw 110 kDa.

In certain embodiments the method of the invention comprises the steps of:

a) providing a separation matrix comprising at least 15 mg/ml multimers of immunoglobulin-binding alkali-stabilized Protein A domains covalently coupled to a porous support,
b) contacting a liquid sample comprising an immunoglobulin with said separation matrix,
c) washing said separation matrix with a washing liquid,
d) eluting the immunoglobulin from the separation matrix with an elution liquid, and
e) cleaning the separation matrix with a cleaning liquid comprising at least 0.5 M NaOH,

wherein in step b) at least 40 mg immunoglobulin per ml separation matrix is contacted with said separation matrix.

In certain embodiments, the method comprises the steps of:

a) contacting a liquid sample comprising an immunoglobulin with a separation matrix as disclosed above,
b) washing said separation matrix with a washing liquid,
c) eluting the immunoglobulin from the separation matrix with an elution liquid, and
d) cleaning the separation matrix with a cleaning liquid, which can alternatively be called a cleaning-in-place (CIP) liquid, e.g. with a contact (incubation) time of at least 10 min.

The method may also comprise steps of, before step a), providing an affinity separation matrix according to any of the embodiments described above and providing a solution comprising an immunoglobulin and at least one other substance as a liquid sample and of, after step c), recovering the eluate and optionally subjecting the eluate to further separation steps, e.g. by anion or cation exchange chromatography, multimodal chromatography and/or hydrophobic interaction chromatography. Suitable compositions of the liquid sample, the washing liquid and the elution liquid, as well as the general conditions for performing the separation are well known in the art of affinity chromatography and in particular in the art of Protein A chromatography. The liquid sample comprising an Fc-containing protein and at least one other substance may comprise host cell proteins (HCP), such as CHO cell, E Coli or yeast proteins. Contents of CHO cell and E Coli proteins can conveniently be determined by immunoassays directed towards these proteins, e.g. the CHO HCP or E Coli HCP ELISA kits from Cygnus Technologies. The host cell proteins or CHO cell/E Coli proteins may be desorbed during step b).

The elution may be performed by using any suitable solution used for elution from Protein A media. This can e.g. be a solution or buffer with pH 5 or lower, such as pH 2.5 - 5 or 3 - 5. It can also in some cases be a solution or buffer with pH 11 or higher, such as pH 11 - 14 or pH 11 - 13. In some embodiments the elution buffer or the elution buffer gradient comprises at least one mono- di- or trifunctional carboxylic acid or salt of such a carboxylic acid. In certain embodiments the elution buffer or the elution buffer gradient comprises at least one anion species selected from the group consisting of acetate, citrate, glycine, succinate, phosphate, and formiate.

In some embodiments, the cleaning liquid is alkaline, such as with a pH of 13 - 14. Such solutions provide efficient cleaning of the matrix, in particular at the upper end of the interval

In certain embodiments, the cleaning liquid comprises 0.1- 2.0 M NaOH or KOH, such as 0.5 - 2.0 or 0.5 - 1.0 M NaOH or KOH. These are efficient cleaning solutions, and in particular so when the NaOH or KOH concentration is above 0.1 M or at least 0.5 M. The high stability of the polypeptides of the invention enables the use of such strongly alkaline solutions.

The method may also include a step of sanitizing the matrix with a sanitization liquid, which may e.g. comprise a peroxide, such as hydrogen peroxide and/or a peracid, such as peracetic acid or performic acid.

In some embodiments, steps a) - d) are repeated at least 10 times, such as at least 50 times, 50 - 200, 50-300 or 50-500 times. This is important for the process economy in that the matrix can be re-used many times.

Steps a) - c) can also be repeated at least 10 times, such as at least 50 times, 50 - 200, 50-300 or 50-500 times, with step d) being performed after a plurality of instances of step c), such that step d) is performed at least 10 times, such as at least 50 times. Step d) can e.g. be performed every second to twentieth instance of step c).

Examples

Mutagenesis of protein

Site-directed mutagenesis was performed by a two-step PCR using oligonucleotides coding for the mutations. As template a plasmid containing a single domain of either Z, B or C was used. The PCR fragments were ligated into an E. coli expression vector. DNA sequencing was used to verify the correct sequence of inserted fragments.

To form multimers of mutants an Acc I site located in the starting codons (GTA GAC) of the B, C or Z domain was used, corresponding to amino acids VD. The vector for the monomeric domain was digested with Acc I and phosphatase treated. Acc I sticky-ends primers were designed, specific for each variant, and two overlapping PCR products were generated from each template. The PCR products were purified and the concentration was estimated by comparing the PCR products on a 2% agarose gel. Equal amounts of the pair wise PCR products were hybridized (90°C -> 25°C in 45min) in ligation buffer. The resulting product consists approximately to ¼ of fragments likely to be ligated into an Acc I site (correct PCR fragments and/or the digested vector). After ligation and transformation colonies were PCR screened to identify constructs containing the desired mutant. Positive clones were verified by DNA sequencing.

Construct expression and purification

The constructs were expressed in the bacterial periplasm by fermentation of *E. coli* K12 in standard media. After fermentation the cells were heat-treated to release the periplasm content into the media. The constructs released into the medium were recovered by microfiltration with a membrane having a 0.2 μm pore size.

Each construct, now in the permeate from the filtration step, was purified by affinity. The permeate was loaded onto a chromatography medium containing immobilized IgG (IgG Sepharose 6FF, GE Healthcare). The loaded product was washed with phosphate buffered saline and eluted by lowering the pH.

The elution pool was adjusted to a neutral pH (pH 8) and reduced by addition of dithiothreitol. The sample was then loaded onto an anion exchanger. After a wash step the construct was eluted in a NaCl gradient to separate it from any contaminants. The elution pool was concentrated by ultrafiltration to 40-50 mg/ml. It should be noted that the successful affinity purification of a construct on an immobilized IgG medium indicates that the construct in question has a high affinity to IgG.

The purified ligands were analyzed with RPC LC-MS to determine the purity and to ascertain that the molecular weight corresponded to the expected (based on the amino acid sequence).

Example 1

The purified monomeric ligands listed in Table 1, further comprising for SEQ ID NO 8-16, 23-28 and 36-48 an AQGT leader sequence at the N-terminus and a cysteine at the C terminus, were immobilized on Biacore CM5 sensor chips (GE Healthcare, Sweden), using the amine coupling kit of GE Healthcare (for carbodiimide coupling of amines on the carboxymethyl groups on the chip) in an amount sufficient to give a signal strength of about 200-1500 RU in a Biacore surface plasmon resonance (SPR) instrument (GE Healthcare, Sweden). To follow the IgG binding capacity of the immobilized surface 1mg/ml human polyclonal IgG (Gammanorm) was flowed over the chip and the signal strength (proportional to the amount of binding) was noted. The surface was then cleaned-in-place (CIP), i.e. flushed with 500mM NaOH for 10 minutes at room temperature (22 +/- 2°C). This was repeated for 96-100 cycles and the immobilized ligand alkaline stability was followed as the remaining IgG binding capacity (signal strength) after each cycle. The results are shown in Table 1 and indicate that at least the ligands Zvar(N11K)1, Zvar(N11E)1, Zvar(N11Y)1, Zvar(N11T)1, Zvar(N11F)1, Zvar(N11L)1, Zvar(N11W)1, ZN11I)1, Zvar(N11M)1, Zvar(N11V)1, Zvar(N11A)1, Zvar(N11H1), Zvar(N11R)1, Zvar(N11E,Q32A)1, Zvar(N11E,Q32E,Q40E)1 and Zvar(N11E,Q32E,K50R)1, Zvar(Q9A,N11E,N43A)1, Zvar(Q9A,N11E,N28A,N43A)1, Zvar(Q9A,N11E,Q40V,A42K,N43E,L44I)1, Zvar(Q9A,N11E,Q40V,A42K,N43A,L44I)1, Zvar(Q9A,N11E,N28A,Q40V,A42K,N43A,L441)1, Zvar(N11K,H18K,S33K,D37E,A42R,N43A,L44I,K50R,L51Y)1, Zvar(Q9A,N11K,H18K,S33K,D37E,A42R,N43A,L44I,K50R,L51Y)1, Zvar(N11K, H18K, D37E, A42R, N43A, L44I)1, Zvar(Q9A, N11K, H18K, D37E, A42R, N43A, L44I)1 and Zvar(Q9A, N11K, H18K, D37E, A42R, N43A, L44I, K50R)1, as well as the varieties of Zvar(Q9A,N11E,Q40V,A42K,N43A,L44I)1 having G,S,Y,Q,T,N,F,L,W,I,M,V,D,E,H,R or K in position 29, the varieties of Zvar(Q9A,N11E,Q40V,A42K,N43A,L44I)1 having F,Y,W,K or R in position 53 and the varieties of Zvar(Q9A,N11E,Q40V,A42K,N43A,L44I)1 where Q9, Q40, A42 or N43 has been deleted, have an improved alkali stability compared to the parental structure Zvar1, used as the reference. Further, the ligands B(Q9A,N11E,Q40V,A42K,N43A,L44I)1 and C(Q9A,N11E,E43A)1 have an improved stability compared to the parental B and C domains, used as references.

Table 1. Monomeric ligands, evaluated by Biacore (0.5 M NaOH).

| Ligand | Sequence | Capacity after 96-100 cycles | Reference capacity after 96-100 cycles | Capacity relative to reference |
|---|---|---|---|---|
| Zvar(N11E,Q32A)1 | SEQ ID NO 12 | 57% | 55% | 1.036 |
| Zvar(N11E)1 | SEQ ID NO 13 | 59% | 55% | 1.073 |
| Zvar(N11E,Q32E,Q40E)1 | SEQ ID NO 14 | 52% | 51% | 1.020 |

(continued)

| Ligand | Sequence | Capacity after 96-100 cycles | Reference capacity after 96-100 cycles | Capacity relative to reference |
|---|---|---|---|---|
| Zvar(N1 1E,Q32E,K50R)1 | SEQ ID NO 15 | 53% | 51% | 1.039 |
| Zvar(N11K)1 | SEQ ID NO 16 | 62% | 49% | 1.270 |
| Zvar(N11Y)1 | SEQ ID NO 38 | 55% | 46% | 1.20 |
| Zvar(N11T)1 | SEQ ID NO 39 | 50% | 46% | 1.09 |
| Zvar(N11F)1 | SEQ ID NO 40 | 55% | 46% | 1.20 |
| Zvar(N11L)1 | SEQ ID NO 41 | 57% | 47% | 1.21 |
| Zvar(N11W)1 | SEQ ID NO 42 | 57% | 47% | 1.21 |
| Zvar(N11I)1 | SEQ ID NO 43 | 57% | 47% | 1.21 |
| Zvar(N11M)1 | SEQ ID NO 44 | 58% | 46% | 1.26 |
| Zvar(N11V)1 | SEQ ID NO 45 | 56% | 46% | 1.22 |
| Zvar(N11A) 1 | SEQ ID NO 46 | 58% | 46% | 1.26 |
| Zvar(N11H) 1 | SEQ ID NO 47 | 57% | 46% | 1.24 |
| Zvar(N11R)1 | SEQ ID NO 48 | 59% | 46% | 1.28 |
| Zvar(Q9A,N11E,N43A)1 | SEQ ID NO 8 | 70% | 47% | 1.49 |
| Zvar(Q9A,N1 1E,N28A,N43A)1 | SEQ ID NO 9 | 68% | 47% | 1.45 |
| Zvar(Q9A,N11E,Q40V,A42K, N43E,L44I)1 | SEQ ID NO 10 | 67% | 47% | 1.43 |
| Zvar(Q9A,N11E,Q40V,A42K, N43A,L44I)1 1 | SEQ ID NO 11 | 66% | 47% | 1.40 |
| Zvar(Q9A,N11E,N28A,Q40V, A42K,N43A,L44I)1 | SEQ ID NO 24 | 65% | 48% | 1.35 |
| Zvar(N11K,H18K,S33K,D37E, A42R,N43A,L44I,K50R,L51Y)1 | SEQ ID NO 23 | 67% | 46% | 1.46 |
| Zvar(Q9A,N 11K,H18K,S33K, D37E,A42R,N43A,L44I,K50R, L51Y)1 | SEQ ID NO 25 | 59% | 46% | 1.28 |
| Zvar(N11K, H18K, D37E, A42R, N43A, L441)1 | SEQ ID NO 26 | 59% | 45% | 1.31 |

(continued)

| Ligand | Sequence | Capacity after 96-100 cycles | Reference capacity after 96-100 cycles | Capacity relative to reference |
|---|---|---|---|---|
| Zvar(Q9A, N11K, H18K, D37E, A42R, N43A, L44I)1 | SEQ ID NO 27 | 63% | 45% | 1.40 |
| Zvar(Q9A, N11K, H18K, D37E, A42R, N43A, L44I, K50R)1 | SEQ ID NO 28 | 67% | 45% | 1.49 |
| B(Q9A,N11E,Q40V,A42K,N43A, L44I)1 | SEQ ID NO 36 | 39% | 35% | 1.11 |
| C(Q9A,N11E,E43A)1 | SEQ ID NO 37 | 60% | 49% | 1.22 |
| Zvar(Q9A,N11E,**A29G**,Q40V, A42K,N43A,L44I)1 | SEQ ID NO 54 | 69% | 48% | 1.44 |
| Zvar(Q9A,N11E,**A29S**,Q40V, A42K,N43A,L44I)1 | SEQ ID NO 55 | 66% | 48% | 1.38 |
| Zvar(Q9A,N11E,**A29Y**,Q40V, A42K,N43A,L44I)1 | SEQ ID NO 56 | 61% | 48% | 1.27 |
| Zvar(Q9A,N11E,**A29Q**,Q40V, A42K,N43A,L44I)1 | SEQ ID NO 57 | 60% | 47% | 1.28 |
| Zvar(Q9A,N11E,**A29T**,Q40V, A42K,N43A,L44I) 1 | SEQ ID NO 58 | 60% | 47% | 1.28 |
| Zvar(Q9A,N11E,**A29N**,Q40V, A42K,N43A,L44I)1 | SEQ ID NO 59 | 61% | 47% | 1.30 |
| Zvar(Q9A,N11E,**A29F**,Q40V, A42K,N43A,L44I)1 | SEQ ID NO 60 | 62% | 46% | 1.35 |
| Zvar(Q9A,N11E,**A29L**,Q40V, A42K,N43A,L44I)1 | SEQ ID NO 61 | 61% | 46% | 1.33 |
| Zvar(Q9A,N11E,**A29W**,Q40V, A42K,N43A,L44I)1 | SEQ ID NO 62 | 60% | 46% | 1.30 |
| Zvar(Q9A,N11E,**A29I**,Q40V, A42K,N43A,L44I)1 | SEQ ID NO 63 | 58% | 47% | 1.23 |
| Zvar(Q9A,N11E,**A29M**,Q40V, A42K,N43A,L44I)1 | SEQ ID NO 64 | 62% | 47% | 1.32 |
| Zvar(Q9A,N11E,**A29V**,Q40V, A42K,N43A,L44I)1 | SEQ ID NO 65 | 62% | 47% | 1.32 |
| Zvar(Q9A,N11E,**A29D**,Q40V, A42K,N43A,L44I)1 | SEQ ID NO 66 | 56% | 47% | 1.19 |
| Zvar(Q9A,N11E,**A29E**,Q40V, A42K,N43A,L44I)1 | SEQ ID NO 67 | 57% | 47% | 1.21 |
| Zvar(Q9A,N11E,**A29H**,Q40V, A42K,N43A,L44I) 1 | SEQ ID NO 68 | 57% | 47% | 1.21 |
| Zvar(Q9A,N11E,**A29R**,Q40V, A42K,N43A,L44I)1 | SEQ ID NO 69 | 58% | 46% | 1.26 |
| Zvar(Q9A,N11E,**A29K**,Q40V, A42K,N43A,L44I)1 | SEQ ID NO 70 | 59% | 46% | 1.28 |
| Zvar(Q9A,N11E,Q40V,A42K, N43A,L44I,**D53F**) 1 | SEQ ID NO 71 | 58% | 46% | 1.26 |

(continued)

| Ligand | Sequence | Capacity after 96-100 cycles | Reference capacity after 96-100 cycles | Capacity relative to reference |
|---|---|---|---|---|
| Zvar(Q9A,N11E,Q40V,A42K, N43A,L44I,**D53Y**)1 | SEQ ID NO 72 | 59% | 46% | 1.28 |
| Zvar(Q9A,N11E,Q40V,A42K, N43A,L44I,**D53W**) 1 | SEQ ID NO 73 | 62% | 46% | 1.35 |
| Zvar(Q9A,N11E,Q40V,A42K, N43A,L44I,**D53K**)1 | SEQ ID NO 74 | 65% | 46% | 1.41 |
| Zvar(Q9A,N11E,Q40V,A42K, N43A,L44I,**D53R**)1 | SEQ ID NO 75 | 60% | 46% | 1.30 |
| Zvar(**Q9del**,N11E,Q40V,A42K, N43A,L44I)1 | SEQ ID NO 76 | 60% | 46% | 1.30 |
| Zvar(Q9A,N11E,**Q40del**,A42K, N43A,L44I)1 | SEQ ID NO 77 | 59% | 46% | 1.28 |
| Zvar(Q9A,N11E,Q40V,**A42del**, N43A,L44I)1 | SEQ ID NO 78 | 57% | 46% | 1.24 |
| Zvar(Q9A,N11E,Q40V,A42K, **N43del**,L44I) 1 | SEQ ID NO 79 | 55% | 46% | 1.20 |

The Biacore experiment can also be used to determine the binding and dissociation rates between the ligand and IgG. This was used with the set-up as described above and with an IgG1 monoclonal antibody as probe molecule. For the reference Zvar1, the on-rate ($10^5$ M$^{-1}$s$^{-1}$) was 3.1 and the off-rate ($10^5$ s$^{-1}$) was 22.1, giving an affinity (off-rate/on-rate) of 713 pM. For Zvar(Q9A,N11E,Q40V,A42K,N43A,L44I)1 (SEQ ID NO. 11), the on-rate was 4.1 and the off-rate 43.7, with affinity 1070 pM. The IgG affinity was thus somewhat higher for the mutated variant.

Example 2

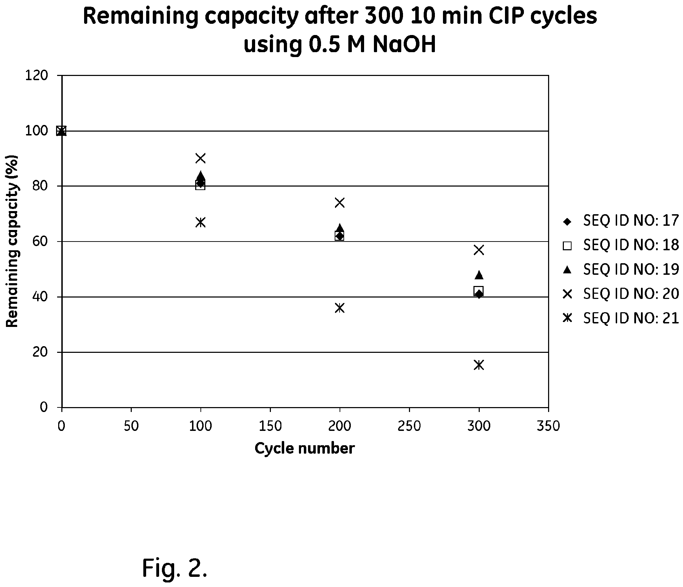

The purified dimeric, tetrameric and hexameric ligands listed in Table 2 were immobilized on Biacore CM5 sensor chips (GE Healthcare, Sweden), using the amine coupling kit of GE Healthcare (for carbodiimide coupling of amines on the carboxymethyl groups on the chip) in an amount sufficient to give a signal strength of about 200-1500 RU in a Biacore instrument (GE Healthcare, Sweden) . To follow the IgG binding capacity of the immobilized surface 1mg/ml human polyclonal IgG (Gammanorm) was flowed over the chip and the signal strength (proportional to the amount of binding) was noted. The surface was then cleaned-in-place (CIP), i.e. flushed with 500mM NaOH for 10 minutes at room temperature (22 +/- 2°C). This was repeated for 300 cycles and the immobilized ligand alkaline stability was followed as the remaining IgG binding capacity (signal strength) after each cycle. The results are shown in Table 2 and in Fig. 2 and indicate that at least the ligands Zvar(Q9A,N11E,N43A)4, Zvar(Q9A,N11E,N28A,N43A)4, Zvar(Q9A,N11E,Q40V,A42K,N43E,L44I)4 and Zvar(Q9A,N11E,Q40V,A42K,N43A,L44I)4, Zvar(Q9A,N11E,D37E,Q40V,A42K,N43A,L44I)4 and Zvar(Q9A,N11E,D37E,Q40V,A42R,N43A,L44I)4 have an improved alkali stability compared to the parental structure Zvar4, which was used as a reference. The hexameric ligand Zvar(Q9A,N11E,Q40V,A42K,N43A,L44I)6 also has improved alkali stability compared to the parental structure Zvar6, used as a reference. Further, Zvar(Q9A,N11E,Q40V,A42K,N43A,L44I) dimers with deletions of a) D2,A3,K4; b) K58,V1,D2; c) P57,K58,V1,D2,A3; d) K4,F5,D6,K7,E8; e) A56,P57,K58; V1,D2,A3 or f) V1,D2,A3,K4,F5,D6,K7,E8 from the linker region between the two monomer units have improved alkali stability compared to the parental structure Zvar2, used as a reference. Also Zvar(Q9A,N11E,Q40V,A42K,N43A,L44I) dimers with an insertion of YEDG between K58 and VI in the linker region have improved alkali stability compared to Zvar2.

Table 2. Dimeric, tetrameric and hexameric ligands, evaluated by Biacore (0.5M NaOH).

| Ligand | SEQ ID NO: | Remaining capacity 100 cycles (%) | Capacity relative to ref. 100 cycles | Remaining capacity 200 cycles (%) | Capacity relative to ref. 200 cycles | Remaining capacity 300 cycles (%) | Capacity relative to ref. 300 cycles |
|---|---|---|---|---|---|---|---|
| Zvar4 | 21 | 67 | 1 | 36 | 1 | 16 | 1 |
| Zvar(Q9A,N11E, N43A)4 | 17 | 81 | 1.21 | 62 | 1.72 | 41 | 2.56 |
| Zvar(Q9A,N11E, N28A,N 43A)4 | 18 | 80 | 1.19 | 62 | 1.72 | 42 | 2.62 |
| Zvar(Q9A,N11E, Q40V,A 42K,N43E, L44I)4 | 19 | 84 | 1.25 | 65 | 1.81 | 48 | 3.00 |
| Zvar(Q9A,N11E, Q40V,A 42K,N43A, L44I)4 | 20 | 90 | 1.34 | 74 | 2.06 | 57 | 3.56 |
| Zvar(Q9A,N11E, N28A,Q 40V,A42K, N43A,L44I)4 | 32 | 84 | 1.24 | Not tested | Not tested | Not tested | Not tested |
| Zvar(Q9A,N11E, Q40V,A 42K,N43A, L44I)6 | 33 | 87 | 1.30 | Not tested | Not tested | Not tested | Not tested |
| Zvar(Q9A,N11E, D37E,Q 40V,A42K, N43A,L441)4 | 34 | 81 | 1.13 | Not tested | Not tested | Not tested | Not tested |
| Zvar(Q9A,N11E, D37E,Q 40V,A42R, N43A,L441)4 | 35 | 84 | 1.17 | Not tested | Not tested | Not tested | Not tested |
| Zvar(Q9A,N11E, Q40V,A 42K,N43A, L44I)2 with D2, A3 and K4 in linker deleted | 80 | 70 | 1.27 | Not tested | Not tested | Not tested | Not tested |
| Zvar(Q9A,N11E, Q40V,A 42K,N43A, L44I)2 with K58, V1 and D2 in linker deleted | 81 | 76 | 1.38 | Not tested | Not tested | Not tested | Not tested |
| Zvar(Q9A,N11E, Q40V,A 42K,N43A, L44I)2 with P57, K58, V1, D2 and A3 in linker deleted | 82 | 74 | 1.35 | Not tested | Not tested | Not tested | Not tested |
| Zvar(Q9A,N11E, Q40V,A 42K,N43A, L44I)2 with K4, F5, D6, K7 and E8 in linker deleted | 83 | 70 | 1.30 | Not tested | Not tested | Not tested | Not tested |

(continued)

| Ligand | SEQ ID NO: | Remaining capacity 100 cycles (%) | Capacity relative to ref. 100 cycles | Remaining capacity 200 cycles (%) | Capacity relative to ref. 200 cycles | Remaining capacity 300 cycles (%) | Capacity relative to ref. 300 cycles |
|---|---|---|---|---|---|---|---|
| Zvar(Q9A,N11E, Q40V,A 42K,N43A, L44I)2 with A56, P57 and K58 in linker deleted | 84 | 68 | 1.26 | Not tested | Not tested | Not tested | Not tested |
| Zvar(Q9A,N11E, Q40V,A 42K,N43A, L44I)2 with V1, D2 and A3 in linker deleted | 85 | 75 | 1.39 | Not tested | Not tested | Not tested | Not tested |
| Zvar(Q9A,N11E, Q40V,A 42K,N43A, L44I)2 with V1, D2, A3, K4, F5, D6, K7 and E8 in linker deleted | 86 | 62 | 1.13 | Not tested | Not tested | Not tested | Not tested |
| Zvar(Q9A,N11E, Q40V,A 42K,N43A, L44I)2 with YEDG inserted in linker between K58 and VI | 87 | 72 | 1.31 | Not tested | Not tested | Not tested | Not tested |
| Zvar2 | 88 | 55 | 1 | Not tested | Not tested | Not tested | Not tested |

Example 3

Example 2 was repeated with 100 CIP cycles of three ligands using 1 M NaOH instead of 500 mM as in Example 2. The results are shown in Table 3 and show that all three ligands have an improved alkali stability also in 1M NaOH, compared to the parental structure Zvar4 which was used as a reference.

Table 3. Tetrameric ligands, evaluated by Biacore (1M NaOH).

| Ligand | Sequence | Remaining capacity 100 cycles (%) | Capacity relative to ref. 100 cycles |
|---|---|---|---|
| Zvar4 | SEQ ID NO 21 | 27 | 1 |
| Zvar(Q9A,N11E,N28A,N43A)4 | SEQ ID NO 18 | 55 | 2.04 |
| Zvar(Q9A,N11E,Q40V,A42K, N43E,L44I)4 | SEQ ID NO 19 | 54 | 2.00 |
| Zvar(Q9A,N11E,Q40V,A42K, N43A,L44I)4 | SEQ ID NO 20 | 56 | 2.07 |

Example 4

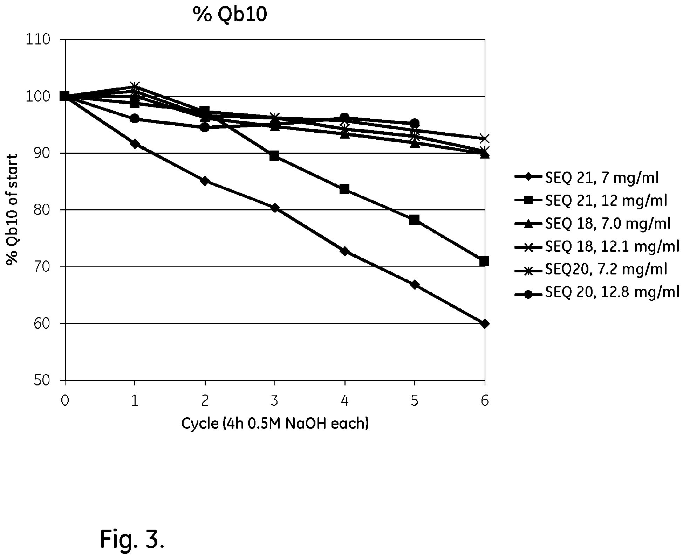

The purified tetrameric ligands of Table 2 (all with an additional N-terminal cysteine) were immobilized on agarose beads using the methods described below and assessed for capacity and stability. The results are shown in Table 4 and Fig. 3.

Table 4. Matrices with tetrametric ligands, evaluated in columns (0.5 M NaOH).

| Ligand | SEQ ID NO. | Ligand content (mg/ml) | Initial IgG capacity Qb10 (mg/ml) | Remainin gIgG capacity Qb10 after six 4 h cycles (mg/ml) | Remainin gIgG capacity after six 4 h cycles (%) | Capaci ty retenti on relative to ref. after six 4 h cycles |
|---|---|---|---|---|---|---|
| Zvar4 | 21 | 7 | 52.5 | 36.5 | 60 | 1 |
| Zvar4 | 21 | 12 | 61.1 | 43.4 | 71 | 1 |
| Zvar(Q9A, N11E,N28A, N43A)4 | 18 | 7.0 | 49.1 | 44.1 | 90 | 1.50 |
| Zvar(Q9A, N11E,N28A, N43A)4 | 18 | 12.1 | 50.0 | 46.2 | 93 | 1.31 |
| Zvar(Q9A, N11E,Q40V, A42K,N43A, L44I)4 | 20 | 7.2 | 49.0 | 44.2 | 90 | 1.50 |
| Zvar(Q9A, N11E,Q40V, A42K,N43A, L44I)4 | 20 | 12.8 | 56.3 | 53.6 | 95 | 1.34 |
| Zvar(N11K, H18K,S33K, D37E,A42R, N43A ,L44I, K50R,L51Y)4 | 30 | 9.7 | 56.3 | 52.0 | 92 | 1.53 |
| Zvar(Q9A, N11K,H18K, D37E,A42R)4 | 31 | 10.8 | 56.9 | 52.5 | 92 | 1.30 |

Activation

The base matrix used was rigid cross-linked agarose beads of 85 micrometers (volume-weighted, d50V) median diameter, prepared according to the methods of US6602990, and with a pore size corresponding to an inverse gel filtration chromatography Kav value of 0.70 for dextran of Mw 110 kDa, according to the methods described in Gel Filtration Principles and Methods, Pharmacia LKB Biotechnology 1991, pp 6-13.

25 mL (g) of drained base matrix, 10.0 mL distilled water and 2.02 g NaOH (s) was mixed in a 100 mL flask with mechanical stirring for 10 min at 25°C. 4.0 mL of epichlorohydrin was added and the reaction progressed for 2 hours. The activated gel was washed with 10 gel sediment volumes (GV) of water.

Coupling

To 20 mL of ligand solution (50 mg/mL) in a 50 ml Falcon tube, 169 mg $NaHCO_3$, 21 mg $Na_2CO_3$, 175 mg NaCl and 7 mg EDTA, was added. The Falcon tube was placed on a roller table for 5-10 min, and then 77 mg of DTE was added. Reduction proceeded for >45 min. The ligand solution was then desalted on a PD10 column packed with Sephadex G-25. The ligand content in the desalted solution was determined by measuring the 276 nm UV absorption.

The activated gel was washed with 3-5 GV {0.1 M phosphate/1 mM EDTA pH 8.6} and the ligand was then coupled according to the method described in US6399750. All buffers used in the experiments had been degassed by nitrogen gas for at least 5-10 min. The ligand content of the gels could be controlled by varying the amount and concentration of the ligand solution.

After immobilization the gels were washed 3xGV with distilled water. The gels + 1 GV {0.1 M phosphate/1 mM EDTA/10% thioglycerol pH 8.6} was mixed and the tubes were left in a shaking table at room temperature overnight.

The gels were then washed alternately with 3xGV {0.1 M TRIS/0.15 M NaCl pH 8.6} and 0.5 M HAc and then 8-10xGV with distilled water. Gel samples were sent to an external laboratory for amino acid analysis and the ligand content (mg/ml gel) was calculated from the total amino acid content.

Protein

Gammanorm 165 mg/ml (Octapharma), diluted to 2mg/ml in Equilibration buffer.

Equilibration buffer

PBS Phosphate buffer 10 mM + 0.14 M NaCl + 0.0027 M KCl, pH 7,4 (Medicago)

Adsorption buffer

PBS Phosphate buffer 10 mM + 0.14 M NaCl + 0.0027 M KCl, pH 7,4 (Medicago)

Elution buffers

100 mM acetate pH 2.9

Dynamic binding capacity

2 ml of resin was packed in TRICORN™ 5 100 columns. The breakthrough capacity was determined with an ÄKTAExplorer 10 system at a residence time of 6 minutes (0.33 ml/min flow rate). Equilibration buffer was run through the bypass column until a stable baseline was obtained. This was done prior to auto zeroing. Sample was applied to the column until a 100% UV signal was obtained. Then, equilibration buffer was applied again until a stable baseline was obtained.

Sample was loaded onto the column until a UV signal of 85% of maximum absorbance was reached. The column was then washed with 5 column volumes (CV) equilibration buffer at flow rate 0.5ml/min. The protein was eluted with 5 CV elution buffer at a flow rate of 0.5 ml/min. Then the column was cleaned with 0.5M NaOH at flow rate 0.2 ml/min and re-equilibrated with equilibration buffer.

For calculation of breakthrough capacity at 10%, the equation below was used. That is the amount of IgG that is loaded onto the column until the concentration of IgG in the column effluent is 10% of the IgG concentration in the feed.

$$q_{10\%} = \frac{C_0}{V_C}\left[V_{app} - V_{sys} - \int_{V_{sys}}^{V_{app}} \frac{A(V) - A_{sub}}{A_{100\%} - A_{sub}} * dv\right]$$

$A_{100\%}$ = 100% UV signal;

$A_{sub}$ = absorbance contribution from non-binding IgG subclass;

$A(V)$ = absorbance at a given applied volume;

$V_c$ = column volume;

$V_{app}$ = volume applied until 10% breakthrough;

$V_{sys}$ = system dead volume;

$C_0$ = feed concentration.

The dynamic binding capacity (DBC) at 10% breakthrough was calculated. The dynamic binding capacity (DBC) was calculated for 10 and 80% breakthrough.

CIP - 0.5 M NaOH

The 10% breakthrough DBC (Qb10) was determined both before and after repeated exposures to alkaline cleaning solutions. Each cycle included a CIP step with 0.5 M NaOH pumped through the column at a rate of 0.5/min for 20 min, after which the column was left standing for 4 h. The exposure took place at room temperature (22 +/- 2°C). After this incubation, the column was washed with equilibration buffer for 20 min at a flow rate of 0.5 ml/min. Table 4 shows the remaining capacity after six 4 h cycles (i.e. 24 h cumulative exposure time to 0.5 M NaOH), both in absolute numbers and relative to the initial capacity.

Example 5

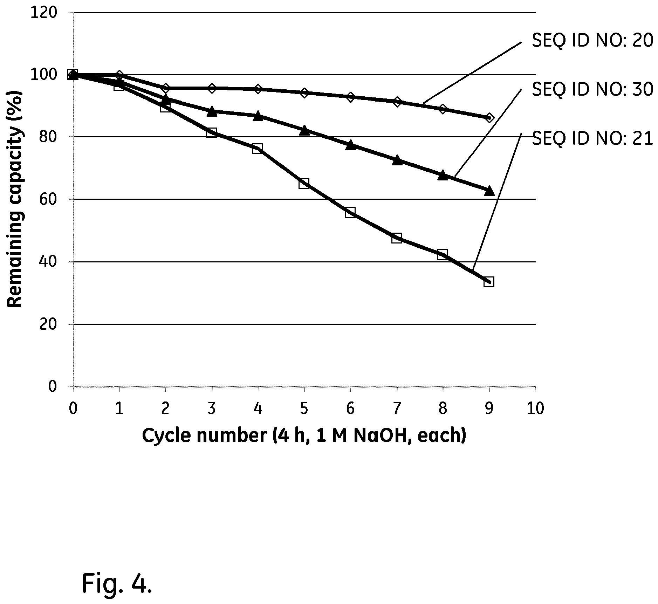

Example 4 was repeated with the tetrameric ligands shown in Table 5, but with 1.0 M NaOH used in the CIP steps instead of 0.5 M. The results are shown in Table 5 and in Fig. 4.

Table 5. Matrices with tetrametric ligands, evaluated in columns - 1.0 M NaOH.

| Ligand | SEQ ID NO. | Ligand content (mg/ml) | Initial IgG capacity Qb10 (mg/ml) | Remainin gIgG capacity Qb10 after six 4 h cycles (mg/ml) | Remainin gIgG capacity after six 4 h cycles (%) | Capaci ty retenti on relative to ref. after six 4 h cycles |
|---|---|---|---|---|---|---|
| Zvar4 | 21 | 12 | 60.1 | 33.5 | 56 | 1 |
| Zvar(Q9A, N11E,Q40V, A42K,N43A, L44I)4 | 20 | 12.8 | 60.3 | 56.0 | 93 | 1.67 |
| Zvar(N11K, H18K,S33K, D37E,A42R, N43A ,L44I, K50R,L51Y)4 | 30 | 9.7 | 62.1 | 48.1 | 77 | 1.44 |

Example 6

Base matrices

The base matrices used were a set of rigid cross-linked agarose bead samples of 59-93 micrometers (volume-weighted, d50V) median diameter (determined on a Malvern Mastersizer 2000 laser diffraction instrument), prepared according to the methods of US6602990 and with a pore size corresponding to an inverse gel filtration chromatography Kd value of 0.62-0.82 for dextran of Mw 110 kDa, according to the methods described above, using HR10/30 columns (GE Healthcare) packed with the prototypes in 0.2 M NaCl and with a range of dextran fractions as probe molecules (flow rate 0.2 ml/min). The dry weight of the bead samples ranged from 53 to 86 mg/ml, as determined by drying 1.0 ml drained filter cake samples at 105 °C over night and weighing.

Table 6. Base matrix samples

| Base matrix | Kd | d50v (μm) | Dry weight (mg/ml) |
|---|---|---|---|
| A18 | 0.704 | 59.0 | 56.0 |
| A20 | 0.70 | 69.2 | 55.8 |
| A27 | 0.633 | 87.2 | 74.2 |
| A28 | 0.638 | 67.4 | 70.2 |
| A29 | 0.655 | 92.6 | 57.5 |
| A32 | 0.654 | 73.0 | 70.5 |
| A33 | 0.760 | 73.1 | 55.5 |

(continued)

| Base matrix | Kd | d50v (μm) | Dry weight (mg/ml) |
|---|---|---|---|
| A38 | 0.657 | 70.9 | 56.2 |
| A39 | 0.654 | 66.0 | 79.1 |
| A40 | 0.687 | 64.9 | 74.9 |
| A41 | 0.708 | 81.7 | 67.0 |
| A42 | 0.638 | 88.0 | 59.4 |
| A43 | 0.689 | 87.5 | 77.0 |
| A45 | 0.670 | 56.6 | 66.0 |
| A52 | 0.620 | 53.10 | 63.70 |
| A53 | 0.630 | 52.6 | 86.0 |
| A54 | 0.670 | 61.3 | 75.3 |
| A55 | 0.640 | 62.0 | 69.6 |
| A56 | 0.740 | 61.0 | 56.0 |
| A56-2 | 0.740 | 51.0 | 56.0 |
| A62a | 0.788 | 48.8 | 70.1 |
| A62b | 0.823 | 50.0 | 46.9 |
| A63a | 0.790 | 66.8 | 59.6 |
| A63b | 0.765 | 54.0 | 79.0 |
| A65a | 0.796 | 58.0 | 60.0 |
| A65b | 0.805 | 57.3 | 46.0 |
| B5 | 0.793 | 69.0 | 84.4 |
| C1 | 0.699 | 71.0 | 73.4 |
| C2 | 0.642 | 66.5 | 81.1 |
| C3 | 0.711 | 62.0 | 82.0 |
| C4 | 0.760 | 62.0 | 82.0 |
| H31 | 0.717 | 82.0 | 59.0 |
| H35 | 0.710 | 81.1 | 61.0 |
| H40 | 0.650 | 52.8 | 65.0 |
| I1 | 0.640 | 50.0 | 67.0 |
| 41 | 0.702 | 81.6 | 60.6 |
| 517 | 0.685 | 87.9 | 64.4 |
| 106 | 0.692 | 86.7 | 64.6 |
| 531C | 0.661 | 51.7 | 63.8 |
| P10 | 0.741 | 59.3 | 70.0 |
| S9 | 0.736 | 64.1 | 72.2 |

Coupling

100 ml base matrix was washed with 10 gel volumes distilled water on a glass filter. The gel was weighed (1 g = 1 ml) and mixed with 30 ml distilled water and 8.08 g NaOH (0.202 mol) in a 250 ml flask with an agitator. The temperature was adjusted to 27 +/- 2 °C in a water bath. 16 ml epichlorohydrin (0.202 mol) was added under vigorous agitation (about 250 rpm) during 90 +/- 10 minutes. The reaction was allowed to continue for another 80 +/- 10 minutes and the gel was then washed with >10 gel volumes distilled water on a glass filter until neutral pH was reached. This activated gel was used directly for coupling as below.

To 16.4 mL of ligand solution (50 mg/mL) in a 50 ml Falcon tube, 139 mg NaHCO$_3$, 17.4 mg Na$_2$CO$_3$, 143.8 mg NaCl and 141 mg EDTA, was added. The Falcon tube was placed on a roller table for 5-10 min, and then 63 mg of DTE was added. Reduction proceeded for >45 min. The ligand solution was then desalted on a PD10 column packed with Sephadex G-25. The ligand content in the desalted solution was determined by measuring the 276 nm UV absorption.

The activated gel was washed with 3-5 GV {0.1 M phosphate/1 mM EDTA pH 8.6} and the ligand was then coupled according to the method described in US6399750 5.2.2, although with considerably higher ligand amounts (see below). All buffers used in the experiments had been degassed by nitrogen gas for at least 5-10 min. The ligand content of the gels was controlled by varying the amount and concentration of the ligand solution, adding 5-20 mg ligand per ml gel. The ligand was either a tetramer (SEQ ID NO. 20) or a hexamer (SEQ ID NO. 33) of an alkali-stabilized mutant.

After immobilization the gels were washed 3xGV with distilled water. The gels + 1 GV {0.1 M phosphate/1 mM EDTA/10% thioglycerol pH 8.6} was mixed and the tubes were left in a shaking table at room temperature overnight. The gels were then washed alternately with 3xGV {0.1 M TRIS/0.15 M NaCl pH 8.6} and 0.5 M HAc and then 8-10xGV with distilled water. Gel samples were sent to an external laboratory for amino acid analysis and the ligand content (mg/ml gel) was calculated from the total amino acid content.

Evaluation

The Qb10 % dynamic capacity for polyclonal human IgG at 2.4 and 6 min residence time was determined as outlined in Example 4.

Table 7. Prototype results

| Prototype | Base matrix | Ligand content (mg/ml) | Multimer | Qb10% 2.4 min (mg/ml) | Qb10% 6 min (mg/ml) |
|---|---|---|---|---|---|
| N1 | A38 | 7.45 | tetramer | 44.4 | 58.25 |
| N2 | A20 | 7.3 | tetramer | 45.12 | 57.21 |
| N3 | A42 | 6.72 | tetramer | 33.56 | 50.02 |
| N4 | A29 | 7.3 | tetramer | 36.34 | 51.8 |
| N5 | A28 | 7.9 | tetramer | 42.38 | 58.25 |
| N6 | A39 | 6.96 | tetramer | 41.88 | 54.67 |
| N7 | A27 | 7.5 | tetramer | 29.19 | 48.73 |
| N8 | A43 | 6.99 | tetramer | 33.43 | 49.79 |
| N9 | A38 | 11.34 | tetramer | 48.1 | 72.78 |
| N10 | A20 | 10.6 | tetramer | 50.66 | 70.07 |
| N11 | A42 | 11.1 | tetramer | 32.25 | 57.78 |
| N12 | A29 | 11 | tetramer | 34.85 | 64.68 |
| N13 | A28 | 11.9 | tetramer | 39.92 | 63.75 |
| N14 | A39 | 10.48 | tetramer | 44.37 | 64.79 |
| N15 | A27 | 12.1 | tetramer | 24.8 | 55.56 |
| N16 | A43 | 10.51 | tetramer | 31.82 | 58.04 |
| N17 | A41 | 8.83 | tetramer | 38.5 | 56.8 |
| N18 | A41 | 8.83 | tetramer | 37.84 | 58.6 |
| N19 | A41 | 8.83 | tetramer | 35.06 | 57.23 |
| N20 | A41 | 5.0 | tetramer | 35.64 | 46.04 |
| N21 | A41 | 13.0 | tetramer | 34.95 | 62.23 |

(continued)

| Prototype | Base matrix | Ligand content (mg/ml) | Multimer | Qb10% 2.4 min (mg/ml) | Qb10% 6 min (mg/ml) |
|---|---|---|---|---|---|
| N22 | A40 | 13.15 | tetramer | 56.85 | 71.09 |
| N23 | A33 | 7.33 | tetramer | 48.69 | 55.76 |
| N24 | A40 | 11.03 | tetramer | 54.96 | 73.8 |
| 033A | A38 | 7.5 | tetramer | 44 | 58 |
| 033B | A38 | 11.3 | tetramer | 48 | 73 |
| 097A | A20 | 7.3 | tetramer | 45 | 57 |
| 097B | A20 | 10.6 | tetramer | 51 | 70 |
| 003A | A28 | 7.9 | tetramer | 42 | 58 |
| 003B | A28 | 11.9 | tetramer | 40 | 64 |
| 003C | A28 | 15.8 | tetramer | 37 | 67 |
| 038A | A39 | 7.0 | tetramer | 42 | 55 |
| 038B | A39 | 10.5 | tetramer | 44 | 65 |
| 074 | A40 | 13.2 | tetramer | 57 | 71 |
| 093 | A33 | 7.3 | tetramer | 49 | 56 |
| 058A | A40 | 11.0 | tetramer | 55 | 74 |
| 077 | A18 | 8.2 | tetramer | 52 | 59 |
| 010 | A32 | 10.7 | tetramer | 40 | 57 |
| 099 | A32 | 13.3 | tetramer | 37 | 66 |
| 030A | B5 | 6.3 | tetramer | 32 | 38 |
| 030B | B5 | 9.6 | tetramer | 45 | 47 |
| 293A | C1 | 5.4 | tetramer | 38 | 47 |
| 293B | C1 | 10.8 | tetramer | 43 | 60 |
| 294A | C2 | 5.1 | tetramer | 39 | 46 |
| 294B | C2 | 10.5 | tetramer | 42 | 57 |
| 336A | H40 | 5.6 | tetramer | 47 | 52 |
| 336B | H40 | 9.1 | tetramer | 52 | 67 |
| 091 | A18 | 13.4 | tetramer | N/A | 63 |
| 092 | A20 | 12.8 | tetramer | 49 | 67 |
| 080 | A33 | 9.4 | tetramer | 51 | 58 |
| 089 | A40 | 6.1 | tetramer | 49 | 59 |
| 688A | A62a | 6.6 | tetramer | 41 | 46 |
| 688B | A62a | 14.8 | tetramer | 55 | 62 |
| 871 | A62a | 9.7 | tetramer | 48 | 60 |
| 934A | A63a | 6.6 | tetramer | 40 | 44 |
| 934B | A63a | 14.0 | tetramer | 48 | 56 |
| 017B | A65a | 13.1 | tetramer | 56 | 64 |
| 041A | A62b | 5.2 | tetramer | 40 | N/A |
| 041B | A62b | 11.1 | tetramer | 52 | N/A |

(continued)

| Prototype | Base matrix | Ligand content (mg/ml) | Multimer | Qb10% 2.4 min (mg/ml) | Qb10% 6 min (mg/ml) |
|-----------|-------------|------------------------|----------|------------------------|----------------------|
| 116A | A65b | 5.8 | tetramer | 42 | 46 |
| 116B | A65b | 8.8 | tetramer | 49 | 56 |
| 017A | A65a | 6.1 | tetramer | 40 | 44 |
| 387A | A62a | 6.4 | tetramer | 43 | 45 |
| 387B | A62a | 7.5 | tetramer | 47 | 56 |
| 432 | A63a | 6.1 | tetramer | 39 | 44 |
| 433A | A65a | 6.6 | tetramer | 42 | 47 |
| 433B | A65a | 13.6 | tetramer | 52 | 61 |
| 579A | I1 | 6.1 | tetramer | 45 | 51 |
| 579B | I1 | 11.2 | tetramer | 57 | 68 |
| 064A | C3 | 5.9 | tetramer | 44 | 52 |
| 064B | C3 | 9.0 | tetramer | 49 | 62 |
| 064C | C3 | 14.3 | tetramer | 51 | 70 |
| 352A | C4 | 10.1 | tetramer | 55 | 63 |
| 352B | C4 | 14.4 | tetramer | 59 | 67 |
| 066A | C3 | 6.8 | hexamer | 48 | 59 |
| 066B | C3 | 11.9 | hexamer | 51 | 73 |
| 066C | C3 | 15.1 | hexamer | 43 | 61 |
| 353A | C4 | 11.2 | hexamer | 62 | 74 |
| 353B | C4 | 15.2 | hexamer | 57 | 82 |
| 872A | A62a | 9.6 | hexamer | 56 | 72 |
| 872B | A62a | 14.5 | hexamer | 62 | 84 |
| 869A | H40 | 6.9 | hexamer | 50 | 56 |
| 869B | H40 | 14.3 | hexamer | 56 | 75 |
| 869C | H40 | 23.0 | hexamer | 41 | 65 |
| 962A | H35 | 6.8 | hexamer | 36 | 49 |
| 962B | H35 | 12.3 | hexamer | 31 | 54 |
| 962C | H35 | 20.3 | hexamer | 20 | 43 |
| 112A | A56 | 7.9 | hexamer | 47 | 55 |
| 112B | A56 | 12.4 | hexamer | 57 | 73 |
| 112C | A56 | 19.2 | hexamer | 55 | 80 |
| 113A | A56 | 7.1 | hexamer | 48 | 57 |
| 113B | A56 | 12.4 | hexamer | 53 | 73 |
| 113C | A56 | 15.2 | hexamer | 48 | 76 |
| 212A | H31 | 6.5 | hexamer | 37 | 38 |
| 212B | H31 | 10.4 | hexamer | 50 | 61 |
| 212C | H31 | 20.0 | hexamer | 31 | 52 |
| 213A | A33 | 6.5 | hexamer | 44 | 53 |

(continued)

| Prototype | Base matrix | Ligand content (mg/ml) | Multimer | Qb10% 2.4 min (mg/ml) | Qb10% 6 min (mg/ml) |
|---|---|---|---|---|---|
| 213B | A33 | 10.9 | hexamer | 50 | 65 |
| 213C | A33 | 11.1 | hexamer | 50 | 68 |
| 432A | A20 | 6.4 | hexamer | 41 | 56 |
| 432B | A20 | 12.4 | hexamer | 38 | 64 |
| 432C | A20 | 21.1 | hexamer | 44 | 43 |
| 433A | A38 | 5.9 | hexamer | 47 | 57 |
| 433B | A38 | 11.6 | hexamer | 48 | 72 |
| 433C | A38 | 15.8 | hexamer | 36 | 62 |
| 742A | A54 | 6.7 | hexamer | 38 | 46 |
| 742B | A54 | 12.6 | hexamer | 45 | 52 |
| 742C | A54 | 21.1 | hexamer | 38 | 65 |
| 726A | A63b | 6.4 | hexamer | 42 | 46 |
| 726B | A63b | 10.6 | hexamer | 49 | 60 |
| 726C | A63b | 16.7 | hexamer | 53 | 69 |
| 793A | A56-2 | 6.8 | hexamer | 50 | 58 |
| 793B | A56-2 | 12.5 | hexamer | 59 | 72 |
| 793C | A56-2 | 19.2 | hexamer | 61 | 82 |
| 517 | 517 | 12.0 | tetramer* | 35 | 56 |
| 106 | 106 | 5.8 | tetramer* | 33 | 45 |
| 531C | 531C | 11.2 | tetramer* | 54 | 65 |
| P10 | P10 | 19.0 | hexamer | | 76 |
| S9 | S9 | 18.4 | hexamer | 56 | 75 |
| * SEQ ID NO 21 | | | | | |

Example 7

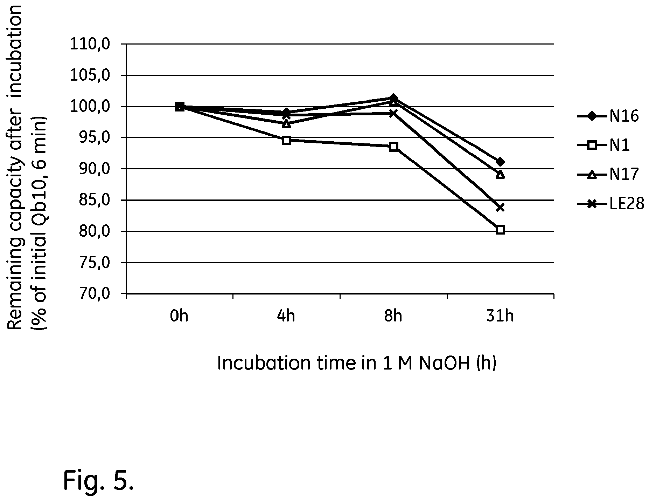

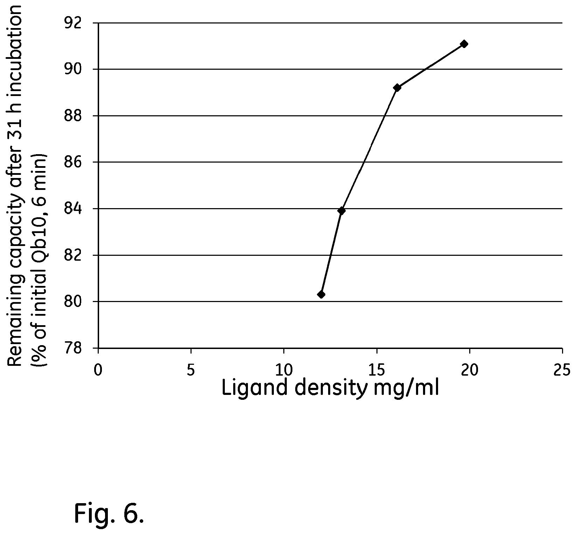

A series of prototypes, prepared as above, with different ligand content (tetramer, SEQ ID N0:20) were incubated in 1 M NaOH for 4, 8 and 31 hours at 22 +/- 2 °C and the dynamic IgG capacity (Qb10%, 6 min residence time) was measured before and after incubation. The prototypes are shown in Table 8 and the results in Figs. 5 and 6. It can be seen that the stability towards this harsh alkali treatment increases with increasing ligand content.

Table 8. Samples for incubation in 1 M NaOH

| Prototype | Ligand content (mg/ml) | Qb10%, 6 min, before incubation (mg/ml) |
|---|---|---|
| N1 | 12 | 78 |
| LE28 | 13 | 79 |
| N17 | 16 | 73 |
| N16 | 20 | 73 |

Example 8

Two crosslinked agarose bead prototypes, prepared as above, with different ligand content (hexamer, SEQ ID NO:33), median bead diameter (d50,v) 62 $\mu$m and Kd 0.70 for dextran of Mw 110 kD, were evaluated with a real mAb feed. The ligand content of prototype A was 14.3 mg/ml and of prototype B 18.9 mg/ml. For comparison, the commercial product MabSelect SuRe® LX (GE Healthcare Life Sciences) was used. The resins were packed in Tricorn columns (GE Healthcare Life Sciences) to bed heights of 10 cm, giving bed volumes of 2 ml and the columns were shown to have peak asymmetry within the 0.8-1.5 interval. The sample loaded was a clarified CHO cell supernatant with 4.9 mg/ml monoclonal IgG1 antibody at physiological pH and the experimental conditions were as listed below in Table 9 (CV = column volumes, RT = residence time).

Table 9. Conditions for evaluation with real feed.

| Equilibration: | 3 CV 20 mM phosphate, 150 mM NaCl pH 7.4, RT=3.4 min |
|---|---|
| Sample loading: | 43 mg mAb/ml resin, RT=6 min |
| Wash 1: | 5 CV 20 mM phosphate, 500 mM NaCl pH 7.4, 1.5 CV at RT=6min and 3.5 CV at RT=3.4 min |
| Wash 2: | 1 CV 50 mM acetate pH 6.0, RT=3.4 min |
| Elution: | 3 CV 50 mM acetate pH 3.5, RT=6 min, peak collected between 150 mAU-150 mAU |
| Strip: | 2 CV 100 mM acetate, RT=3.4 min |
| CIP: | 3 CV 0.1 M NaOH, RT=6 min |
| Re-equilibration: | 5 CV 20 mM phosphate, 150 mM NaCl pH 7.4, RT=3.4 min |

The mAb peak was collected using a UV watch function and the concentration of the mAb was determined by UV measurement at 280 nm (extinction coefficient 1.5). All absorbance detections were performed using a spectrophotometer, including the measurements for the yield calculations.

Samples for HCP (host cell protein) analyses were prepared by adding 10% Preservation buffer (0.2 M $NaH_2PO_4{}^*H_2O$ (5.3%), 0.2 M $Na_2HPO_4{}^*12\ H_2O$ (94.7%), 0.5% Tween 20, 1% BSA pH 8) to the samples directly after each run made (e.g. 50 $\mu$l preservation buffer to 450 $\mu$l sample). The HCP content was measured using commercial anti-CHO antibodies (Cygnus Technologies) and a Gyrolab (Gyros AB, Sweden) work station.

The results are presented in Table 10 below and show that the performance of the prototypes is in the same range as for the commercial product. The HCP content in the feed was 331 000 ppm.

Table 10. Results from real feed evaluation

| Resin | Yield (%) | Elution pool (CV) | HCP in pool (ppm) |
|---|---|---|---|
| MabSelect SuRe LX | 90 | 1.5 | 914 |
| MabSelect SuRe LX | 95 | 1.6 | 1021 |
| Prototype A | 96 | 1.3 | 1076 |
| Prototype A | 95 | 1.3 | 1105 |
| Prototype B | 96 | 1.3 | 1040 |
| Prototype B | 93 | 1.3 | 1104 |

Example 9

A crosslinked agarose bead matrix prototype, prepared as above, with 14.5 mg/ml ligand (hexamer, SEQ ID NO:33), median bead diameter (d50,v) 57.4 $\mu$m, Kd 0.72 for dextran of Mw 110 kD and dry weight 70.3 mg/ml, was evaluated for elution pH with two real mAb feeds (mAb1 2.4 g/l and mAb2 4.9 g/l) IgG1, physiological pH, and a sample of polyclonal human IgG (Gammanorm, Octapharma). For comparison, the commercial product MabSelect SuRe® LX (GE Healthcare Life Sciences) was used. The resins were packed in Tricorn columns (GE Healthcare Life Sciences) to bed heights of 10 cm, giving bed volumes of 2 ml and the columns were shown to have peak asymmetry within the 0.8-1.5 interval. The samples loaded were clarified CHO cell supernatants with IgG1 mAbs at physiological pH and the experimental conditions were as listed below in Table 11 (CV = column volumes, RT = residence time).

Table 11. Conditions for elution pH evaluation.

| Equilibration: | 5 CV 20 mM phosphate, 150 mM NaCl pH 7.4, RT=3.4 min |
|---|---|

(continued)

| Sample loading: | 10 mg mAb/ml resin, RT=6 min |
| Wash: | 6 CV 20 mM phosphate, 150 mM NaCl pH 7.4, RT=3.4 min |
| Elution: | 30 CV 100 mM citrate pH 6-3 gradient, RT=6 min |
| CIP: | 3 CV 0.1 M NaOH, RT=6 min |
| Re-equilibration: | 8 CV 20 mM phosphate, 150 mM NaCl pH 7.4, RT=3.4 min |

The results are shown below in Table 12 and indicate that the antibodies elute at similar pH levels as on the reference, although with some individual variation depending on the particular antibody-resin combination.

Table 12. Results from elution pH evaluation

| Sample | Elution pH MabSelect SuRe LX | Elution pH prototype |
|---|---|---|
| mAb 1 | 3.67 | 3.53 |
| mAb 2 | 3.68 | 3.80 |
| Polyclonal IgG | 4.01 (peak 1) | 4.24 (peak 1) |
| | 3.70 (peak 2) | 3.81 (peak 2) |

Fractions from the pH-gradient elution of polyclonal IgG were also analysed with respect to content of IgG1, IgG2 and IgG4, using a Biacore SPR instrument (GE Healthcare Life Sciences) with antibodies against the four different IgG classes immobilized on a CM5 Biacore chip.

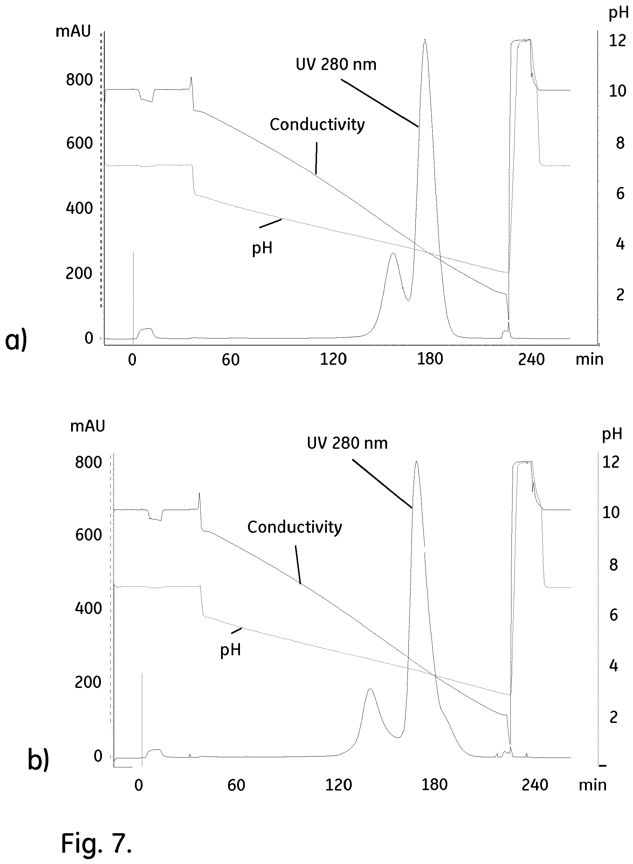

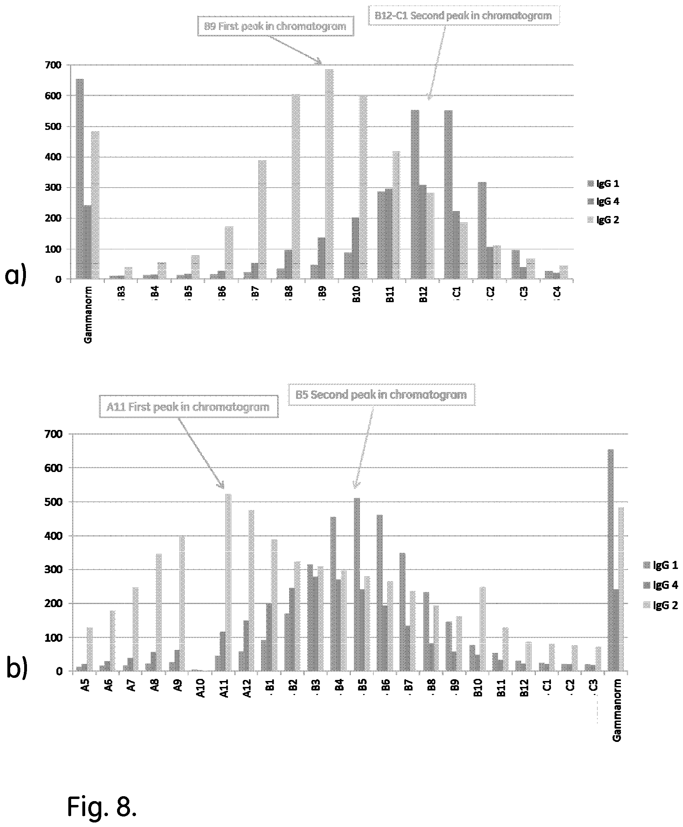

The chromatograms for polyclonal IgG on the reference and the prototype are shown in Fig 7 and the IgG class analyses are shown in Fig. 8. The data show that all three classes bind to both resins in a similar way and that the first peak predominantly contains IgG2, while IgG1 and IgG4 elute mainly in the second peak. The anti-IgG3 antibodies cross-reacted with IgG4, so no reliable results for IgG3 were obtained. IgG3 is generally known to show no or only weak binding to Protein A.

Example 10

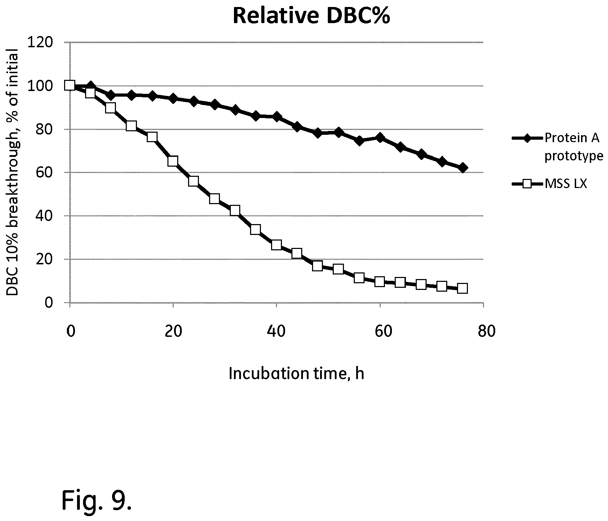

A crosslinked agarose bead matrix prototype, prepared as above, with 12.6 mg/ml ligand (tetramer, SEQ ID NO:20), 84.9 $\mu$m median bead diameter (d50,v), Kd 0.71 for dextran Mw 110 kD and 62.2 mg/ml dry weight, was evaluated with respect to alkali stability, using the commercial product MabSelect SuRe LX as a reference. Tricorn 5 columns packed with the resins to 10 cm bed height were flushed with 3 column volumes of 1 M NaOH. The flow was then stopped for 240 minutes (corresponding to 16 normal CIP cycles of 15 min/cycle) before washing out the NaOH solution by 3 column volumes of PBS buffer. The dynamic binding capacity for polyclonal IgG (Gammanorm, Octapharma) was then measured and the process was repeated with another injection of 1 M NaOH. The dynamic capacity was measured after each 240 min incubation cycle with 1 M NaOH. In the capacity measurements, the columns were equilibrated with PBS buffer before the 2 mg/ml sample was loaded (residence time 6 min) until a UV signal of 85% of maximum absorbance was reached. Then the column was washed with PBS buffer, eluted with 500 mM acetic acid pH 3.0 and re-equilibrated. The dynamic binding capacity at 10% and 80% breakthrough was calculated as described above. The results are shown in Fig. 9 and they show that the prototype was significantly more stable than the commercial product.

Example 11

Pressure-flow testing of matrices

300 ml sedimented matrix was packed in a FineLine™ 35 column (GE Healthcare Life Sciences, Uppsala, Sweden), with 35 mm inner diameter and 330 mm tube height. The gel was suspended in distilled water to produce a slurry volume of 620 ml and the height of the packed bed was 300 +/- 10 mm. The packing pressure was 0.10 +/- 0.02 bar (10 +/- 2 kPa).

Distilled water was then pumped through the column at increasing pump rates and the flow rate (expressed as the linear flow velocity, cm/h) and back pressure (MPa) was measured after 5 min for each pump setting. The measurements were continued until a max flow rate and a max pressure was reached, i.e. the flow rate and back pressure

EP 3 455 242 B1

achieved when the flow rate starts to diminish at increasing back pressures.

Table 9 Pressure flow performance of matrices

| Matrix | Max flow velocity, cm/h | Max pressure (MPa) |
|---|---|---|
| 517 | 1343 | 0.56 |
| 106 | 1306 | 0.56 |
| 531C | 513 | 0.51 |
| P10 | 862 | 0.60 |
| S9 | 1172 | 0.64 |

The P10 and S9 matrices have a higher rigidity, as indicated by the max pressure, and can thus sustain comparatively high flow velocities despite their low (59-64 micrometers) median particle diameters.

## Claims

1. A method of isolating an immunoglobulin, comprising the steps of:

   a) providing a separation matrix comprising at least 15 mg/ml multimers of immunoglobulin-binding alkali-stabilized Protein A domains covalently coupled to a porous support, wherein said porous support comprises cross-linked agarose particles having a volume-weighted median diameter (d50,v) of 56-70 micrometers, and a dry solids weight of 55-80 mg/ml and a pore size corresponding to an inverse gel filtration chromatography Kd value of 0.69-0.85 for dextran of Mw 110 kDa, and wherein said separation matrix has a max pressure of at least 0.58, such as at least 0.60, MPa when packed at 300 +/-10 mm bed height in a FineLine™ 35 column,
   b) contacting a liquid sample comprising an immunoglobulin with said separation matrix, wherein at least 40 mg immunoglobulin per ml separation matrix is contacted with said separation matrix,
   c) washing said separation matrix with a washing liquid,
   d) eluting the immunoglobulin from the separation matrix with an elution liquid, and
   e) cleaning the separation matrix with a cleaning liquid,

   wherein said alkali-stabilized Protein A domains comprise mutants of a parental Fc-binding domain of Staphylococcus Protein A (SpA), as defined by SEQ ID NO 51 or SEQ ID NO 52, wherein the amino acid residues at positions 13 and 44 of SEQ ID NO 51 or 52 are asparagines and wherein at least the asparagine residue at position 3 of SEQ ID NO 51 or 52 has been mutated to an amino acid selected from the group consisting of glutamic acid, lysine, tyrosine, threonine, phenylalanine, leucine, isoleucine, tryptophan, methionine, valine, alanine, histidine and arginine, such as to a glutamic acid.

2. The method of claim 1, wherein the mutants comprise further mutations in one or more of positions 1, 2, 7, 10, 15, 20, 21, 24, 25, 28, 29, 32, 34, 35, 36, 39, 42 and 43 in SEQ ID NO 51 or 52.

3. The method of claim 1 or 2, wherein the glutamine residue at position 1 of SEQ ID NO 51 or 52 has been mutated to an alanine or has been deleted.

4. The method of any preceding claim, wherein the asparagine or glutamic acid residue at position 35 of SEQ ID NO 51 or 52 has been mutated to an alanine and/or wherein the amino acid residue at position 32 of SEQ ID NO 51 or 52 is a valine.

5. The method of any preceding claim, wherein said separation matrix comprises 15-21, 17-21 or 18-20 mg/ml of said multimers of immunoglobulin-binding alkali-stabilized Protein A domains covalently coupled to a porous support.

6. The method of any preceding claim, wherein said multimers comprise tetramers, pentamers, hexamers or heptamers of alkali-stabilized Protein A domains, such as hexamers of alkali-stabilized Protein A domains.

7. The method of any preceding claim, wherein the domains are linked by linkers comprising up to 25 amino acids, such as 3-25 or 3-20 amino acids, and/or wherein at least two domains are linked by linkers comprising or consisting essentially of a sequence having at least 90% identity with an amino acid sequence selected from the group consisting of APKVDAKFDKE, APKVDNKFNKE, APKADNKFNKE, APKVFDKE, APAKFDKE, AKFDKE, APKVDA, VDAKFDKE, APKKFDKE, APK, APKYEDGVDAKFDKE and YEDG.

8. The method of any preceding claim, wherein in step e) said cleaning liquid comprises at least 0.5 M NaOH or at least 1 M NaOH.

9. The method of any preceding claim, wherein steps b) - e) are repeated at least 10 times, such as at least 50 times or 50 - 200 times.

10. The method of any preceding claim, wherein:

i) said liquid sample is a clarified cell broth and wherein in step d) said immunoglobulin is recovered as an eluate comprising less than 2000 ppm host cell proteins;
ii) in step d) said elution liquid has a pH of 2.5-4.5;
iii) said immunoglobulin comprises IgG1, IgG2 and/or IgG4;
iv) said washing liquid has a pH of 5-8 and optionally comprises one or more of a detergent, a water-miscible organic solvent, a chaotrope, arginine or an arginine derivative, calcium ions and tetraalkylammonium ions;
v) said multimers are coupled to said support via thioether links;
vi) in step b) the pH is 6-8; and/or
vii) in step b) the residence time of said liquid sample on said separation matrix is 2-20 min.

**Patentansprüche**

1. Verfahren zum Isolieren eines Immunglobulins, umfassend die folgenden Schritte:

a) Bereitstellen einer Trennungsmatrix, umfassend zumindest 15 mg/ml Multimere von immunglobulinbindenden alkalisch stabilisierten Protein-A-Domänen, welche kovalent an einen porösen Träger gekoppelt sind, wobei der poröse Träger vernetzte Agarosepartikel mit einem volumengewichteten mittleren Durchmesser (d50,v) von 56-70 Mikrometer und einem Trockensubstanzgewicht von 55-80 mg/ml und einer Porengröße, welche einem inversen Gelfiltrationschromatographie-Kd-Wert von 0,69-0,85 für Dextran mit Mw 110 kDa entspricht, umfasst, und wobei die Trennungsmatrix einen maximalen Druck von zumindest 0,58 MPa, beispielsweise 0,60 MPa, aufweist, wenn bei 300 +/-10 mm Betthöhe in einer FineLine™ 35-Säule gepackt,
b) Kontaktieren einer flüssigen Probe, welche Immunglobulin umfasst, mit der Trennungsmatrix, wobei zumindest 40 mg Immunglobulin pro ml Trennungsmatrix mit der Trennungsmatrix kontaktiert wird,
c) Waschen der Trennungsmatrix mit einer Waschflüssigkeit,
d) Eluieren des Immunglobulins aus der Trennungsmatrix mit einer Elutionsflüssigkeit, und
e) Reinigen der Trennungsmatrix mit einer Reinigungsflüssigkeit,

wobei die alkalisch stabilisierten Protein-A-Domänen Mutanten einer übergeordneten Fe-bindenden Domäne von Staphylokokkus-Protein-A (SpA) gemäß Definition durch SEQ ID-Nr. 51 oder SEQ ID-Nr. 52 umfassen, wobei die Aminosäurenrückstände an Position 13 und 44 von SEQ ID-Nr. 51 oder 52 Asparagine sind, und wobei zumindest der Asparaginrückstand an Position 3 von SEQ ID-Nr. 51 oder 52 in eine Aminosäure ausgewählt aus der Gruppe, bestehend aus Glutaminsäure, Lysin, Tyrosin, Threonin, Phenylalanin, Leucin, Isoleucin, Tryptophan, Methionin, Valin, Alanin, Histidin und Arginin, beispielsweise zu Glutaminsäure, mutiert wurde.

2. Verfahren nach Anspruch 1, wobei die Mutanten weiter Mutationen in einer oder mehreren von Position 1, 2, 7, 10, 15, 20, 21, 24, 25, 28, 29, 32, 34, 35, 36, 39, 42 und 43 in SEQ ID-Nr. 51 oder 52 umfassen.

3. Verfahren nach Anspruch 1 oder 2, wobei der Glutaminrückstand an Position 1 von SEQ ID-Nr. 51 oder 52 in ein Alanin mutiert wurde oder gelöscht wurde.

4. Verfahren nach einem der vorstehenden Ansprüche, wobei der Asparagin- oder Glutaminsäurerückstand an Position 35 von SEQ ID-Nr. 51 oder 52 in ein Alanin mutiert wurde und/oder wobei der Aminosäurerückstand an Position 32 von SEQ ID-Nr. 51 oder 52 ein Valin ist.

5. Verfahren nach einem der vorstehenden Ansprüche, wobei die Trennungsmatrix 15-21, 17-21 oder 18-20 mg/ml der Multimere der immunglobulinbindenden alkalisch stabilisierten Protein-A-Domänen umfasst, welche kovalent an einen porösen Träger gekoppelt sind.

6. Verfahren nach einem der vorstehenden Ansprüche, wobei die Multimere Tetramere, Pentamere, Hexamere oder Heptamere von alkalisch stabilisierten Protein-A-Domänen, beispielsweise Heptamere von alkalisch stabilisierten Protein-A-Domänen, umfassen.

7. Verfahren nach einem der vorstehenden Ansprüche, wobei die Domänen durch Linker verknüpft sind, welche bis zu 25 Aminosäuren, beispielsweise 3-25 oder 3-20 Aminosäuren, umfassen, und/oder wobei zumindest zwei Domänen durch Linker verknüpft sind, welche eine Sequenz mit zumindest 90% Identität mit einer Aminosäuresequenz ausgewählt aus der Gruppe, bestehend aus APKVDAKFDKE, APKVDNKFNKE, APKADNKFNKE, APKVFDKE, APAKFDKE, AKFDKE, APKVDA, VDAKFDKE, APKKFDKE, APK, APKYEDGVDAKFDKE und YEDG umfassen oder im Wesentlichen daraus bestehen.

8. Verfahren nach einem der vorstehenden Ansprüche, wobei in Schritt e) die Reinigungsflüssigkeit zumindest 0,5 M NaOH oder zumindest 1 M NaOH umfasst.

9. Verfahren nach einem der vorstehenden Ansprüche, wobei Schritte b) - e) zumindest 10 Mal, beispielsweise zumindest 50 Mal oder 50-200 Mal, wiederholt werden.

10. Verfahren nach einem der vorstehenden Ansprüche, wobei:

i) die flüssige Probe eine geklärte Zellbouillon ist und wobei in Schritt d) das Immunglobulin als ein Eluat wiedergewonnen wird, welches weniger als 2000 ppm Wirtszellenproteine umfasst.
ii) in Schritt d) die Elutionsflüssigkeit einen pH von 2,5-4,5 aufweist;
iii) das Immunglobulin IgG1, IgG2 und/oder IgG4 umfasst;
iv) die Waschflüssigkeit einen pH von 5-8 aufweist und optional eines oder mehrere von einem Detergens, einem wassermischbaren organischen Lösungsmittel, einem Chaotrop, Arginin oder einem Argininderivat, Calciumionen und Tetraalkylammoniumionen umfasst;
v) die Multimere über Thioetherverknüpfungen mit dem Träger gekoppelt sind;
vi) in Schritt b) der pH 6-8 ist; und/oder
vii) in Schritt b) die Verweildauer der flüssigen Probe auf der Trennungsmatrix 2-20 Min. beträgt.

## Revendications

1. Procédé d'isolement d'une immunoglobuline, comprenant les étapes de :

a) fourniture d'une matrice de séparation comprenant au moins 15 mg/ml de multimères de domaines de la protéine A stabilisés par des alcalis et se liant à l'immunoglobuline, couplés de manière covalente à un support poreux, dans lequel ledit support poreux comprend des particules d'agarose réticulées ayant un diamètre moyen pondéré en volume (d50,v) de 56-70 micromètres, un poids en matière sèche de 55-80 mg/ml et une taille de pore correspondant à une valeur Kd de chromatographie par filtration sur gel en phase inverse de 0,69-0,85 pour dextrane de 110 kDa de poids moléculaire, et dans lequel ladite matrice de séparation a une pression maximale d'au moins 0,58, telle que d'au moins 0,60 MPa lorsqu'elle est garnie à une hauteur de lit de 300 +/- 10 mm dans une colonne FineLine™ 35,
b) mise en contact d'un échantillon liquide comprenant une immunoglobuline avec ladite matrice de séparation, dans lequel au moins 40 mg d'immunoglobuline par ml de matrice de séparation sont mis en contact avec ladite matrice de séparation,
c) lavage de ladite matrice de séparation avec un liquide de lavage,
d) élution de l'immunoglobuline de la matrice de séparation avec un liquide d'élution, et
e) nettoyage de la matrice de séparation avec un liquide de nettoyage,

dans lequel lesdits domaines de la protéine A stabilisés par des alcalis comprennent des mutants d'un domaine de liaison à un Fc parental de la protéine A de staphylocoque (SpA), comme défini par SEQ ID NO 51 ou SEQ ID NO 52, dans lequel les résidus d'acides aminés aux positions 13 et 44 de SEQ ID NO 51 ou 52 sont des asparagines et dans lequel au moins le résidu d'asparagine à la position 3 de SEQ ID NO 51 ou 52 a été muté en un acide aminé choisi dans le groupe constitué par l'acide glutamique, la lysine, la tyrosine, la thréonine, la phénylalanine, la leucine, l'isoleucine, le tryptophane, la méthionine, la valine, l'alanine, l'histidine et l'arginine, tel qu'un acide glutamique.

**2.** Procédé selon la revendication 1, dans lequel les mutants comprennent d'autres mutations dans une ou plusieurs des positions 1, 2, 7, 10, 15, 20, 21, 24, 25, 28, 29, 32, 34, 35, 36, 39, 42 et 43 dans la SEQ ID NO 51 ou 52.

**3.** Procédé selon la revendication 1 ou 2, dans lequel le résidu de glutamine à la position 1 de SEQ ID NO 51 ou 52 a été muté en une alanine ou a été supprimé.

**4.** Procédé selon l'une quelconque des revendications précédentes, dans lequel le résidu d'asparagine ou d'acide glutamique à la position 35 de SEQ ID NO 51 ou 52 a été muté en une alanine et/ou dans lequel le résidu d'acide aminé à la position 32 de SEQ ID NO 51 ou 52 est une valine.

**5.** Procédé selon l'une quelconque des revendications précédentes, dans lequel ladite matrice de séparation comprend 15-21, 17-21 ou 18-20 mg/ml desdits multimères de domaines de la protéine A stabilisés par des alcalis et se liant à l'immunoglobuline, couplés de manière covalente à un support poreux.

**6.** Procédé selon l'une quelconque des revendications précédentes, dans lequel lesdits multimères comprennent des tétramères, pentamères, hexamères ou heptamères de domaines de la protéine A stabilisés par des alcalis, tels que des hexamères de domaines de la protéine A stabilisés par des alkalis.

**7.** Procédé selon l'une quelconque des revendications précédentes, dans lequel les domaines sont liés par des liants comprenant jusqu'à 25 acides aminés, tel que de 3-25 ou de 3-20 acides aminés, et/ou dans lequel au moins deux domaines sont liés par des liants comprenant ou constitués essentiellement par une séquence ayant au moins une identité à 90 % avec une séquence d'acides aminés choisie dans le groupe constitué de APKVDAKFDKE, APKVDNKFNKE, APKADNKFNKE, APKVFDKE, APAKFDKE, AKFDKE, APKVDA, VDAKFDKE, APKKFDKE, APK, APKYEDGVDAKFDKE et YEDG.

**8.** Procédé selon l'une quelconque des revendications précédentes, dans lequel à l'étape e), ledit liquide de nettoyage comprend au moins 0,5 M de NaOH ou au moins 1 M de NaOH.

**9.** Procédé selon l'une quelconque des revendications précédentes, dans lequel les étapes b) à e) sont répétées au moins 10 fois, tel qu'au moins 50 fois ou de 50-200 fois.

**10.** Procédé selon l'une quelconque des revendications précédentes, dans lequel :

i) ledit échantillon liquide est un bouillon de cellules clarifié et dans lequel à l'étape d), ladite immunoglobuline est récupérée sous forme d'un éluat comprenant moins de 2 000 ppm de protéines de cellules hôtes ;
ii) à l'étape d), ledit liquide d'élution a un pH de 2,5-4,5 ;
iii) ladite immunoglobuline comprend IgG1, IgG2 et/ou IgG4 ;
iv) ledit liquide de lavage a un pH de 5-8 et comprend éventuellement un ou plusieurs d'un détergent, d'un solvant organique miscible dans l'eau, d'un chaotrope, d'une arginine ou d'un dérivé d'arginine, d'ions de calcium et d'ions de tétraalkylammonium ;
v) lesdits multimères sont couplés audit support via des liaisons thioéther ;
vi) à l'étape b), le pH est de 6-8 ; et/ou
vii) à l'étape b), le temps de séjour dudit échantillon liquide sur ladite matrice de séparation est de 2-20 min.

Alignment of Fc-binding domains

```
E         ---  -------AQQ   NAFYQVLNMP   NLNADQRNGF   IQSLKDDPSQ   SANVLGEAQK   LNDSQAPK   51   (SEQ ID NO: 1)
D         ADA  QQNKFNKDQQ   SAFYEILNMP   NLNEEQRNGF   IQSLKDDPSQ   STNVLGEAKK   LNESQAPK   61   (SEQ ID NO: 2)
A         --A  DNN-FNKEQQ   NAFYEILNMP   NLNEEQRNGF   IQSLKDDPSQ   SANLLAEAKK   LNESQAPK   58   (SEQ ID NO: 3)
B         ---  ADNKFNKEQQ   NAFYEILHLP   NLNEEQRNGF   IQSLKDDPSQ   SANLLAEAKK   LNDAQAPK   58   (SEQ ID NO: 4)
C         ---  ADNKFNKEQQ   NAFYEILHLP   NLTEEQRNGF   IQSLKDDPSV   SKEILAEAKK   LNDAQAPK   58   (SEQ ID NO: 5)
Z         ---  VDNKFNKEQQ   NAFYEILHLP   NLNEEQRNAF   IQSLKDDPSQ   SANLLAEAKK   LNDAQAPK   58   (SEQ ID NO: 6)
Zvar      ---  VDAKFDKEQQ   NAFYEILHLP   NLTEEQRNAF   IQSLKDDPSQ   SANLLAEAKK   LNDAQAPK   58   (SEQ ID NO: 7)


          ---  -------QQ   NAFYEILHLP   NLTEEQRNAF   IQSLKDDPSQ   SANLLAEAKK   LNDAQ---   47   (SEQ ID NO: 51)
          ---  -------QQ   NAFYEILHLP   NLTEEQRNGF   IQSLKDDPSV   SKEILAEAKK   LNDAQ---   47   (SEQ ID NO: 52)

Pos          1        10           20           30           40           50           58
```

Fig. 1

EP 3 455 242 B1

Fig. 2.

Fig. 3.

Fig. 4.

Fig. 5.

Fig. 6.

Fig. 7.

Fig. 8.

Fig. 9.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20050143566 A
- US 5143844 A
- US 8329860 B
- JP 2006304633 A
- US 8674073 B
- US 20100221844 A
- US 20120208234 A
- US 9051375 B
- US 20140031522 A
- US 20130274451 A
- WO 2014146350 A
- WO 2015005859 A
- WO 2012083425 A
- SE 2014050872 W
- US 9018305 B
- US 6602990 B
- US 7396467 B
- WO 2015048330 A
- WO 2015166072 A
- WO 2014159064 A
- US 8084032 B
- US 8853371 B
- US 20080167450 A
- US 20110144311 A
- US 20130096284 A
- US 20120238730 A
- US 20140018525 A
- WO 2013033517 A
- US 20140228548 A
- US 20150093800 A
- US 6127526 A
- US 6870034 B
- US 7820799 B
- US 7834162 B
- US 8263750 B
- US 7714111 B
- US 9284347 B
- US 20120283416 A
- US 20130197197 A
- WO 2014186350 A
- WO 2014192877 A
- US 20140094593 A
- US 20160108084 A
- US 20160024147 A
- US 4704366 A
- US 4801687 A
- US 4933435 A
- EP 2782925 A1
- US 20140154270 A
- US 6399750 B
- US 6399750522 A

**Non-patent literature cited in the description**

- **SUSANNE GÜLICH ; MARTIN LINHULT ; PER-AKE NYGREN ; MATHIAS UHLÉN ; SOPHIA HOBER.** *Journal of Biotechnology,* 2000, vol. 80, 169-178
- **ALTSHUL et al.** *J. Mol. Biol.,* 1990, vol. 215, 403-410
- **N PAKIMAN et al.** *J Appl Sci,* 2012, vol. 12, 1136-1141
- Gel Filtration Principles and Methods. Pharmacia LKB Biotechnology, 1991, 6-13
- **S HJERTÉN.** *Biochim Biophys Acta,* 1964, vol. 79 (2), 393-398

SEQUENCE LISTING

<110> GE Healthcare Bioprocess R&D AB

<120> MUTATED IMMUNOGLOBULIN-BINDING POLYPEPTIDES

<130> 315715

<160> 95

<170> PatentIn version 3.5

<210> 1
<211> 51
<212> PRT
<213> Staphylococcus aureus

<400> 1

```
Ala Gln Gln Asn Ala Phe Tyr Gln Val Leu Asn Met Pro Asn Leu Asn
1               5                   10                  15

Ala Asp Gln Arg Asn Gly Phe Ile Gln Ser Leu Lys Asp Asp Pro Ser
            20                  25                  30

Gln Ser Ala Asn Val Leu Gly Glu Ala Gln Lys Leu Asn Asp Ser Gln
            35                  40                  45

Ala Pro Lys
        50
```

<210> 2
<211> 61
<212> PRT
<213> Staphylococcus aureus

<400> 2

```
         Ala  Asp  Ala  Gln  Gln  Asn  Lys  Phe  Asn  Lys  Asp  Gln  Gln  Ser  Ala  Phe
         1              5                  10                      15

         Tyr  Glu  Ile  Leu  Asn  Met  Pro  Asn  Leu  Asn  Glu  Glu  Gln  Arg  Asn  Gly
                        20                  25                      30

         Phe  Ile  Gln  Ser  Leu  Lys  Asp  Asp  Pro  Ser  Gln  Ser  Thr  Asn  Val  Leu
                        35                  40                      45

         Gly  Glu  Ala  Lys  Lys  Leu  Asn  Glu  Ser  Gln  Ala  Pro  Lys
              50                  55                      60
```

<210> 3
<211> 58
<212> PRT
<213> Staphylococcus aureus

<400> 3

```
         Ala  Asp  Asn  Asn  Phe  Asn  Lys  Glu  Gln  Gln  Asn  Ala  Phe  Tyr  Glu  Ile
         1              5                  10                      15

         Leu  Asn  Met  Pro  Asn  Leu  Asn  Glu  Glu  Gln  Arg  Asn  Gly  Phe  Ile  Gln
                        20                  25                      30

         Ser  Leu  Lys  Asp  Asp  Pro  Ser  Gln  Ser  Ala  Asn  Leu  Leu  Ala  Glu  Ala
                        35                  40                      45

         Lys  Lys  Leu  Asn  Glu  Ser  Gln  Ala  Pro  Lys
              50                  55
```

<210> 4
<211> 58
<212> PRT
<213> Staphylococcus aureus

<400> 4

```
Ala Asp Asn Lys Phe Asn Lys Glu Gln Gln Asn Ala Phe Tyr Glu Ile
1               5               10              15

Leu His Leu Pro Asn Leu Asn Glu Glu Gln Arg Asn Gly Phe Ile Gln
            20              25              30

Ser Leu Lys Asp Asp Pro Ser Gln Ser Ala Asn Leu Leu Ala Glu Ala
            35              40              45

Lys Lys Leu Asn Asp Ala Gln Ala Pro Lys
    50              55
```

<210> 5
<211> 58
<212> PRT
<213> Staphylococcus aureus

<400> 5

```
Ala Asp Asn Lys Phe Asn Lys Glu Gln Gln Asn Ala Phe Tyr Glu Ile
1               5               10              15

Leu His Leu Pro Asn Leu Thr Glu Glu Gln Arg Asn Gly Phe Ile Gln
            20              25              30

Ser Leu Lys Asp Asp Pro Ser Val Ser Lys Glu Ile Leu Ala Glu Ala
            35              40              45

Lys Lys Leu Asn Asp Ala Gln Ala Pro Lys
    50              55
```

<210> 6
<211> 58
<212> PRT
<213> Escherichia coli

<400> 6

```
Val Asp Asn Lys Phe Asn Lys Glu Gln Gln Asn Ala Phe Tyr Glu Ile
1               5               10              15

Leu His Leu Pro Asn Leu Asn Glu Glu Gln Arg Asn Ala Phe Ile Gln
            20              25              30

Ser Leu Lys Asp Asp Pro Ser Gln Ser Ala Asn Leu Leu Ala Glu Ala
            35              40              45

Lys Lys Leu Asn Asp Ala Gln Ala Pro Lys
    50              55
```

<210> 7
<211> 58
<212> PRT
<213> Escherichia coli

<400> 7

```
Val Asp Ala Lys Phe Asp Lys Glu Gln Gln Asn Ala Phe Tyr Glu Ile
1               5                   10                  15

Leu His Leu Pro Asn Leu Thr Glu Glu Gln Arg Asn Ala Phe Ile Gln
            20                  25                  30

Ser Leu Lys Asp Asp Pro Ser Gln Ser Ala Asn Leu Leu Ala Glu Ala
        35                  40                  45

Lys Lys Leu Asn Asp Ala Gln Ala Pro Lys
    50                  55
```

<210> 8
<211> 58
<212> PRT
<213> Escherichia coli

<400> 8

```
Val Asp Ala Lys Phe Asp Lys Glu Ala Gln Glu Ala Phe Tyr Glu Ile
1               5                   10                  15

Leu His Leu Pro Asn Leu Thr Glu Glu Gln Arg Asn Ala Phe Ile Gln
            20                  25                  30

Ser Leu Lys Asp Asp Pro Ser Gln Ser Ala Ala Leu Leu Ala Glu Ala
                35                  40                  45

Lys Lys Leu Asn Asp Ala Gln Ala Pro Lys
    50                  55
```

<210> 9
<211> 58
<212> PRT
<213> Escherichia coli

<400> 9

```
Val Asp Ala Lys Phe Asp Lys Glu Ala Gln Glu Ala Phe Tyr Glu Ile
1               5               10              15

Leu His Leu Pro Asn Leu Thr Glu Glu Gln Arg Ala Ala Phe Ile Gln
            20              25              30

Ser Leu Lys Asp Asp Pro Ser Gln Ser Ala Ala Leu Leu Ala Glu Ala
        35              40              45

Lys Lys Leu Asn Asp Ala Gln Ala Pro Lys
    50              55
```

<210> 10
<211> 58
<212> PRT
<213> Escherichia coli

<400> 10

```
Val Asp Ala Lys Phe Asp Lys Glu Ala Gln Glu Ala Phe Tyr Glu Ile
1               5               10              15

Leu His Leu Pro Asn Leu Thr Glu Glu Gln Arg Asn Ala Phe Ile Gln
            20              25              30

Ser Leu Lys Asp Asp Pro Ser Val Ser Lys Glu Ile Leu Ala Glu Ala
        35              40              45

Lys Lys Leu Asn Asp Ala Gln Ala Pro Lys
    50              55
```

<210> 11
<211> 58
<212> PRT
<213> Escherichia coli

<400> 11

```
Val Asp Ala Lys Phe Asp Lys Glu Ala Gln Glu Ala Phe Tyr Glu Ile
1               5               10              15

Leu His Leu Pro Asn Leu Thr Glu Glu Gln Arg Asn Ala Phe Ile Gln
            20              25              30

Ser Leu Lys Asp Asp Pro Ser Val Ser Lys Ala Ile Leu Ala Glu Ala
        35              40              45

Lys Lys Leu Asn Asp Ala Gln Ala Pro Lys
    50              55
```

<210> 12
<211> 58
<212> PRT
<213> Escherichia coli

<400> 12

```
Val Asp Ala Lys Phe Asp Lys Glu Gln Gln Glu Ala Phe Tyr Glu Ile
1               5                   10                  15

Leu His Leu Pro Asn Leu Thr Glu Glu Gln Arg Asn Ala Phe Ile Ala
            20                  25                  30

Ser Leu Lys Asp Asp Pro Ser Gln Ser Ala Asn Leu Leu Ala Glu Ala
        35                  40                  45

Lys Lys Leu Asn Asp Ala Gln Ala Pro Lys
    50                  55
```

<210> 13
<211> 58
<212> PRT
<213> Escherichia coli

<400> 13

```
Val Asp Ala Lys Phe Asp Lys Glu Gln Gln Glu Ala Phe Tyr Glu Ile
1               5                   10                  15

Leu His Leu Pro Asn Leu Thr Glu Glu Gln Arg Asn Ala Phe Ile Gln
            20                  25                  30

Ser Leu Lys Asp Asp Pro Ser Gln Ser Ala Asn Leu Leu Ala Glu Ala
        35                  40                  45

Lys Lys Leu Asn Asp Ala Gln Ala Pro Lys
    50                  55
```

<210> 14
<211> 58
<212> PRT
<213> Escherichia coli

<400> 14

```
Val Asp Ala Lys Phe Asp Lys Glu Gln Gln Glu Ala Phe Tyr Glu Ile
1               5               10                  15

Leu His Leu Pro Asn Leu Thr Glu Glu Gln Arg Asn Ala Phe Ile Glu
            20              25                  30

Ser Leu Lys Asp Asp Pro Ser Glu Ser Ala Asn Leu Leu Ala Glu Ala
            35              40                  45

Lys Lys Leu Asn Asp Ala Gln Ala Pro Lys
        50              55
```

<210> 15
<211> 58
<212> PRT
<213> Escherichia coli

<400> 15

```
Val Asp Ala Lys Phe Asp Lys Glu Gln Gln Glu Ala Phe Tyr Glu Ile
1               5               10                  15

Leu His Leu Pro Asn Leu Thr Glu Glu Gln Arg Asn Ala Phe Ile Glu
            20              25                  30

Ser Leu Lys Asp Asp Pro Ser Gln Ser Ala Asn Leu Leu Ala Glu Ala
            35              40                  45

Lys Arg Leu Asn Asp Ala Gln Ala Pro Lys
        50              55
```

<210> 16
<211> 58
<212> PRT
<213> Escherichia coli

<400> 16

```
Val Asp Ala Lys Phe Asp Lys Glu Gln Gln Lys Ala Phe Tyr Glu Ile
1               5               10                  15

Leu His Leu Pro Asn Leu Thr Glu Glu Gln Arg Asn Ala Phe Ile Gln
            20              25                  30

Ser Leu Lys Asp Asp Pro Ser Gln Ser Ala Asn Leu Leu Ala Glu Ala
            35              40                  45

Lys Lys Leu Asn Asp Ala Gln Ala Pro Lys
        50              55
```

43

<210> 17
<211> 237
<212> PRT
<213> Escherichia coli

<400> 17

```
Ala Gln Gly Thr Val Asp Ala Lys Phe Asp Lys Glu Ala Gln Glu Ala
1               5                   10                  15

Phe Tyr Glu Ile Leu His Leu Pro Asn Leu Thr Glu Glu Gln Arg Asn
            20                  25                  30

Ala Phe Ile Gln Ser Leu Lys Asp Asp Pro Ser Gln Ser Ala Ala Leu
            35                  40                  45

Leu Ala Glu Ala Lys Lys Leu Asn Asp Ala Gln Ala Pro Lys Val Asp
        50                  55                  60

Ala Lys Phe Asp Lys Glu Ala Gln Glu Ala Phe Tyr Glu Ile Leu His
65                  70                  75                  80

Leu Pro Asn Leu Thr Glu Glu Gln Arg Asn Ala Phe Ile Gln Ser Leu
                85                  90                  95

Lys Asp Asp Pro Ser Gln Ser Ala Ala Leu Leu Ala Glu Ala Lys Lys
            100                 105                 110

Leu Asn Asp Ala Gln Ala Pro Lys Val Asp Ala Lys Phe Asp Lys Glu
            115                 120                 125

Ala Gln Glu Ala Phe Tyr Glu Ile Leu His Leu Pro Asn Leu Thr Glu
            130                 135                 140

Glu Gln Arg Asn Ala Phe Ile Gln Ser Leu Lys Asp Asp Pro Ser Gln
145                 150                 155                 160

Ser Ala Ala Leu Leu Ala Glu Ala Lys Lys Leu Asn Asp Ala Gln Ala
                165                 170                 175

Pro Lys Val Asp Ala Lys Phe Asp Lys Glu Ala Gln Glu Ala Phe Tyr
            180                 185                 190

Glu Ile Leu His Leu Pro Asn Leu Thr Glu Glu Gln Arg Asn Ala Phe
            195                 200                 205

Ile Gln Ser Leu Lys Asp Asp Pro Ser Gln Ser Ala Ala Leu Leu Ala
            210                 215                 220
```

Glu Ala Lys Lys Leu Asn Asp Ala Gln Ala Pro Lys Cys
225                 230                 235

<210> 18
<211> 237
<212> PRT
<213> Escherichia coli

<400> 18

Ala Gln Gly Thr Val Asp Ala Lys Phe Asp Lys Glu Ala Gln Glu Ala
1                 5                 10                 15

Phe Tyr Glu Ile Leu His Leu Pro Asn Leu Thr Glu Glu Gln Arg Ala
                20                 25                 30

Ala Phe Ile Gln Ser Leu Lys Asp Asp Pro Ser Gln Ser Ala Ala Leu
            35                 40                 45

Leu Ala Glu Ala Lys Lys Leu Asn Asp Ala Gln Ala Pro Lys Val Asp
    50                 55                 60

Ala Lys Phe Asp Lys Glu Ala Gln Glu Ala Phe Tyr Glu Ile Leu His
65                 70                 75                 80

Leu Pro Asn Leu Thr Glu Glu Gln Arg Ala Ala Phe Ile Gln Ser Leu
                85                 90                 95

Lys Asp Asp Pro Ser Gln Ser Ala Ala Leu Leu Ala Glu Ala Lys Lys
                100                105                110

Leu Asn Asp Ala Gln Ala Pro Lys Val Asp Ala Lys Phe Asp Lys Glu
            115                120                125

Ala Gln Glu Ala Phe Tyr Glu Ile Leu His Leu Pro Asn Leu Thr Glu
    130                135                140

Glu Gln Arg Ala Ala Phe Ile Gln Ser Leu Lys Asp Asp Pro Ser Gln
145                150                155                160

Ser Ala Ala Leu Leu Ala Glu Ala Lys Lys Leu Asn Asp Ala Gln Ala
                165                170                175

Pro Lys Val Asp Ala Lys Phe Asp Lys Glu Ala Gln Glu Ala Phe Tyr
                180                185                190

Glu Ile Leu His Leu Pro Asn Leu Thr Glu Glu Gln Arg Ala Ala Phe
            195                200                205

```
Ile Gln Ser Leu Lys Asp Asp Pro Ser Gln Ser Ala Ala Leu Leu Ala
    210                 215             220

Glu Ala Lys Lys Leu Asn Asp Ala Gln Ala Pro Lys Cys
    225                 230             235
```

<210> 19
<211> 237
<212> PRT
<213> Escherichia coli

<400> 19

```
Ala Gln Gly Thr Val Asp Ala Lys Phe Asp Lys Glu Ala Gln Glu Ala
1               5               10              15

Phe Tyr Glu Ile Leu His Leu Pro Asn Leu Thr Glu Glu Gln Arg Asn
            20              25              30

Ala Phe Ile Gln Ser Leu Lys Asp Asp Pro Ser Val Ser Lys Glu Ile
        35              40              45

Leu Ala Glu Ala Lys Lys Leu Asn Asp Ala Gln Ala Pro Lys Val Asp
    50              55              60

Ala Lys Phe Asp Lys Glu Ala Gln Glu Ala Phe Tyr Glu Ile Leu His
65              70              75                          80

Leu Pro Asn Leu Thr Glu Glu Gln Arg Asn Ala Phe Ile Gln Ser Leu
                85              90                          95

Lys Asp Asp Pro Ser Val Ser Lys Glu Ile Leu Ala Glu Ala Lys Lys
            100             105             110

Leu Asn Asp Ala Gln Ala Pro Lys Val Asp Ala Lys Phe Asp Lys Glu
        115             120             125

Ala Gln Glu Ala Phe Tyr Glu Ile Leu His Leu Pro Asn Leu Thr Glu
    130             135             140

Glu Gln Arg Asn Ala Phe Ile Gln Ser Leu Lys Asp Asp Pro Ser Val
145             150             155             160

Ser Lys Glu Ile Leu Ala Glu Ala Lys Lys Leu Asn Asp Ala Gln Ala
                165             170             175

Pro Lys Val Asp Ala Lys Phe Asp Lys Glu Ala Gln Glu Ala Phe Tyr
            180             185             190
```

```
Glu Ile Leu His Leu Pro Asn Leu Thr Glu Glu Gln Arg Asn Ala Phe
    195                 200             205

Ile Gln Ser Leu Lys Asp Asp Pro Ser Val Ser Lys Glu Ile Leu Ala
    210                 215             220

Glu Ala Lys Lys Leu Asn Asp Ala Gln Ala Pro Lys Cys
225                 230             235
```

<210> 20
<211> 237
<212> PRT
<213> Escherichia coli

<400> 20

```
Ala Gln Gly Thr Val Asp Ala Lys Phe Asp Lys Glu Ala Gln Glu Ala
1               5               10              15

Phe Tyr Glu Ile Leu His Leu Pro Asn Leu Thr Glu Glu Gln Arg Asn
            20              25              30

Ala Phe Ile Gln Ser Leu Lys Asp Asp Pro Ser Val Ser Lys Ala Ile
        35              40              45

Leu Ala Glu Ala Lys Lys Leu Asn Asp Ala Gln Ala Pro Lys Val Asp
    50              55              60

Ala Lys Phe Asp Lys Glu Ala Gln Glu Ala Phe Tyr Glu Ile Leu His
65              70              75              80

Leu Pro Asn Leu Thr Glu Glu Gln Arg Asn Ala Phe Ile Gln Ser Leu
            85              90              95

Lys Asp Asp Pro Ser Val Ser Lys Ala Ile Leu Ala Glu Ala Lys Lys
            100             105             110

Leu Asn Asp Ala Gln Ala Pro Lys Val Asp Ala Lys Phe Asp Lys Glu
            115             120             125

Ala Gln Glu Ala Phe Tyr Glu Ile Leu His Leu Pro Asn Leu Thr Glu
        130             135             140

Glu Gln Arg Asn Ala Phe Ile Gln Ser Leu Lys Asp Asp Pro Ser Val
145             150             155             160

Ser Lys Ala Ile Leu Ala Glu Ala Lys Lys Leu Asn Asp Ala Gln Ala
            165             170             175
```

```
        Pro Lys Val Asp Ala Lys Phe Asp Lys Glu Ala Gln Glu Ala Phe Tyr
                    180                 185                 190

        Glu Ile Leu His Leu Pro Asn Leu Thr Glu Glu Gln Arg Asn Ala Phe
                    195                 200                 205

        Ile Gln Ser Leu Lys Asp Asp Pro Ser Val Ser Lys Ala Ile Leu Ala
                    210                 215                 220

        Glu Ala Lys Lys Leu Asn Asp Ala Gln Ala Pro Lys Cys
        225                 230                 235
```

<210> 21
<211> 237
<212> PRT
<213> Escherichia coli

<400> 21

```
        Ala Gln Gly Thr Val Asp Ala Lys Phe Asp Lys Glu Gln Gln Asn Ala
        1               5                   10                  15

        Phe Tyr Glu Ile Leu His Leu Pro Asn Leu Thr Glu Glu Gln Arg Asn
                    20                  25                  30

        Ala Phe Ile Gln Ser Leu Lys Asp Asp Pro Ser Gln Ser Ala Asn Leu
                    35                  40                  45

        Leu Ala Glu Ala Lys Lys Leu Asn Asp Ala Gln Ala Pro Lys Val Asp
                    50                  55                  60

        Ala Lys Phe Asp Lys Glu Gln Gln Asn Ala Phe Tyr Glu Ile Leu His
        65                  70                  75                  80

        Leu Pro Asn Leu Thr Glu Glu Gln Arg Asn Ala Phe Ile Gln Ser Leu
                    85                  90                  95

        Lys Asp Asp Pro Ser Gln Ser Ala Asn Leu Leu Ala Glu Ala Lys Lys
                    100                 105                 110

        Leu Asn Asp Ala Gln Ala Pro Lys Val Asp Ala Lys Phe Asp Lys Glu
                    115                 120                 125

        Gln Gln Asn Ala Phe Tyr Glu Ile Leu His Leu Pro Asn Leu Thr Glu
                    130                 135                 140

        Glu Gln Arg Asn Ala Phe Ile Gln Ser Leu Lys Asp Asp Pro Ser Gln
        145                 150                 155                 160
```

```
        Ser Ala Asn Leu Leu Ala Glu Ala Lys Lys Leu Asn Asp Ala Gln Ala
                        165                 170                 175

        Pro Lys Val Asp Ala Lys Phe Asp Lys Glu Gln Gln Asn Ala Phe Tyr
                        180                 185                 190

        Glu Ile Leu His Leu Pro Asn Leu Thr Glu Glu Gln Arg Asn Ala Phe
                        195                 200                 205

        Ile Gln Ser Leu Lys Asp Asp Pro Ser Gln Ser Ala Asn Leu Leu Ala
                        210                 215                 220

        Glu Ala Lys Lys Leu Asn Asp Ala Gln Ala Pro Lys Cys
        225                 230                 235
```

<210> 22
<211> 58
<212> PRT
<213> Staphylococcus aureus

<400> 22

```
        Ala Asp Asn Asn Phe Asn Lys Glu Gln Gln Asn Ala Phe Tyr Glu Ile
        1                   5                   10                  15

        Leu Asn Met Pro Asn Leu Asn Glu Glu Gln Arg Asn Gly Phe Ile Gln
                        20                  25                  30

        Ser Leu Lys Asp Asp Pro Ser Gln Ser Ala Asn Leu Leu Ser Glu Ala
                        35                  40                  45

        Lys Lys Leu Asn Glu Ser Gln Ala Pro Lys
                50                  55
```

<210> 23
<211> 58
<212> PRT
<213> Escherichia coli

<400> 23

```
        Val Asp Ala Lys Phe Asp Lys Glu Gln Gln Lys Ala Phe Tyr Glu Ile
        1                   5                   10                  15

        Leu Lys Leu Pro Asn Leu Thr Glu Glu Gln Arg Asn Ala Phe Ile Gln
                        20                  25                  30

        Lys Leu Lys Asp Glu Pro Ser Gln Ser Arg Ala Ile Leu Ala Glu Ala
                        35                  40                  45
```

```
            Lys Arg Tyr Asn Asp Ala Gln Ala Pro Lys
                50                  55
```

<210> 24
<211> 58
<212> PRT
<213> Escherichia coli

<400> 24

```
        Val Asp Ala Lys Phe Asp Lys Glu Ala Gln Glu Ala Phe Tyr Glu Ile
        1               5                   10                  15

        Leu His Leu Pro Asn Leu Thr Glu Glu Gln Arg Ala Ala Phe Ile Gln
                        20                  25                  30

        Ser Leu Lys Asp Asp Pro Ser Val Ser Lys Ala Ile Leu Ala Glu Ala
                    35                  40                  45

        Lys Lys Leu Asn Asp Ala Gln Ala Pro Lys
            50                  55
```

<210> 25
<211> 58
<212> PRT
<213> Escherichia coli

<400> 25

```
        Val Asp Ala Lys Phe Asp Lys Glu Ala Gln Lys Ala Phe Tyr Glu Ile
        1               5                   10                  15

        Leu Lys Leu Pro Asn Leu Thr Glu Glu Gln Arg Ala Ala Phe Ile Gln
                        20                  25                  30

        Lys Leu Lys Asp Glu Pro Ser Gln Ser Arg Ala Ile Leu Ala Glu Ala
                    35                  40                  45

        Lys Arg Tyr Asn Asp Ala Gln Ala Pro Lys
            50                  55
```

<210> 26
<211> 58
<212> PRT
<213> Escherichia coli

<400> 26

```
Val Asp Ala Lys Phe Asp Lys Glu Gln Gln Lys Ala Phe Tyr Glu Ile
1               5                   10                  15


Leu Lys Leu Pro Asn Leu Thr Glu Glu Gln Arg Asn Ala Phe Ile Gln

            20                  25                  30


Ser Leu Lys Asp Glu Pro Ser Gln Ser Arg Ala Ile Leu Ala Glu Ala
            35                  40                  45


Lys Lys Leu Asn Asp Ala Gln Ala Pro Lys
        50                  55
```

<210> 27
<211> 58
<212> PRT
<213> Escherichia coli

<400> 27

```
Val Asp Ala Lys Phe Asp Lys Glu Ala Gln Lys Ala Phe Tyr Glu Ile
1               5                   10                  15


Leu Lys Leu Pro Asn Leu Thr Glu Glu Gln Arg Asn Ala Phe Ile Gln
            20                  25                  30


Ser Leu Lys Asp Glu Pro Ser Gln Ser Arg Ala Ile Leu Ala Glu Ala
            35                  40                  45


Lys Lys Leu Asn Asp Ala Gln Ala Pro Lys
        50                  55
```

<210> 28
<211> 58
<212> PRT
<213> Escherichia coli

<400> 28

```
Val Asp Ala Lys Phe Asp Lys Glu Ala Gln Lys Ala Phe Tyr Glu Ile
1               5               10              15

Leu Lys Leu Pro Asn Leu Thr Glu Glu Gln Arg Asn Ala Phe Ile Gln
            20              25              30

Ser Leu Lys Asp Glu Pro Ser Gln Ser Arg Ala Ile Leu Ala Glu Ala
            35              40              45

Lys Arg Leu Asn Asp Ala Gln Ala Pro Lys
            50              55
```

<210> 29
<211> 58
<212> PRT
<213> Escherichia coli

<400> 29

```
Val Asp Ala Lys Phe Asp Lys Glu Ala Gln Lys Ala Phe Tyr Glu Ile
1               5               10              15

Leu Lys Leu Pro Asn Leu Thr Glu Glu Gln Arg Asn Ala Phe Ile Gln
            20              25              30

Ser Leu Lys Asp Glu Pro Ser Gln Ser Arg Asn Leu Leu Ala Glu Ala
            35              40              45

Lys Lys Leu Asn Asp Ala Gln Ala Pro Lys
            50              55
```

<210> 30
<211> 237
<212> PRT
<213> Escherichia coli

<400> 30

52

EP 3 455 242 B1

```
Ala Gln Gly Thr Val Asp Ala Lys Phe Asp Lys Glu Gln Gln Lys Ala
1               5                   10                  15

Phe Tyr Glu Ile Leu Lys Leu Pro Asn Leu Thr Glu Glu Gln Arg Asn
            20              25                  30

Ala Phe Ile Gln Lys Leu Lys Asp Glu Pro Ser Gln Ser Arg Ala Ile
        35                  40                  45

Leu Ala Glu Ala Lys Arg Tyr Asn Asp Ala Gln Ala Pro Lys Val Asp
    50                  55                  60

Ala Lys Phe Asp Lys Glu Gln Gln Lys Ala Phe Tyr Glu Ile Leu Lys
65                  70                  75                  80

Leu Pro Asn Leu Thr Glu Glu Gln Arg Asn Ala Phe Ile Gln Lys Leu
                85                  90                  95

Lys Asp Glu Pro Ser Gln Ser Arg Ala Ile Leu Ala Glu Ala Lys Arg
                100                 105                 110

Tyr Asn Asp Ala Gln Ala Pro Lys Val Asp Ala Lys Phe Asp Lys Glu
        115                 120                 125

Gln Gln Lys Ala Phe Tyr Glu Ile Leu Lys Leu Pro Asn Leu Thr Glu
    130                 135                 140

Glu Gln Arg Asn Ala Phe Ile Gln Lys Leu Lys Asp Glu Pro Ser Gln
145                 150                 155                 160

Ser Arg Ala Ile Leu Ala Glu Ala Lys Arg Tyr Asn Asp Ala Gln Ala
                165                 170                 175

Pro Lys Val Asp Ala Lys Phe Asp Lys Glu Gln Gln Lys Ala Phe Tyr
        180                 185                 190

Glu Ile Leu Lys Leu Pro Asn Leu Thr Glu Glu Gln Arg Asn Ala Phe
        195                 200                 205

Ile Gln Lys Leu Lys Asp Glu Pro Ser Gln Ser Arg Ala Ile Leu Ala
    210                 215                 220

Glu Ala Lys Arg Tyr Asn Asp Ala Gln Ala Pro Lys Cys
225                 230                 235
```

<210> 31
<211> 237
<212> PRT

53

<213> Escherichia coli

<400> 31

```
Ala Gln Gly Thr Val Asp Ala Lys Phe Asp Lys Glu Ala Gln Lys Ala
1               5                   10                  15

Phe Tyr Glu Ile Leu Lys Leu Pro Asn Leu Thr Glu Glu Gln Arg Asn
            20                  25                  30

Ala Phe Ile Gln Ser Leu Lys Asp Glu Pro Ser Gln Ser Arg Asn Leu
        35                  40                  45

Leu Ala Glu Ala Lys Lys Leu Asn Asp Ala Gln Ala Pro Lys Val Asp
    50                  55                  60

Ala Lys Phe Asp Lys Glu Ala Gln Lys Ala Phe Tyr Glu Ile Leu Lys
65                  70                  75                  80

Leu Pro Asn Leu Thr Glu Glu Gln Arg Asn Ala Phe Ile Gln Ser Leu
            85                  90                  95

Lys Asp Glu Pro Ser Gln Ser Arg Asn Leu Leu Ala Glu Ala Lys Lys
            100                 105                 110

Leu Asn Asp Ala Gln Ala Pro Lys Val Asp Ala Lys Phe Asp Lys Glu
            115                 120                 125

Ala Gln Lys Ala Phe Tyr Glu Ile Leu Lys Leu Pro Asn Leu Thr Glu
    130                 135                 140

Glu Gln Arg Asn Ala Phe Ile Gln Ser Leu Lys Asp Glu Pro Ser Gln
145                 150                 155                 160

Ser Arg Asn Leu Leu Ala Glu Ala Lys Lys Leu Asn Asp Ala Gln Ala
            165                 170                 175

Pro Lys Val Asp Ala Lys Phe Asp Lys Glu Ala Gln Lys Ala Phe Tyr
            180                 185                 190

Glu Ile Leu Lys Leu Pro Asn Leu Thr Glu Glu Gln Arg Asn Ala Phe
            195                 200                 205

Ile Gln Ser Leu Lys Asp Glu Pro Ser Gln Ser Arg Asn Leu Leu Ala
            210                 215                 220

Glu Ala Lys Lys Leu Asn Asp Ala Gln Ala Pro Lys Cys
225                 230                 235
```

<210> 32
<211> 237
<212> PRT
<213> Escherichia coli

<400> 32

```
Ala Gln Gly Thr Val Asp Ala Lys Phe Asp Lys Glu Ala Gln Glu Ala
1               5                   10                  15

Phe Tyr Glu Ile Leu His Leu Pro Asn Leu Thr Glu Glu Gln Arg Ala
            20                  25                  30

Ala Phe Ile Gln Ser Leu Lys Asp Asp Pro Ser Val Ser Lys Ala Ile
            35                  40                  45

Leu Ala Glu Ala Lys Lys Leu Asn Asp Ala Gln Ala Pro Lys Val Asp
        50                  55                  60

Ala Lys Phe Asp Lys Glu Ala Gln Glu Ala Phe Tyr Glu Ile Leu His
65                  70                  75                  80

Leu Pro Asn Leu Thr Glu Glu Gln Arg Ala Ala Phe Ile Gln Ser Leu
                85                  90                  95

Lys Asp Asp Pro Ser Val Ser Lys Ala Ile Leu Ala Glu Ala Lys Lys
            100                 105                 110

Leu Asn Asp Ala Gln Ala Pro Lys Val Asp Ala Lys Phe Asp Lys Glu
            115                 120                 125
```

```
        Ala Gln Glu Ala Phe Tyr Glu Ile Leu His Leu Pro Asn Leu Thr Glu
            130                 135                 140

        Glu Gln Arg Ala Ala Phe Ile Gln Ser Leu Lys Asp Asp Pro Ser Val
            145                 150                 155                 160

        Ser Lys Ala Ile Leu Ala Glu Ala Lys Lys Leu Asn Asp Ala Gln Ala
                            165                 170                 175

        Pro Lys Val Asp Ala Lys Phe Asp Lys Glu Ala Gln Glu Ala Phe Tyr
                            180                 185                 190

        Glu Ile Leu His Leu Pro Asn Leu Thr Glu Glu Gln Arg Ala Ala Phe
                            195                 200                 205

        Ile Gln Ser Leu Lys Asp Asp Pro Ser Val Ser Lys Ala Ile Leu Ala
            210                 215                 220

        Glu Ala Lys Lys Leu Asn Asp Ala Gln Ala Pro Lys Cys
            225                 230                 235
```

<210> 33
<211> 353
<212> PRT
<213> Escherichia coli

<400> 33

```
        Ala Gln Gly Thr Val Asp Ala Lys Phe Asp Lys Glu Ala Gln Glu Ala
        1                   5                   10                  15

        Phe Tyr Glu Ile Leu His Leu Pro Asn Leu Thr Glu Glu Gln Arg Asn
                            20                  25                  30

        Ala Phe Ile Gln Ser Leu Lys Asp Asp Pro Ser Val Ser Lys Ala Ile
                            35                  40                  45

        Leu Ala Glu Ala Lys Lys Leu Asn Asp Ala Gln Ala Pro Lys Val Asp
            50                  55                  60

        Ala Lys Phe Asp Lys Glu Ala Gln Glu Ala Phe Tyr Glu Ile Leu His
        65                  70                  75                  80

        Leu Pro Asn Leu Thr Glu Glu Gln Arg Asn Ala Phe Ile Gln Ser Leu
                            85                  90                  95

        Lys Asp Asp Pro Ser Val Ser Lys Ala Ile Leu Ala Glu Ala Lys Lys
                            100                 105                 110
```

```
Leu Asn Asp Ala Gln Ala Pro Lys Val Asp Ala Lys Phe Asp Lys Glu
        115                 120                 125

Ala Gln Glu Ala Phe Tyr Glu Ile Leu His Leu Pro Asn Leu Thr Glu
        130                 135                 140

Glu Gln Arg Asn Ala Phe Ile Gln Ser Leu Lys Asp Asp Pro Ser Val
145                 150                 155                 160

Ser Lys Ala Ile Leu Ala Glu Ala Lys Lys Leu Asn Asp Ala Gln Ala
                165                 170                 175

Pro Lys Val Asp Ala Lys Phe Asp Lys Glu Ala Gln Glu Ala Phe Tyr
            180                 185                 190

Glu Ile Leu His Leu Pro Asn Leu Thr Glu Glu Gln Arg Asn Ala Phe
            195                 200                 205

Ile Gln Ser Leu Lys Asp Asp Pro Ser Val Ser Lys Ala Ile Leu Ala
    210                 215                 220

Glu Ala Lys Lys Leu Asn Asp Ala Gln Ala Pro Lys Val Asp Ala Lys
225                 230                 235                 240

Phe Asp Lys Glu Ala Gln Glu Ala Phe Tyr Glu Ile Leu His Leu Pro
            245                 250                 255

Asn Leu Thr Glu Glu Gln Arg Asn Ala Phe Ile Gln Ser Leu Lys Asp
            260                 265                 270

Asp Pro Ser Val Ser Lys Ala Ile Leu Ala Glu Ala Lys Lys Leu Asn
            275                 280                 285

Asp Ala Gln Ala Pro Lys Val Asp Ala Lys Phe Asp Lys Glu Ala Gln
        290                 295                 300

Glu Ala Phe Tyr Glu Ile Leu His Leu Pro Asn Leu Thr Glu Glu Gln
305                 310                 315                 320

Arg Asn Ala Phe Ile Gln Ser Leu Lys Asp Asp Pro Ser Val Ser Lys
                325                 330                 335

Ala Ile Leu Ala Glu Ala Lys Lys Leu Asn Asp Ala Gln Ala Pro Lys
            340                 345                 350

Cys
```

<210> 34
<211> 237
<212> PRT
<213> Escherichia coli

<400> 34

```
Ala Gln Gly Thr Val Asp Ala Lys Phe Asp Lys Glu Ala Gln Glu Ala
1               5                   10                  15

Phe Tyr Glu Ile Leu His Leu Pro Asn Leu Thr Glu Glu Gln Arg Asn
            20                  25                  30

Ala Phe Ile Gln Ser Leu Lys Asp Glu Pro Ser Val Ser Lys Ala Ile
        35                  40                  45

Leu Ala Glu Ala Lys Lys Leu Asn Asp Ala Gln Ala Pro Lys Val Asp
    50                  55                  60

Ala Lys Phe Asp Lys Glu Ala Gln Glu Ala Phe Tyr Glu Ile Leu His
65                  70                  75                  80

Leu Pro Asn Leu Thr Glu Glu Gln Arg Asn Ala Phe Ile Gln Ser Leu
                85                  90                  95

Lys Asp Glu Pro Ser Val Ser Lys Ala Ile Leu Ala Glu Ala Lys Lys
            100                 105                 110

Leu Asn Asp Ala Gln Ala Pro Lys Val Asp Ala Lys Phe Asp Lys Glu
        115                 120                 125

Ala Gln Glu Ala Phe Tyr Glu Ile Leu His Leu Pro Asn Leu Thr Glu
        130                 135                 140

Glu Gln Arg Asn Ala Phe Ile Gln Ser Leu Lys Asp Glu Pro Ser Val
145                 150                 155                 160

Ser Lys Ala Ile Leu Ala Glu Ala Lys Lys Leu Asn Asp Ala Gln Ala
            165                 170                 175

Pro Lys Val Asp Ala Lys Phe Asp Lys Glu Ala Gln Glu Ala Phe Tyr
            180                 185                 190

Glu Ile Leu His Leu Pro Asn Leu Thr Glu Glu Gln Arg Asn Ala Phe
            195                 200                 205

Ile Gln Ser Leu Lys Asp Glu Pro Ser Val Ser Lys Ala Ile Leu Ala
            210                 215                 220
```

```
                    Glu Ala Lys Lys Leu Asn Asp Ala Gln Ala Pro Lys Cys
                    225             230             235
```

<210> 35
<211> 237
<212> PRT
<213> Escherichia coli

<400> 35

```
        Ala Gln Gly Thr Val Asp Ala Lys Phe Asp Lys Glu Ala Gln Glu Ala
        1               5               10              15


        Phe Tyr Glu Ile Leu His Leu Pro Asn Leu Thr Glu Glu Gln Arg Asn
                    20              25              30


        Ala Phe Ile Gln Ser Leu Lys Asp Glu Pro Ser Val Ser Arg Ala Ile
                    35              40              45


        Leu Ala Glu Ala Lys Lys Leu Asn Asp Ala Gln Ala Pro Lys Val Asp
            50              55              60


        Ala Lys Phe Asp Lys Glu Ala Gln Glu Ala Phe Tyr Glu Ile Leu His
        65              70              75              80


        Leu Pro Asn Leu Thr Glu Glu Gln Arg Asn Ala Phe Ile Gln Ser Leu
                    85              90              95


        Lys Asp Glu Pro Ser Val Ser Arg Ala Ile Leu Ala Glu Ala Lys Lys
                    100             105             110


        Leu Asn Asp Ala Gln Ala Pro Lys Val Asp Ala Lys Phe Asp Lys Glu
                    115             120             125


        Ala Gln Glu Ala Phe Tyr Glu Ile Leu His Leu Pro Asn Leu Thr Glu
                    130             135             140


        Glu Gln Arg Asn Ala Phe Ile Gln Ser Leu Lys Asp Glu Pro Ser Val
        145             150             155             160


        Ser Arg Ala Ile Leu Ala Glu Ala Lys Lys Leu Asn Asp Ala Gln Ala
                    165             170             175


        Pro Lys Val Asp Ala Lys Phe Asp Lys Glu Ala Gln Glu Ala Phe Tyr
                    180             185             190


        Glu Ile Leu His Leu Pro Asn Leu Thr Glu Glu Gln Arg Asn Ala Phe
                    195             200             205
```

```
Ile Gln Ser Leu Lys Asp Glu Pro Ser Val Ser Arg Ala Ile Leu Ala
    210             215             220
```

```
Glu Ala Lys Lys Leu Asn Asp Ala Gln Ala Pro Lys Cys
225             230             235
```

<210> 36
<211> 58
<212> PRT
<213> Escherichia coli

<400> 36

```
Ala Asp Asn Lys Phe Asn Lys Glu Ala Gln Glu Ala Phe Tyr Glu Ile
1               5               10              15
```

```
Leu His Leu Pro Asn Leu Asn Glu Glu Gln Arg Asn Gly Phe Ile Gln
            20              25              30
```

```
Ser Leu Lys Asp Asp Pro Ser Val Ser Lys Ala Ile Leu Ala Glu Ala
            35              40              45
```

```
Lys Lys Leu Asn Asp Ala Gln Ala Pro Lys
    50              55
```

<210> 37
<211> 58
<212> PRT
<213> Escherichia coli

<400> 37

```
Ala Asp Asn Lys Phe Asn Lys Glu Ala Gln Glu Ala Phe Tyr Glu Ile
1               5               10              15
```

```
Leu His Leu Pro Asn Leu Thr Glu Glu Gln Arg Asn Gly Phe Ile Gln
            20              25              30
```

```
Ser Leu Lys Asp Asp Pro Ser Val Ser Lys Ala Ile Leu Ala Glu Ala
            35              40              45
```

```
Lys Lys Leu Asn Asp Ala Gln Ala Pro Lys
    50              55
```

<210> 38
<211> 58
<212> PRT
<213> Escherichia coli

<400> 38

```
Val Asp Ala Lys Phe Asp Lys Glu Gln Gln Tyr Ala Phe Tyr Glu Ile
1               5               10              15

Leu His Leu Pro Asn Leu Thr Glu Glu Gln Arg Asn Ala Phe Ile Gln
            20              25              30

Ser Leu Lys Asp Asp Pro Ser Gln Ser Ala Asn Leu Leu Ala Glu Ala
            35              40              45

Lys Lys Leu Asn Asp Ala Gln Ala Pro Lys
        50              55
```

<210> 39
<211> 58
<212> PRT
<213> Escherichia coli

<400> 39

```
Val Asp Ala Lys Phe Asp Lys Glu Gln Gln Thr Ala Phe Tyr Glu Ile
1               5               10              15

Leu His Leu Pro Asn Leu Thr Glu Glu Gln Arg Asn Ala Phe Ile Gln
            20              25              30

Ser Leu Lys Asp Asp Pro Ser Gln Ser Ala Asn Leu Leu Ala Glu Ala
            35              40              45

Lys Lys Leu Asn Asp Ala Gln Ala Pro Lys
        50              55
```

<210> 40
<211> 58
<212> PRT
<213> Escherichia coli

<400> 40

```
Val Asp Ala Lys Phe Asp Lys Glu Gln Gln Phe Ala Phe Tyr Glu Ile
1               5               10              15

Leu His Leu Pro Asn Leu Thr Glu Glu Gln Arg Asn Ala Phe Ile Gln
            20              25              30

Ser Leu Lys Asp Asp Pro Ser Gln Ser Ala Asn Leu Leu Ala Glu Ala
            35              40              45

Lys Lys Leu Asn Asp Ala Gln Ala Pro Lys
    50              55
```

<210> 41

<211> 58
<212> PRT
<213> Escherichia coli

<400> 41

```
Val Asp Ala Lys Phe Asp Lys Glu Gln Gln Leu Ala Phe Tyr Glu Ile
1               5               10              15

Leu His Leu Pro Asn Leu Thr Glu Glu Gln Arg Asn Ala Phe Ile Gln
            20              25              30

Ser Leu Lys Asp Asp Pro Ser Gln Ser Ala Asn Leu Leu Ala Glu Ala
            35              40              45

Lys Lys Leu Asn Asp Ala Gln Ala Pro Lys
    50              55
```

<210> 42
<211> 58
<212> PRT
<213> Escherichia coli

<400> 42

```
Val Asp Ala Lys Phe Asp Lys Glu Gln Gln Trp Ala Phe Tyr Glu Ile
1               5                   10                  15

Leu His Leu Pro Asn Leu Thr Glu Glu Gln Arg Asn Ala Phe Ile Gln
            20                  25                  30

Ser Leu Lys Asp Asp Pro Ser Gln Ser Ala Asn Leu Leu Ala Glu Ala
        35                  40                  45

Lys Lys Leu Asn Asp Ala Gln Ala Pro Lys
    50                  55
```

<210> 43
<211> 58
<212> PRT
<213> Escherichia coli

<400> 43

```
Val Asp Ala Lys Phe Asp Lys Glu Gln Gln Ile Ala Phe Tyr Glu Ile
1               5                   10                  15

Leu His Leu Pro Asn Leu Thr Glu Glu Gln Arg Asn Ala Phe Ile Gln
            20                  25                  30

Ser Leu Lys Asp Asp Pro Ser Gln Ser Ala Asn Leu Leu Ala Glu Ala
        35                  40                  45

Lys Lys Leu Asn Asp Ala Gln Ala Pro Lys
    50                  55
```

<210> 44
<211> 58
<212> PRT
<213> Escherichia coli

<400> 44

```
Val Asp Ala Lys Phe Asp Lys Glu Gln Gln Met Ala Phe Tyr Glu Ile
1               5                   10                  15

Leu His Leu Pro Asn Leu Thr Glu Glu Gln Arg Asn Ala Phe Ile Gln
            20                  25                  30

Ser Leu Lys Asp Asp Pro Ser Gln Ser Ala Asn Leu Leu Ala Glu Ala
        35                  40                  45

Lys Lys Leu Asn Asp Ala Gln Ala Pro Lys
    50                  55
```

<210> 45
<211> 58
<212> PRT
<213> Escherichia coli

<400> 45

```
Val Asp Ala Lys Phe Asp Lys Glu Gln Gln Val Ala Phe Tyr Glu Ile
1               5               10                  15

Leu His Leu Pro Asn Leu Thr Glu Glu Gln Arg Asn Ala Phe Ile Gln
            20              25                  30

Ser Leu Lys Asp Asp Pro Ser Gln Ser Ala Asn Leu Leu Ala Glu Ala
            35              40                  45

Lys Lys Leu Asn Asp Ala Gln Ala Pro Lys
            50              55
```

<210> 46
<211> 58
<212> PRT
<213> Escherichia coli

<400> 46

```
Val Asp Ala Lys Phe Asp Lys Glu Gln Gln Ala Ala Phe Tyr Glu Ile
1               5               10                  15

Leu His Leu Pro Asn Leu Thr Glu Glu Gln Arg Asn Ala Phe Ile Gln

            20              25                  30

Ser Leu Lys Asp Asp Pro Ser Gln Ser Ala Asn Leu Leu Ala Glu Ala
            35              40                  45

Lys Lys Leu Asn Asp Ala Gln Ala Pro Lys
            50              55
```

<210> 47
<211> 58
<212> PRT
<213> Escherichia coli

<400> 47

```
Val Asp Ala Lys Phe Asp Lys Glu Gln Gln His Ala Phe Tyr Glu Ile
1               5               10              15

Leu His Leu Pro Asn Leu Thr Glu Glu Gln Arg Asn Ala Phe Ile Gln
            20              25              30

Ser Leu Lys Asp Asp Pro Ser Gln Ser Ala Asn Leu Leu Ala Glu Ala
            35              40              45

Lys Lys Leu Asn Asp Ala Gln Ala Pro Lys
    50              55
```

<210> 48
<211> 58
<212> PRT
<213> Escherichia coli

<400> 48

```
Val Asp Ala Lys Phe Asp Lys Glu Gln Gln Arg Ala Phe Tyr Glu Ile
1               5               10              15

Leu His Leu Pro Asn Leu Thr Glu Glu Gln Arg Asn Ala Phe Ile Gln
            20              25              30

Ser Leu Lys Asp Asp Pro Ser Gln Ser Ala Asn Leu Leu Ala Glu Ala
            35              40              45

Lys Lys Leu Asn Asp Ala Gln Ala Pro Lys
    50              55
```

<210> 49
<211> 58
<212> PRT
<213> Escherichia coli

<400> 49

```
Val Asp Ala Lys Phe Asp Lys Glu Ala Gln Glu Ala Phe Tyr Glu Ile
1               5               10              15

Leu His Leu Pro Asn Leu Thr Glu Glu Gln Arg Asn Ala Phe Ile Gln
            20              25              30

Ser Leu Lys Asp Glu Pro Ser Val Ser Lys Ala Ile Leu Ala Glu Ala
            35              40              45

Lys Lys Leu Asn Asp Ala Gln Ala Pro Lys
    50              55
```

<210> 50
<211> 58
<212> PRT
<213> Escherichia coli

<400> 50

| Val | Asp | Ala | Lys | Phe | Asp | Lys | Glu | Ala | Gln | Glu | Ala | Phe | Tyr | Glu | Ile |
| 1 | | | | 5 | | | | | 10 | | | | | 15 | |

| Leu | His | Leu | Pro | Asn | Leu | Thr | Glu | Glu | Gln | Arg | Asn | Ala | Phe | Ile | Gln |
| | | | 20 | | | | | 25 | | | | | 30 | | |

| Ser | Leu | Lys | Asp | Glu | Pro | Ser | Val | Ser | Arg | Ala | Ile | Leu | Ala | Glu | Ala |
| | | 35 | | | | | 40 | | | | | 45 | | | |

| Lys | Lys | Leu | Asn | Asp | Ala | Gln | Ala | Pro | Lys |
| | 50 | | | | | 55 | | | |

<210> 51
<211> 47
<212> PRT
<213> Escherichia coli

<400> 51

| Gln | Gln | Asn | Ala | Phe | Tyr | Glu | Ile | Leu | His | Leu | Pro | Asn | Leu | Thr | Glu |
| 1 | | | | 5 | | | | | 10 | | | | | 15 | |

| Glu | Gln | Arg | Asn | Ala | Phe | Ile | Gln | Ser | Leu | Lys | Asp | Asp | Pro | Ser | Gln |
| | | 20 | | | | | 25 | | | | | 30 | | | |

| Ser | Ala | Asn | Leu | Leu | Ala | Glu | Ala | Lys | Lys | Leu | Asn | Asp | Ala | Gln |
| | | 35 | | | | | 40 | | | | | 45 | | |

<210> 52
<211> 47
<212> PRT
<213> Staphylococcus aureus

<400> 52

| Gln | Gln | Asn | Ala | Phe | Tyr | Glu | Ile | Leu | His | Leu | Pro | Asn | Leu | Thr | Glu |
| 1 | | | | 5 | | | | | 10 | | | | | 15 | |

| Glu | Gln | Arg | Asn | Gly | Phe | Ile | Gln | Ser | Leu | Lys | Asp | Asp | Pro | Ser | Val |
| | | 20 | | | | | 25 | | | | | 30 | | | |

| Ser | Lys | Glu | Ile | Leu | Ala | Glu | Ala | Lys | Lys | Leu | Asn | Asp | Ala | Gln |
| | | 35 | | | | | 40 | | | | | 45 | | |

<210> 53
<211> 47
<212> PRT
<213> Escherichia coli

<220>
<221> misc_feature
<222> (1)..(1)
<223> Xaa can be any naturally occurring amino acid

<220>
<221> misc_feature
<222> (3)..(3)
<223> Xaa can be any naturally occurring amino acid

<220>
<221> misc_feature
<222> (10)..(10)
<223> Xaa can be any naturally occurring amino acid

<220>
<221> misc_feature
<222> (20) .. (21)
<223> Xaa can be any naturally occurring amino acid

<220>
<221> misc_feature
<222> (24) .. (25)
<223> Xaa can be any naturally occurring amino acid

<220>
<221> misc_feature
<222> (29) .. (29)
<223> Xaa can be any naturally occurring amino acid

<220>
<221> misc_feature
<222> (32)..(32)
<223> Xaa can be any naturally occurring amino acid

<220>
<221> misc_feature
<222> (34)..(36)
<223> Xaa can be any naturally occurring amino acid

<220>
<221> misc_feature
<222> (42)..(43)
<223> Xaa can be any naturally occurring amino acid

<220>
<221> misc_feature
<222> (45)..(45)
<223> Xaa can be any naturally occurring amino acid

<400> 53

```
        Xaa Gln Xaa Ala Phe Tyr Glu Ile Leu Xaa Leu Pro Asn Leu Thr Glu
        1               5                   10                  15

        Glu Gln Arg Xaa Xaa Phe Ile Xaa Xaa Leu Lys Asp Xaa Pro Ser Xaa
                    20              25                  30

        Ser Xaa Xaa Xaa Leu Ala Glu Ala Lys Xaa Xaa Asn Xaa Ala Gln
            35                  40                  45
```

<210> 54
<211> 58
<212> PRT
<213> Escherichia coli

<400> 54

```
        Val Asp Ala Lys Phe Asp Lys Glu Ala Gln Glu Ala Phe Tyr Glu Ile
        1               5                   10                  15

        Leu His Leu Pro Asn Leu Thr Glu Glu Gln Arg Asn Gly Phe Ile Gln
                    20              25                  30

        Ser Leu Lys Asp Asp Pro Ser Val Ser Lys Ala Ile Leu Ala Glu Ala
            35                  40                  45

        Lys Lys Leu Asn Asp Ala Gln Ala Pro Lys
            50                  55
```

<210> 55
<211> 58
<212> PRT
<213> Escherichia coli

<400> 55

```
        Val Asp Ala Lys Phe Asp Lys Glu Ala Gln Glu Ala Phe Tyr Glu Ile
        1               5                   10                  15

        Leu His Leu Pro Asn Leu Thr Glu Glu Gln Arg Asn Ser Phe Ile Gln
                    20              25                  30

        Ser Leu Lys Asp Asp Pro Ser Val Ser Lys Ala Ile Leu Ala Glu Ala
            35                  40                  45

        Lys Lys Leu Asn Asp Ala Gln Ala Pro Lys
            50                  55
```

<210> 56
<211> 58
<212> PRT
<213> Escherichia coli

<400> 56

```
Val Asp Ala Lys Phe Asp Lys Glu Ala Gln Glu Ala Phe Tyr Glu Ile
1               5                   10                  15

Leu His Leu Pro Asn Leu Thr Glu Glu Gln Arg Asn Tyr Phe Ile Gln
            20                  25                  30

Ser Leu Lys Asp Asp Pro Ser Val Ser Lys Ala Ile Leu Ala Glu Ala
        35                  40                  45

Lys Lys Leu Asn Asp Ala Gln Ala Pro Lys
    50                  55
```

<210> 57
<211> 58
<212> PRT
<213> Escherichia coli

<400> 57

```
Val Asp Ala Lys Phe Asp Lys Glu Ala Gln Glu Ala Phe Tyr Glu Ile
1               5                   10                  15

Leu His Leu Pro Asn Leu Thr Glu Glu Gln Arg Asn Gln Phe Ile Gln
            20                  25                  30

Ser Leu Lys Asp Asp Pro Ser Val Ser Lys Ala Ile Leu Ala Glu Ala
        35                  40                  45

Lys Lys Leu Asn Asp Ala Gln Ala Pro Lys
    50                  55
```

<210> 58
<211> 58
<212> PRT
<213> Escherichia coli

<400> 58

```
Val Asp Ala Lys Phe Asp Lys Glu Ala Gln Glu Ala Phe Tyr Glu Ile
1               5                   10                  15

Leu His Leu Pro Asn Leu Thr Glu Glu Gln Arg Asn Thr Phe Ile Gln
```

```
                        20                    25                          30


        Ser Leu Lys Asp Asp Pro Ser Val Ser Lys Ala Ile Leu Ala Glu Ala
                35                    40                    45


        Lys Lys Leu Asn Asp Ala Gln Ala Pro Lys
                50                    55
```

<210> 59
<211> 58
<212> PRT
<213> Escherichia coli

<400> 59

```
        Val Asp Ala Lys Phe Asp Lys Glu Ala Gln Glu Ala Phe Tyr Glu Ile
        1               5                   10                    15


        Leu His Leu Pro Asn Leu Thr Glu Glu Gln Arg Asn Asn Phe Ile Gln
                        20                    25                    30


        Ser Leu Lys Asp Asp Pro Ser Val Ser Lys Ala Ile Leu Ala Glu Ala
                35                    40                    45


        Lys Lys Leu Asn Asp Ala Gln Ala Pro Lys
                50                    55
```

<210> 60
<211> 58
<212> PRT
<213> Escherichia coli

<400> 60

```
        Val Asp Ala Lys Phe Asp Lys Glu Ala Gln Glu Ala Phe Tyr Glu Ile
        1               5                   10                    15


        Leu His Leu Pro Asn Leu Thr Glu Glu Gln Arg Asn Phe Phe Ile Gln
                        20                    25                    30


        Ser Leu Lys Asp Asp Pro Ser Val Ser Lys Ala Ile Leu Ala Glu Ala
                35                    40                    45


        Lys Lys Leu Asn Asp Ala Gln Ala Pro Lys
                50                    55
```

<210> 61
<211> 58
<212> PRT
<213> Escherichia coli

<400> 61

```
      Val Asp Ala Lys Phe Asp Lys Glu Ala Gln Glu Ala Phe Tyr Glu Ile
      1               5               10              15

      Leu His Leu Pro Asn Leu Thr Glu Glu Gln Arg Asn Leu Phe Ile Gln
                      20              25              30

      Ser Leu Lys Asp Asp Pro Ser Val Ser Lys Ala Ile Leu Ala Glu Ala
                  35              40              45

      Lys Lys Leu Asn Asp Ala Gln Ala Pro Lys
          50              55
```

<210> 62
<211> 58
<212> PRT
<213> Escherichia coli

<400> 62

```
      Val Asp Ala Lys Phe Asp Lys Glu Ala Gln Glu Ala Phe Tyr Glu Ile
      1               5               10              15

      Leu His Leu Pro Asn Leu Thr Glu Glu Gln Arg Asn Trp Phe Ile Gln
                      20              25              30

      Ser Leu Lys Asp Asp Pro Ser Val Ser Lys Ala Ile Leu Ala Glu Ala
                  35              40              45

      Lys Lys Leu Asn Asp Ala Gln Ala Pro Lys
          50              55
```

<210> 63
<211> 58
<212> PRT
<213> Escherichia coli

<400> 63

```
Val Asp Ala Lys Phe Asp Lys Glu Ala Gln Glu Ala Phe Tyr Glu Ile
1               5               10              15

Leu His Leu Pro Asn Leu Thr Glu Glu Gln Arg Asn Ile Phe Ile Gln
            20              25              30

Ser Leu Lys Asp Asp Pro Ser Val Ser Lys Ala Ile Leu Ala Glu Ala
            35              40              45

Lys Lys Leu Asn Asp Ala Gln Ala Pro Lys
        50              55
```

<210> 64
<211> 58
<212> PRT
<213> Escherichia coli

<400> 64

```
Val Asp Ala Lys Phe Asp Lys Glu Ala Gln Glu Ala Phe Tyr Glu Ile
1               5               10              15

Leu His Leu Pro Asn Leu Thr Glu Glu Gln Arg Asn Met Phe Ile Gln
            20              25              30

Ser Leu Lys Asp Asp Pro Ser Val Ser Lys Ala Ile Leu Ala Glu Ala
            35              40              45

Lys Lys Leu Asn Asp Ala Gln Ala Pro Lys
        50              55
```

<210> 65
<211> 58
<212> PRT
<213> Escherichia coli

<400> 65

```
Val Asp Ala Lys Phe Asp Lys Glu Ala Gln Glu Ala Phe Tyr Glu Ile
1               5               10              15

Leu His Leu Pro Asn Leu Thr Glu Glu Gln Arg Asn Val Phe Ile Gln
            20              25              30

Ser Leu Lys Asp Asp Pro Ser Val Ser Lys Ala Ile Leu Ala Glu Ala
            35              40              45

Lys Lys Leu Asn Asp Ala Gln Ala Pro Lys
        50              55
```

<210> 66
<211> 58
<212> PRT
<213> Escherichia coli

<400> 66

```
Val Asp Ala Lys Phe Asp Lys Glu Ala Gln Glu Ala Phe Tyr Glu Ile
1               5                   10                  15

Leu His Leu Pro Asn Leu Thr Glu Glu Gln Arg Asn Asp Phe Ile Gln
            20                  25                  30

Ser Leu Lys Asp Asp Pro Ser Val Ser Lys Ala Ile Leu Ala Glu Ala
            35                  40                  45

Lys Lys Leu Asn Asp Ala Gln Ala Pro Lys
            50                  55
```

<210> 67
<211> 58
<212> PRT
<213> Escherichia coli

<400> 67

```
Val Asp Ala Lys Phe Asp Lys Glu Ala Gln Glu Ala Phe Tyr Glu Ile
1               5                   10                  15

Leu His Leu Pro Asn Leu Thr Glu Glu Gln Arg Asn Glu Phe Ile Gln
            20                  25                  30

Ser Leu Lys Asp Asp Pro Ser Val Ser Lys Ala Ile Leu Ala Glu Ala
            35                  40                  45

Lys Lys Leu Asn Asp Ala Gln Ala Pro Lys
            50                  55
```

<210> 68
<211> 58
<212> PRT
<213> Escherichia coli

<400> 68

73

```
Val Asp Ala Lys Phe Asp Lys Glu Ala Gln Glu Ala Phe Tyr Glu Ile
1               5                   10                  15

Leu His Leu Pro Asn Leu Thr Glu Glu Gln Arg Asn His Phe Ile Gln
            20                  25                  30

Ser Leu Lys Asp Asp Pro Ser Val Ser Lys Ala Ile Leu Ala Glu Ala
            35                  40                  45

Lys Lys Leu Asn Asp Ala Gln Ala Pro Lys
    50                  55
```

<210> 69
<211> 58
<212> PRT
<213> Escherichia coli

<400> 69

```
Val Asp Ala Lys Phe Asp Lys Glu Ala Gln Glu Ala Phe Tyr Glu Ile
1               5                   10                  15

Leu His Leu Pro Asn Leu Thr Glu Glu Gln Arg Asn Arg Phe Ile Gln
            20                  25                  30

Ser Leu Lys Asp Asp Pro Ser Val Ser Lys Ala Ile Leu Ala Glu Ala
            35                  40                  45

Lys Lys Leu Asn Asp Ala Gln Ala Pro Lys
    50                  55
```

<210> 70
<211> 58
<212> PRT
<213> Escherichia coli

<400> 70

```
Val Asp Ala Lys Phe Asp Lys Glu Ala Gln Glu Ala Phe Tyr Glu Ile
1               5                   10                  15

Leu His Leu Pro Asn Leu Thr Glu Glu Gln Arg Asn Lys Phe Ile Gln
            20                  25                  30

Ser Leu Lys Asp Asp Pro Ser Val Ser Lys Ala Ile Leu Ala Glu Ala
            35                  40                  45

Lys Lys Leu Asn Asp Ala Gln Ala Pro Lys
    50                  55
```

<210> 71
<211> 58
<212> PRT
<213> Escherichia coli

<400> 71

```
Val Asp Ala Lys Phe Asp Lys Glu Ala Gln Glu Ala Phe Tyr Glu Ile
1               5                   10                  15

Leu His Leu Pro Asn Leu Thr Glu Glu Gln Arg Asn Ala Phe Ile Gln
            20                  25                  30

Ser Leu Lys Asp Asp Pro Ser Val Ser Lys Ala Ile Leu Ala Glu Ala
            35                  40                  45

Lys Lys Leu Asn Phe Ala Gln Ala Pro Lys
        50                  55
```

<210> 72
<211> 58
<212> PRT
<213> Escherichia coli

<400> 72

```
Val Asp Ala Lys Phe Asp Lys Glu Ala Gln Glu Ala Phe Tyr Glu Ile
1               5                   10                  15

Leu His Leu Pro Asn Leu Thr Glu Glu Gln Arg Asn Ala Phe Ile Gln
            20                  25                  30

Ser Leu Lys Asp Asp Pro Ser Val Ser Lys Ala Ile Leu Ala Glu Ala
            35                  40                  45

Lys Lys Leu Asn Tyr Ala Gln Ala Pro Lys
        50                  55
```

<210> 73
<211> 58
<212> PRT
<213> Escherichia coli

<400> 73

Val Asp Ala Lys Phe Asp Lys Glu Ala Gln Glu Ala Phe Tyr Glu Ile
1               5               10              15

Leu His Leu Pro Asn Leu Thr Glu Glu Gln Arg Asn Ala Phe Ile Gln
            20              25              30

Ser Leu Lys Asp Asp Pro Ser Val Ser Lys Ala Ile Leu Ala Glu Ala
            35              40              45

Lys Lys Leu Asn Trp Ala Gln Ala Pro Lys
    50              55

<210> 74
<211> 58
<212> PRT
<213> Escherichia coli

<400> 74

Val Asp Ala Lys Phe Asp Lys Glu Ala Gln Glu Ala Phe Tyr Glu Ile
1               5               10              15

Leu His Leu Pro Asn Leu Thr Glu Glu Gln Arg Asn Ala Phe Ile Gln
            20              25              30

Ser Leu Lys Asp Asp Pro Ser Val Ser Lys Ala Ile Leu Ala Glu Ala
            35              40              45

Lys Lys Leu Asn Lys Ala Gln Ala Pro Lys
    50              55

<210> 75
<211> 58
<212> PRT
<213> Escherichia coli

<400> 75

Val Asp Ala Lys Phe Asp Lys Glu Ala Gln Glu Ala Phe Tyr Glu Ile
1               5               10              15

Leu His Leu Pro Asn Leu Thr Glu Glu Gln Arg Asn Ala Phe Ile Gln
            20              25              30

Ser Leu Lys Asp Asp Pro Ser Val Ser Lys Ala Ile Leu Ala Glu Ala
            35              40              45

Lys Lys Leu Asn Arg Ala Gln Ala Pro Lys
    50              55

<210> 76
<211> 57
<212> PRT
<213> Escherichia coli

<400> 76

Val Asp Ala Lys Phe Asp Lys Glu Gln Glu Ala Phe Tyr Glu Ile Leu
1               5                   10                  15

His Leu Pro Asn Leu Thr Glu Glu Gln Arg Asn Ala Phe Ile Gln Ser
            20                  25                  30

Leu Lys Asp Asp Pro Ser Val Ser Lys Ala Ile Leu Ala Glu Ala Lys
        35                  40                  45

Lys Leu Asn Asp Ala Gln Ala Pro Lys
    50                  55

<210> 77
<211> 57
<212> PRT
<213> Escherichia coli

<400> 77

Val Asp Ala Lys Phe Asp Lys Glu Ala Gln Glu Ala Phe Tyr Glu Ile
1               5                   10                  15

Leu His Leu Pro Asn Leu Thr Glu Glu Gln Arg Asn Ala Phe Ile Gln
            20                  25                  30

Ser Leu Lys Asp Asp Pro Ser Ser Lys Ala Ile Leu Ala Glu Ala Lys
            35                  40                  45

Lys Leu Asn Asp Ala Gln Ala Pro Lys
    50                  55

<210> 78
<211> 57
<212> PRT
<213> Escherichia coli

<400> 78

```
Val Asp Ala Lys Phe Asp Lys Glu Ala Gln Glu Ala Phe Tyr Glu Ile
1               5               10              15

Leu His Leu Pro Asn Leu Thr Glu Glu Gln Arg Asn Ala Phe Ile Gln
            20              25              30

Ser Leu Lys Asp Asp Pro Ser Val Ser Ala Ile Leu Ala Glu Ala Lys
        35              40              45

Lys Leu Asn Asp Ala Gln Ala Pro Lys
    50              55
```

<210> 79
<211> 57
<212> PRT
<213> Escherichia coli

<400> 79

```
Val Asp Ala Lys Phe Asp Lys Glu Ala Gln Glu Ala Phe Tyr Glu Ile
1               5               10              15

Leu His Leu Pro Asn Leu Thr Glu Glu Gln Arg Asn Ala Phe Ile Gln
            20              25              30

Ser Leu Lys Asp Asp Pro Ser Val Ser Lys Ile Leu Ala Glu Ala Lys
        35              40              45

Lys Leu Asn Asp Ala Gln Ala Pro Lys
    50              55
```

<210> 80
<211> 114
<212> PRT
<213> Escherichia coli

<400> 80

```
Val Asp Ala Lys Phe Asp Lys Glu Ala Gln Glu Ala Phe Tyr Glu Ile
1               5               10              15
```

```
Leu His Leu Pro Asn Leu Thr Glu Glu Gln Arg Asn Ala Phe Ile Gln
            20                  25              30

Ser Leu Lys Asp Asp Pro Ser Val Ser Lys Ala Ile Leu Ala Glu Ala
            35                  40              45

Lys Lys Leu Asn Asp Ala Gln Ala Pro Lys Val Phe Asp Lys Glu Ala
            50                  55              60

Gln Glu Ala Phe Tyr Glu Ile Leu His Leu Pro Asn Leu Thr Glu Glu
65                  70                  75                  80

Gln Arg Asn Ala Phe Ile Gln Ser Leu Lys Asp Asp Pro Ser Val Ser
                85                  90                  95

Lys Ala Ile Leu Ala Glu Ala Lys Lys Leu Asn Asp Ala Gln Ala Pro
                100                 105                 110

Lys Cys
```

<210> 81
<211> 114
<212> PRT
<213> Escherichia coli

<400> 81

```
Val Asp Ala Lys Phe Asp Lys Glu Ala Gln Glu Ala Phe Tyr Glu Ile
1               5                   10                  15

Leu His Leu Pro Asn Leu Thr Glu Glu Gln Arg Asn Ala Phe Ile Gln
            20                  25              30

Ser Leu Lys Asp Asp Pro Ser Val Ser Lys Ala Ile Leu Ala Glu Ala
            35                  40              45

Lys Lys Leu Asn Asp Ala Gln Ala Pro Ala Lys Phe Asp Lys Glu Ala
            50                  55              60

Gln Glu Ala Phe Tyr Glu Ile Leu His Leu Pro Asn Leu Thr Glu Glu
65                  70                  75                  80

Gln Arg Asn Ala Phe Ile Gln Ser Leu Lys Asp Asp Pro Ser Val Ser
                85                  90                  95

Lys Ala Ile Leu Ala Glu Ala Lys Lys Leu Asn Asp Ala Gln Ala Pro
                100                 105                 110
```

Lys Cys

<210> 82
<211> 114
<212> PRT
<213> Escherichia coli

<400> 82

Val Asp Ala Lys Phe Asp Lys Glu Ala Gln Glu Ala Phe Tyr Glu Ile
1               5                   10                  15

Leu His Leu Pro Asn Leu Thr Glu Glu Gln Arg Asn Ala Phe Ile Gln
            20                  25                  30

Ser Leu Lys Asp Asp Pro Ser Val Ser Lys Ala Ile Leu Ala Glu Ala
            35                  40                  45

Lys Lys Leu Asn Asp Ala Gln Ala Pro Ala Lys Phe Asp Lys Glu Ala
        50                  55                  60

Gln Glu Ala Phe Tyr Glu Ile Leu His Leu Pro Asn Leu Thr Glu Glu
65                  70                  75                  80

Gln Arg Asn Ala Phe Ile Gln Ser Leu Lys Asp Asp Pro Ser Val Ser
                85                  90                  95

Lys Ala Ile Leu Ala Glu Ala Lys Lys Leu Asn Asp Ala Gln Ala Pro
                100                 105                 110

Lys Cys

<210> 83
<211> 112
<212> PRT
<213> Escherichia coli

<400> 83

Val Asp Ala Lys Phe Asp Lys Glu Ala Gln Glu Ala Phe Tyr Glu Ile
1               5                   10                  15

Leu His Leu Pro Asn Leu Thr Glu Glu Gln Arg Asn Ala Phe Ile Gln
            20                  25                  30

Ser Leu Lys Asp Asp Pro Ser Val Ser Lys Ala Ile Leu Ala Glu Ala
            35                  40                  45

```
Lys Lys Leu Asn Asp Ala Gln Ala Pro Lys Val Asp Ala Ala Gln Glu
    50                  55                  60

Ala Phe Tyr Glu Ile Leu His Leu Pro Asn Leu Thr Glu Glu Gln Arg
65                  70                  75                  80

Asn Ala Phe Ile Gln Ser Leu Lys Asp Asp Pro Ser Val Ser Lys Ala
                85                  90                  95

Ile Leu Ala Glu Ala Lys Lys Leu Asn Asp Ala Gln Ala Pro Lys Cys
                100                 105                 110
```

<210> 84
<211> 114
<212> PRT
<213> Escherichia coli

<400> 84

```
Val Asp Ala Lys Phe Asp Lys Glu Ala Gln Glu Ala Phe Tyr Glu Ile
1               5                   10                  15

Leu His Leu Pro Asn Leu Thr Glu Glu Gln Arg Asn Ala Phe Ile Gln
            20                  25                  30

Ser Leu Lys Asp Asp Pro Ser Val Ser Lys Ala Ile Leu Ala Glu Ala
        35                  40                  45

Lys Lys Leu Asn Asp Ala Gln Val Asp Ala Lys Phe Asp Lys Glu Ala
    50                  55                  60

Gln Glu Ala Phe Tyr Glu Ile Leu His Leu Pro Asn Leu Thr Glu Glu
65                  70                  75                  80

Gln Arg Asn Ala Phe Ile Gln Ser Leu Lys Asp Asp Pro Ser Val Ser
                85                  90                  95

Lys Ala Ile Leu Ala Glu Ala Lys Lys Leu Asn Asp Ala Gln Ala Pro
                100                 105                 110

Lys Cys
```

<210> 85
<211> 114
<212> PRT
<213> Escherichia coli

<400> 85

```
Val Asp Ala Lys Phe Asp Lys Glu Ala Gln Glu Ala Phe Tyr Glu Ile

1               5               10              15


Leu His Leu Pro Asn Leu Thr Glu Glu Gln Arg Asn Ala Phe Ile Gln
            20              25              30

Ser Leu Lys Asp Asp Pro Ser Val Ser Lys Ala Ile Leu Ala Glu Ala
        35              40              45

Lys Lys Leu Asn Asp Ala Gln Ala Pro Lys Lys Phe Asp Lys Glu Ala
    50              55              60

Gln Glu Ala Phe Tyr Glu Ile Leu His Leu Pro Asn Leu Thr Glu Glu
65              70              75              80

Gln Arg Asn Ala Phe Ile Gln Ser Leu Lys Asp Asp Pro Ser Val Ser
            85              90              95

Lys Ala Ile Leu Ala Glu Ala Lys Lys Leu Asn Asp Ala Gln Ala Pro
            100             105             110


Lys Cys
```

<210> 86
<211> 109
<212> PRT
<213> Escherichia coli

<400> 86

```
Val Asp Ala Lys Phe Asp Lys Glu Ala Gln Glu Ala Phe Tyr Glu Ile
1               5                   10                  15

Leu His Leu Pro Asn Leu Thr Glu Glu Gln Arg Asn Ala Phe Ile Gln
            20                  25                  30

Ser Leu Lys Asp Asp Pro Ser Val Ser Lys Ala Ile Leu Ala Glu Ala
            35                  40                  45

Lys Lys Leu Asn Asp Ala Gln Ala Pro Lys Ala Gln Glu Ala Phe Tyr
    50                  55                  60

Glu Ile Leu His Leu Pro Asn Leu Thr Glu Glu Gln Arg Asn Ala Phe
65                  70                  75                  80

Ile Gln Ser Leu Lys Asp Asp Pro Ser Val Ser Lys Ala Ile Leu Ala
                85                  90                  95

Glu Ala Lys Lys Leu Asn Asp Ala Gln Ala Pro Lys Cys
                100                 105
```

<210> 87
<211> 121
<212> PRT
<213> Escherichia coli

<400> 87

```
Val Asp Ala Lys Phe Asp Lys Glu Ala Gln Glu Ala Phe Tyr Glu Ile
1               5               10              15

Leu His Leu Pro Asn Leu Thr Glu Glu Gln Arg Asn Ala Phe Ile Gln
            20              25              30

Ser Leu Lys Asp Asp Pro Ser Val Ser Lys Ala Ile Leu Ala Glu Ala
        35              40              45

Lys Lys Leu Asn Asp Ala Gln Ala Pro Lys Tyr Glu Asp Gly Val Asp
    50              55              60

Ala Lys Phe Asp Lys Glu Ala Gln Glu Ala Phe Tyr Glu Ile Leu His
65              70              75              80

Leu Pro Asn Leu Thr Glu Glu Gln Arg Asn Ala Phe Ile Gln Ser Leu
                85              90              95

Lys Asp Asp Pro Ser Val Ser Lys Ala Ile Leu Ala Glu Ala Lys Lys
            100             105             110

Leu Asn Asp Ala Gln Ala Pro Lys Cys
            115             120
```

<210> 88
<211> 117
<212> PRT
<213> Escherichia coli

<400> 88

```
Val Asp Ala Lys Phe Asp Lys Glu Ala Gln Glu Ala Phe Tyr Glu Ile
1               5               10              15

Leu His Leu Pro Asn Leu Thr Glu Glu Gln Arg Asn Ala Phe Ile Gln
            20              25              30

Ser Leu Lys Asp Asp Pro Ser Val Ser Lys Ala Ile Leu Ala Glu Ala
        35              40              45

Lys Lys Leu Asn Asp Ala Gln Ala Pro Lys Val Asp Ala Lys Phe Asp
    50              55              60
```

```
            Lys Glu Ala Gln Glu Ala Phe Tyr Glu Ile Leu His Leu Pro Asn Leu
            65              70              75              80


            Thr Glu Glu Gln Arg Asn Ala Phe Ile Gln Ser Leu Lys Asp Asp Pro
                            85              90              95


            Ser Val Ser Lys Ala Ile Leu Ala Glu Ala Lys Lys Leu Asn Asp Ala
                            100             105             110


            Gln Ala Pro Lys Cys
                            115
```

<210> 89
<211> 55
<212> PRT
<213> Escherichia coli

<400> 89

```
            Val Phe Asp Lys Glu Ala Gln Glu Ala Phe Tyr Glu Ile Leu His Leu
            1               5               10              15


            Pro Asn Leu Thr Glu Glu Gln Arg Asn Ala Phe Ile Gln Ser Leu Lys
                            20              25              30


            Asp Asp Pro Ser Val Ser Lys Ala Ile Leu Ala Glu Ala Lys Lys Leu
                            35              40              45


            Asn Asp Ala Gln Ala Pro Lys
                            50              55
```

<210> 90
<211> 55
<212> PRT
<213> Escherichia coli

<400> 90

```
            Ala Lys Phe Asp Lys Glu Ala Gln Glu Ala Phe Tyr Glu Ile Leu His
            1               5               10              15


            Leu Pro Asn Leu Thr Glu Glu Gln Arg Asn Ala Phe Ile Gln Ser Leu
                            20              25              30


            Lys Asp Asp Pro Ser Val Ser Lys Ala Ile Leu Ala Glu Ala Lys Lys
                            35              40              45


            Leu Asn Asp Ala Gln Ala Pro
                            50              55
```

<210> 91
<211> 53
<212> PRT
<213> Escherichia coli

<400> 91

```
Val Asp Ala Ala Gln Glu Ala Phe Tyr Glu Ile Leu His Leu Pro Asn
1               5                   10                  15

Leu Thr Glu Glu Gln Arg Asn Ala Phe Ile Gln Ser Leu Lys Asp Asp
            20                  25                  30

Pro Ser Val Ser Lys Ala Ile Leu Ala Glu Ala Lys Lys Leu Asn Asp
            35                  40                  45

Ala Gln Ala Pro Lys
            50
```

<210> 92
<211> 55
<212> PRT
<213> Escherichia coli

<400> 92

```
Val Asp Ala Lys Phe Asp Lys Glu Ala Gln Glu Ala Phe Tyr Glu Ile
1               5                   10                  15

Leu His Leu Pro Asn Leu Thr Glu Glu Gln Arg Asn Ala Phe Ile Gln
            20                  25                  30

Ser Leu Lys Asp Asp Pro Ser Val Ser Lys Ala Ile Leu Ala Glu Ala
            35                  40                  45

Lys Lys Leu Asn Asp Ala Gln
            50                  55
```

<210> 93
<211> 55
<212> PRT
<213> Escherichia coli

<400> 93

```
Lys Phe Asp Lys Glu Ala Gln Glu Ala Phe Tyr Glu Ile Leu His Leu
1               5                   10                  15

Pro Asn Leu Thr Glu Glu Gln Arg Asn Ala Phe Ile Gln Ser Leu Lys
            20                  25                  30

Asp Asp Pro Ser Val Ser Lys Ala Ile Leu Ala Glu Ala Lys Lys Leu

                35                  40                  45

        Asn Asp Ala Gln Ala Pro Lys
            50                  55
```

<210> 94
<211> 50
<212> PRT
<213> Escherichia coli

<400> 94

```
Ala Gln Glu Ala Phe Tyr Glu Ile Leu His Leu Pro Asn Leu Thr Glu
1               5                   10                  15

Glu Gln Arg Asn Ala Phe Ile Gln Ser Leu Lys Asp Asp Pro Ser Val
            20                  25                  30

Ser Lys Ala Ile Leu Ala Glu Ala Lys Lys Leu Asn Asp Ala Gln Ala
        35                  40                  45

Pro Lys
    50
```

<210> 95
<211> 62
<212> PRT
<213> Escherichia coli

<400> 95

```
        Val Asp Ala Lys Phe Asp Lys Glu Ala Gln Glu Ala Phe Tyr Glu Ile
        1               5               10                  15


        Leu His Leu Pro Asn Leu Thr Glu Glu Gln Arg Asn Ala Phe Ile Gln
                    20              25                  30


        Ser Leu Lys Asp Asp Pro Ser Val Ser Lys Ala Ile Leu Ala Glu Ala
                    35              40                  45


        Lys Lys Leu Asn Asp Ala Gln Ala Pro Lys Tyr Glu Asp Gly
            50              55                  60
```